(19) Europäisches Patentamt
European Patent Office
Office européen des brevets

(11) Veröffentlichungsnummer : **0 327 800 B1**

# (12) EUROPÄISCHE PATENTSCHRIFT

(45) Veröffentlichungstag der Patentschrift :
**27.05.92 Patentblatt 92/22**

(51) Int. Cl.$^5$ : **C07D 237/04,** C07D 417/12, C07D 403/12, C07D 401/10, A61K 31/50

(21) Anmeldenummer : **89100153.9**

(22) Anmeldetag : **05.01.89**

## (54) 6-Phenyldihydro-3(2H)-pyridazinone, Verfahren zu ihrer Herstellung und diese Verbindungen enthaltende Arzneimittel.

(30) Priorität : **12.02.88 DE 3804490**

(43) Veröffentlichungstag der Anmeldung :
**16.08.89 Patentblatt 89/33**

(45) Bekanntmachung des Hinweises auf die Patenterteilung :
**27.05.92 Patentblatt 92/22**

(84) Benannte Vertragsstaaten :
**AT BE CH DE ES FR GB GR IT LI LU NL SE**

(56) Entgegenhaltungen :
**EP-A- 0 186 484**
**EP-A- 0 304 534**
**DE-A- 2 207 517**

(73) Patentinhaber : **HEUMANN PHARMA GMBH & CO**
**Heideloffstrasse 18 - 28**
**W-8500 Nürnberg 1 (DE)**

(72) Erfinder : **Herter, Rolf, Dr. Dipl.-Chem.**
**Bayernstrasse 42**
**W-8540 Schwabach (DE)**
Erfinder : **Engler, Heidrun, Dr. med.vet.**
**Ringstrasse 23**
**W-8501 Cadolzburg (DE)**
Erfinder : **Mörsdorf, Peter, Dr. Dipl.-Chem.**
**Ziegelstrasse 64**
**W-8506 Langenzenn (DE)**
Erfinder : **Schickaneder, Helmut, Dr. Dipl.-Chem.**
**Moosäcker 25**
**W-8501 Eckental (DE)**
Erfinder : **Pfahlert, Volker, Dr.**
**Hummelsteiner Weg 14**
**W-8500 Nürnberg (DE)**
Erfinder : **Ahrens, Kurt Henning, Dr.**
**Praterstrasse 9**
**W-8500 Nürnberg (DE)**

(74) Vertreter : **Kraus, Walter, Dr. et al**
**Patentanwälte Kraus, Weisert & Partner**
**Thomas-Wimmer-Ring 15**
**W-8000 München 22 (DE)**

**EP 0 327 800 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Digitalisglykoside, wie Digoxin und Digitoxin, und Sympathomimetika waren viele Jahre lang die einzigen Möglichkeiten für die medikamentöse Behandlung der Herzinsuffizienz. Die Nachteile dieser Substanzen, wie unerwünschte chronotrope und arrhythmogene Nebenwirkungen, Tachyphylaxie und, im Fall der Sympathomimetika, fehlende orale Verfügbarkeit, führten zu einer intensiven Suche nach neuen positiv inotrop wirkenden Verbindungsklassen.

Dabei wurden auch in der Reihe der 3(2H)-Pyridazinone Substanzen gefunden, wie zum Beispiel Pimobendan (DE-OS 28 37 161) oder Imazodan (EP-OS 0 075 436), die positiv inotrope Wirkungen aufweisen.

Andererseits sind auch substituierte 6-Phenyl-3(2H)-pyridazinone bekannt, für die nur antihypertensive bzw. antithrombotische Wirkungen beschrieben werden (DE-OS 21 50 436, DE-OS 22 07 517, US-PS 4 624 951).

Der vorliegenden Erfindung liegt die Aufgabe zugrunde, neue substituierte 6-Phenyldihydro-3(2H)-pyridazinone zur Verfügung zu stellen, die bei guter oraler Verfügbarkeit eine gute antihypertensive und gleichzeitig eine hohe positiv inotrope Wirksamkeit besitzen.

Gegenstand der Erfindung sind substituierte 6-Phenyldihydro-3(2H)-pyridazinone der allgemeinen Formel I

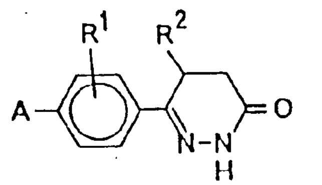

(I)

in der $R^1$ eine Nitrogruppe, eine Aminogruppe, eine Hydroxygruppe, eine Cyanogruppe oder ein Halogenatom bedeutet,
$R^2$ für ein Wasserstoffatom, eine Methylgruppe oder eine Hydroxymethylgruppe steht und
A für die Gruppe

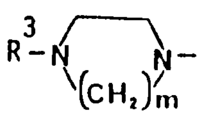 , $R^3-NH-(CH_2)_n-NH-$, 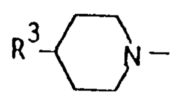

oder 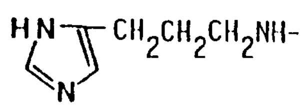

steht, worin $R^3$ ein Wasserstoffatom, eine gegebenenfalls mit einer Amino- oder Hydroxygruppe substituierte $C_1$-$C_6$-Alkylgruppe, eine gegebenenfalls substituierte Aryl- oder Heteroarylgruppe, eine Cyanogruppe, eine Acylgruppe, eine gegebenenfalls substituierte Aroylgruppe, eine Carboxylgruppe oder eine $C_1$-$C_6$-Alkoxycarbonylgruppe bedeutet, m eine ganze Zahl von 1 bis 3 und n eine ganze Zahl von 2 bis 4 ist, sowie die physiologisch annehmbaren Salze davon.

In der allgemeinen Formel I bedeutet der Rest $R^1$, der vorzugsweise in meta-Position zum Pyridazinonring gebunden ist, eine Nitrogruppe, eine Aminogruppe, eine Hydroxygruppe, eine Cyanogruppe oder ein Halogenatom, beispielsweise ein Fluor-, Chlor- oder Bromatom, vorzugsweise ein Fluor- oder Chloratom. Ganz besonders bevorzugt werden Verbindungen, bei denen $R^1$ für eine Nitro- oder Aminogruppe steht und $R^1$ in meta-Position zum Pyridazinonring gebunden ist.

$R^2$ bedeutet ein Wasserstoffatom, eine Methylgruppe oder Hydroxymethylgruppe, vorzugsweise eine Methylgruppe.

A steht für eine der Gruppen

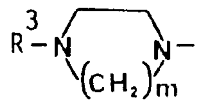 , $R^3$-NH-$(CH_2)_n$-NH-,

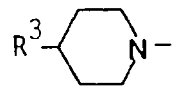

oder 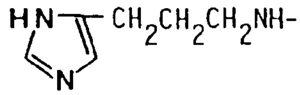

In den oben genannten Gruppierungen ist $R^3$ ein Wasserstoffatom oder eine gegebenenfalls mit einer Amino- oder Hydroxygruppe substituierte $C_1$-$C_6$-Alkylgruppe, vorzugsweise $C_1$-$C_4$-Alkylgruppe. Beispiele für die $C_1$-$C_6$-Alkylgruppe sind die Methyl-, Ethyl-, n-Propyl-, Isopropyl- und n-Butylgruppe. Im Falle der Substitution dieser $C_1$-$C_6$-Alkylgruppe wird die endständige Substitution bevorzugt.

$R^3$ kann weiterhin für eine Aryl- oder Heteroarylgruppe stehen, die gegebenenfalls mit einem oder zwei Halogenatomen, beispielsweise Fluor-, Chlor- oder Bromatomen, einer oder zwei $C_1$-$C_3$-Alkylgruppen, beispielsweise Methyl-, Ethyl- oder Propylgruppen, oder einer oder zwei $C_1$-$C_3$-Alkoxygruppen, beispielsweise Methoxy- oder Ethoxygruppen, substituiert ist. Beispiele für Arylgruppen sind die Phenyl- und die Naphthylgruppe, wobei die Phenylgruppe bevorzugt wird. Beispiele für Heteroarylgruppen sind die Furanyl-, Thiophenyl-, Imidazolyl-, Pyridyl- und Pyrimidinylgruppe, wobei die Imidazolyl-, die Pyridyl- und die Pyrimidinylgruppe bevorzugt werden.

Des weiteren kann $R^3$ für eine Cyanogruppe, eine $C_1$-$C_6$-Acylgruppe, beispielsweise eine Formyl-, Acetyl- oder Propionylgruppe, oder eine Aroylgruppe, beispielsweise eine Benzoylgruppe, die gegebenenfalls mit einem oder mehreren, zum Beispiel zwei, Halogenatomen, $C_1$-$C_3$-Alkylgruppen oder $C_1$-$C_3$-Alkoxygruppen substituiert ist, stehen. Einzelbeispiele für die Substituenten der Aroylgruppen sind die oben im Zusammenhang mit den Substituenten der Aryl- oder Heteroarylgruppe genannten speziellen Gruppen bzw. Atome.

Schließlich kann $R^3$ noch eine Carboxylgruppe oder $C_1$-$C_6$-Alkoxycarbonylgruppe bedeuten, beispielsweise eine Methoxycarbonyl-, Ethoxycarbonyl- oder tert.-Butoxycarbonylgruppe, wobei die Ethoxycarbonylgruppe bevorzugt wird. In allen Fällen ist m eine ganze Zahl von 1 bis 3, vorzugsweise die Zahl 2. n hat den Wert 2, 3 oder 4, wobei die Werte 2 und 3 bevorzugt werden.

Eine bevorzugte Verbindungsgruppe gemäß der vorliegenden Erfindung ist dadurch gekennzeichnet, daß in der allgemeinen Formel I $R^1$ für eine Nitrogruppe, eine Aminogruppe, eine Hydroxygruppe, eine Cyanogruppe oder ein Halogenatom, insbesondere eine Nitro- oder Aminogruppe, steht, $R^2$ ein Wasserstoffatom, eine Methylgruppe oder eine Hydroxymethylgruppe bedeutet, wobei die Methylgruppe bevorzugt wird, und A die Gruppe

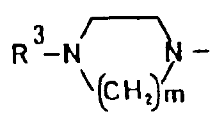

ist, worin

$R^3$ ein Wasserstoffatom, eine gegebenenfalls mit einer Amino- oder Hydroxygruppe substituierte $C_1$-$C_6$-Alkylgruppe, insbesondere eine 2-Aminoethyl- oder 3-Aminopropylgruppe, eine gegebenenfalls substituierte Aryl- oder Heteroarylgruppe, eine Cyanogruppe, eine Acylgruppe, eine gegebenenfalls substituierte Aroylgruppe oder eine $C_1$-$C_6$-Alkoxycarbonylgruppe, insbesondere eine Methoxycarbonyl- oder Ethoxycarbonylgruppe, ist und m einen Wert von 1 bis 3, vorzugsweise den Wert 2, hat.

Eine weitere bevorzugte Verbindungsgruppe gemäß der vorliegenden Erfindung ist dadurch gekennzeichnet, daß in der allgemeinen Formel I $R^1$ für eine Nitrogruppe, eine Aminogruppe, eine Hydroxygruppe, eine Cyanogruppe oder ein Halogenatom steht, $R^2$ ein Wasserstoffatom oder eine Methylgruppe bedeutet, A für die Gruppe $R^3$-NH-$(CH_2)_n$-NH- steht, worin $R^3$ ein Wasserstoffatom, eine gegebenenfalls substituierte Aryl- oder Heteroarylgruppe, eine Acylgruppe, eine gegebenenfalls substituierte Aroylgruppe oder $C_1$-$C_6$-Alkoxycarbo-

nylgruppe ist und n einen Wert von 2 bis 4, vorzugsweise den Wert 2 oder 3, hat.

Eine weitere bevorzugte Gruppe von erfindungsgemäßen Verbindungen ist dadurch gekennzeichnet, daß in der allgemeinen Formel I $R^1$ für eine Nitrogruppe, eine Aminogruppe, eine Hydroxygruppe, eine Cyanogruppe oder ein Halogenatom steht, $R^2$ ein Wasserstoffatom oder eine Methylgruppe ist und A die Gruppe

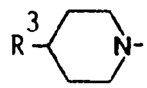

bedeutet, wobei $R^3$ eine gegebenenfalls mit einer Amino- oder Hydroxygruppe substituierte $C_1$-$C_6$-Alkylgruppe, eine gegebenenfalls substituierte Aryl- oder Heteroarylgruppe, eine Acylgruppe, eine gegebenenfalls substituierte Aroylgruppe, eine Carboxylgruppe oder eine $C_1$-$C_6$-Alkoxycarbonylgruppe bedeutet.

Schließlich ist eine weitere bevorzugte Verbindungsgruppe gemäß der vorliegenden Erfindung dadurch gekennzeichnet, daß $R^1$ für eine Nitrogruppe, eine Aminogruppe, eine Hydroxygruppe, eine Cyanogruppe oder ein Halogenatom und $R^2$ für ein Wasserstoffatom, eine Methylgruppe oder eine Hydroxymethylgruppe steht und A die Gruppe

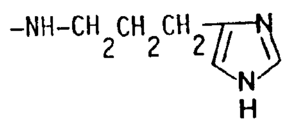

ist.

Bevorzugte Einzelverbindungen sind die folgenden Verbindungen:
6-[3-Nitro-4-(1-piperazinyl)-phenyl]-4,5-dihydro-5-methyl-3(2H)-pyridazinon;
6-[4-(4-Ethoxycarbonylpiperazin-1-yl)-3-nitrophenyl]-4,5-dihydro-5-methyl-3(2H)-pyridazinon;
6-[3-Amino-4-(4-ethoxycarbonylpiperazin-1-yl)-phenyl]-4,5-dihydro-5-methyl-3(2H)-pyridazinon;
6-[4-(4-Acetylpiperazin-1-yl)-3-nitrophenyl]-4,5-dihydro-5-methyl-3(2H)-pyridazinon;
6-[4-(4-Ethoxycarbonylpiperazin-1-yl)-3-nitrophenyl]-4,5-dihydro-3(2H)-pyridazinon;
und deren physiologisch annehmbare Salze.

Die erfindungsgemäßen Verbindungen können nach mehreren Verfahren hergestellt werden.

Ein Verfahren, das allgemein zur Herstellung der erfindungsgemäßen Verbindungen, d.h. von Verbindungen der allgemeinen Formel I, bei der A, $R^1$ und $R^2$ wie oben definiert sind, geeignet ist, ist dadurch gekennzeichnet, daß man eine Verbindung der allgemeinen Formel II

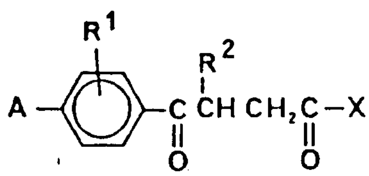

(II)

in der A, $R^1$ und $R^2$ wie oben definiert sind und X für die Gruppe -OH oder die Gruppe $-OR^4$ steht, in der $R^4$ eine gegebenenfalls substituierte $C_1$-$C_6$-Alkylgruppe oder eine gegebenenfalls substituierte Arylgruppe ist, mit Hydrazin oder einem chemischen Äquivalent davon zu einer Verbindung der allgemeinen Formel I umsetzt und die erhaltene Verbindung gegebenenfalls in an sich bekannter Weise in ihr physiologisch annehmbares Salz umwandelt.

Unter "chemischen Äquivalenten des Hydrazins" werden dabei Hydrazinhydrat, Hydrazinethanolat bzw. ähnliche Solvate oder seine Salze verstanden. Die Umsetzungen mit Hydrazin oder einem chemischen Äquivalent davon erfolgen vorzugsweise mit einem Überschuß an Reagenz und in einem polaren Lösungsmittel, wie zum Beispiel Essigsäure, oder einem Alkohol, wie Methanol, Ethanol oder Isopropanol. Die Reaktion wird bei Temperaturen von Raumtemperatur bis zur Rückflußtemperatur des verwendeten Lösungsmittels, vorzugsweise bei Rückflußtemperatur durchgeführt.

Verbindungen der allgemeinen Formel I, bei denen A und $R^2$ wie oben definiert sind und $R^1$ für eine Nitrogruppe steht, können auch dadurch hergestellt werden, daß man eine Verbindung der allgemeinen Formel III

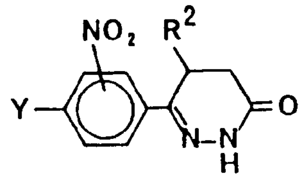

(III)

in der $R^2$ wie oben definiert ist und Y für ein Halogenatom, beispielsweise ein Fluor-, Chlor- oder Bromatom, steht, mit einer Verbindung der allgemeinen Formel IV

H-A (IV)

in der A wie oben definiert ist, umsetzt. Dabei wird die Verbindung der Formel IV vorzugsweise im Überschuß, insbesondere in einer zwei- bis dreifachen molaren Menge, bezogen auf die Verbindung der allgemeinen Formel III, eingesetzt. Die Umsetzungen werden in einem inerten Lösungsmittel, wie Dimethylformamid, Dimethylsulfoxid, Acetonitril, Dioxan oder Tetrahydrofuran, und bei erhöhter Temperatur, vorzugsweise im Temperaturbereich von 80 bis 180°C, durchgeführt. Zum Abfangen der in der Reaktion entstehenden Säure H-X dient entweder der vorhandene Überschuß der Verbindung der allgemeinen Formel IV, oder es wird dem Reaktionsgemisch eine Hilfsbase, beispielsweise Kaliumcarbonat, Triethylamin oder Pyridin, zugesetzt.

Verbindungen der allgemeinen Formel I, bei denen A und $R^2$ wie oben definiert sind und $R^1$ für eine Aminogruppe steht, können auch dadurch hergestellt werden, daß man eine Verbindung der allgemeinen Formal Ia

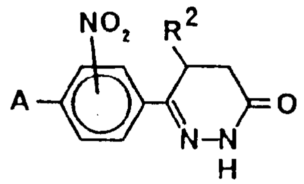

(Ia)

in der A und $R^2$ wie oben definiert sind und $R^1$ eine Nitrogruppe ist, mit einem geeigneten Reduktionsmittel reduziert. Geeignete Reduktionsmittel für die Reduktion der Nitrogruppe zur Aminogruppe sind beispielsweise unedle Metalle bzw. ihre Salze in saurem Medium, wie Eisen in Salzsäure, Zinn oder Zinndichlorid in Salzsäure oder Zink in Essigsäure; Schwefelverbindungen, wie Ammoniumsulfid, Natriumsulfid, Natriumdithionit; Hydrazin oder ein chemisches Äquivalent davon in Gegenwart eines Katalysators, wie Raney-Nickel; Cyclohexen in Gegenwart eines Edelmetallkatalysators, wie Platinoxid oder Palladium; Wasserstoff in Gegenwart von Katalysatoren, beispielsweise Raney-Nickel, Palladium oder Platinoxid.

Verbindungen der allgemeinen Formel I, bei denen A und $R^2$ die oben angegebenen Bedeutungen haben und $R^1$ für eine Hydroxygruppe, eine Cyanogruppe oder ein Halogenatom steht, können auch dadurch hergestellt werden, daß man eine Verbindung der allgemeinen Formel Ib

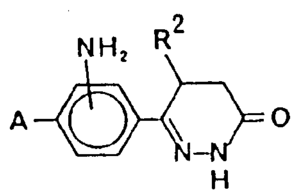

(Ib)

in der A und $R^2$ wie oben definiert sind, zu einer Verbindung der allgemeinen Formel V

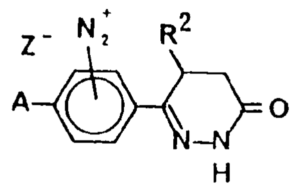

(V)

in der A und $R^2$ wie oben definiert sind und Z für ein Halogenatom, eine Hydroxygruppe, eine Cyanogruppe oder die Tetrafluoroboratgruppe steht, diazotiert und das Diazoniumsalz der allgemeinen Formel V, gegebenenfalls in Gegenwart von Kupferpulver oder eines Kupfer-(I)-halogenids oder von Kupfer-(I)-cyanid, thermisch zersetzt. Die Diazotierung des aromatischen Amins der Formel Ib erfolgt beispielsweise mit einem Alkalimetallnitrit, wie zum Beispiel Natriumnitrit, und wird in saurer wäßriger Lösung bei einer Temperatur zwischen -10°C und +10°C, vorzugsweise bei einer Temperatur zwischen 0 und +5°C, durchgeführt.

Die Zersetzung des Diazoniumsalzes erfolgt thermisch bei Temperaturen zwischen 30 und 150°C. Dabei wird die Diazoniumgruppe am aromatischen Ring durch eine Hydroxygruppe, eine Cyanogruppe oder ein Halogenatom ersetzt. Für den Fall, daß das Halogenatom ein Chlor-, Brom- oder Jodatom ist, wird vorteilhafterweise Kupferpulver oder das entsprechende Kupfer-(I)-halogenid zugesetzt. Im Fall der Einführung von $R^1$ = Fluor ist $Z^-$ ein Fluorid- oder Tetrafluoroborat-Anion. Bei der Einführung einer Cyanogruppe setzt man das Diazoniumsalz der Formel V beispielsweise mit Kupfer-(I)-cyanid, das in Kaliumcyanid komplex gelöst ist, um.

Verbindungen der allgemeinen Formel I, bei denen $R^1$ und $R^2$ wie oben definiert sind und A für die Gruppe

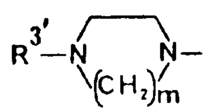

oder die Gruppe $R^{3'}$-NH-$(CH_2)_n$-NH- steht, wobei m und n wie oben definiert sind und $R^{3'}$ für eine Cyanogruppe, eine Acylgruppe, eine gegebenenfalls substituierte Aroylgruppe oder eine $C_1$-$C_6$-Alkoxycarbonylgruppe steht, können auch dadurch hergestellt werden, daß man eine Verbindung der allgemeinen Formeln Ic oder Id

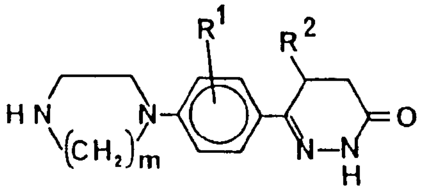

(Ic)

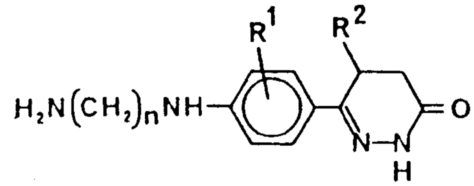

(Id)

in denen $R^1$, $R^2$, m und n die oben angegebenen Bedeutungen besitzen, mit einem Acylierungsmittel der allgemeinen Formel VI

$R^{3'}$-L (VI)

in der $R^{3'}$ die oben angegebene Bedeutung hat und L für ein Halogenatom, insbesondere ein Chlor- oder Bromatom, die Gruppe -$OR^{3'}$, die Hydroxygruppe oder einen über ein Stickstoffatom gebundenen Rest eines Azols oder Benzazols mit mindestens zwei Stickstoffatomen im Fünfring steht, umgesetzt. Beispiele für die genannten Azole bzw. Benzazole sind der Imidazol-, 1,2,4-Triazol-, Tetrazol-, Benzimidazol- oder Benzotriazolring. Wird als Acylierungsmittel eine Verbindung der allgemeinen Formel VI verwendet, bei der L für die Gruppe -OH steht, so ist der Zusatz eines Aktivierungsmittels zweckmäßig, das die Aufgabe hat, das Acylierungspotential der Carbonsäure zu erhöhen. Geeignete derartige Mittel sind wasserentziehende bzw. wasserbindende Mittel, wie zum Beispiel Carbodiimide, oder solche Agenzien, welche die Carbonsäuren in die entsprechenden, als Acylierungsmittel wirkenden Säurehalogenide, Anhydride, gemischte Carbonsäure-Kohlensäure-Anhydride oder Azolide überführen, wie zum Beispiel Phosgen, Chlorameisensäureester oder N,N'-Carbonyldiimidazol. Die Umsetzung zwischen dem Acylierungsmittel der allgemeinen Formel VI und den Verbindungen der allgemeinen Formeln Ic und Id wird zweckmäßigerweise in einem inerten Lösungsmittel, zum Beispiel einem halogenierten Kohlenwasserstoff, einem Ether oder Lösungsmitteln, wie Pyridin oder Dimethylformamid, bei

Temperaturen zwischen -20°C und dem Siedepunkt des verwendeten Lösungsmittels durchgeführt. Das Molverhältnis zwischen dem Acylierungsmittel der Formel VI und den Verbindungen der allgemeinen Formeln Ic und Id beträgt normalerweise 3:1 bis 1:1, vorzugsweise 2:1 bis 1:1. Falls bei der Acylierungsreaktion eine Säure abgespalten wird, ist der Zusatz eines Säurefängers, wie zum Beispiel eines tertiären Amins, wie Triethylamin oder Pyridin, zweckmäßig.

Verbindungen der allgemeinen Formel I, bei denen $R^1$ und $R^2$ wie oben definiert sind und A für die Gruppe

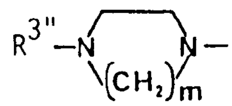

oder die Gruppe $R^{3'}$-NH-$(CH_2)_n$-NH- steht, worin m und n wie oben definiert sind und $R^{3''}$ für ein Wasserstoffatom steht, können schließlich dadurch hergestellt werden, daß man eine Verbindung der allgemeinen Formel I, in der $R^1$ und $R^2$ wie oben definiert sind und A für die Gruppen

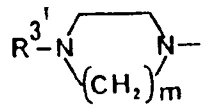

oder $R^{3'}$-NH-$(CH_2)_n$-NH- steht, worin $R^{3'}$ eine Cyanogruppe, eine Acylgruppe, eine gegebenenfalls substituierte Aroylgruppe oder eine $C_1$-$C_6$-Alkoxycarbonylgruppe ist, sauer oder basisch hydrolysiert und gegebenenfalls decarboxyliert.

Die saure Hydrolyse wird beispielsweise in wäßrigen Mineralsäuren, wie Chlor- oder Bromwasserstoffsäure oder Schwefelsäure, und bei erhöhten Temperaturen durchgeführt. In speziellen Fällen, zum Beispiel für $R^{3'}$ = tert.-Butoxycarbonyl, können aber auch schonendere Methoden, wie die Hydrolyse mit Trifluoressigsäure in einem Chlorkohlenwasserstoff, wie Dichlormethan oder Chloroform, Anwendung finden.

Die basische Hydrolyse erfolgt in verdünnten Lösungen von Alkali- oder Erdalkalicarbonaten bzw. Alkali- oder Erdalkalihydroxiden in Wasser, niedrigen Alkoholen oder Gemischen von beiden und bei Temperaturen von Raumtemperatur bis zur Rückflußtemperatur des verwendeten Lösungsmittels.

Die nach den einzelnen Verfahrensvarianten erhaltenen Verbindungen werden in üblicher Weise isoliert und gereinigt, beispielsweise durch Umkristallisation, chromatographische Arbeitsweisen usw.

Die bei den einzelnen Verfahrensvarianten erhaltenen Verbindungen können gegebenenfalls in ihr physiologisch annehmbares Salz umgewandelt werden. Diese Salze können zum Beispiel mit Mineralsäuren, wie Chlor-, Brom-, Jodwasserstoffsäure, Phosphorsäure, Metaphosphorsäure, Salpetersäure oder Schwefelsäure, oder mit organischen Säuren, wie Ameisensäure, Essigsäure, Propionsäure, Phenylessigsäure, Weinsäure, Zitronensäure, Fumarsäure, Methansulfonsäure, Embonsäure etc., gebildet werden.

Die erfindungsgemäßen Verbindungen der allgemeinen Formel I können sowohl in einer Reihe von tautomeren Formen als auch in mehreren stereoisomeren Formen vorliegen. Die Erfindung umfaßt daher neben den Salzen und Hydraten der oben beschriebenen Verbindungen der allgemeinen Formel I auch alle tautomeren und stereoisomeren Formen.

Die erfindungsgemäßen Verbindungen können zur Verabreichung in jeder beliebigen Weise formuliert werden. Die Erfindung umfaßt daher auch Arzneimittel, die mindestens eine erfindungsgemäße Verbindung zur Verwendung in der Human- oder Veterinärmedizin enthalten. Solche Arzneimittel können herkömmlicherweise unter Verwendung eines oder mehrerer pharmazeutisch annehmbarer Träger oder von Verdünnungsmitteln hergestellt werden.

Die erfindungsgemäßen Verbindungen können daher für die orale, bukkale, topische, parenterale oder rektale Verabreichung formuliert werden.

Für die orale Verabreichung kann das Arzneimittel in Form von beispielsweise Tabletten, Kapseln, Pulvern, Lösungen, Sirups oder Suspensionen vorliegen, die unter Verwendung von annehmbaren Verdünnungsmitteln auf herkömmliche Weise hergestellt worden sind.

Für die bukkale Verabreichung kann das Arzneimittel die Form von Tabletten oder Briefchen einnehmen, die in herkömmlicher Weise formuliert worden sind.

Die erfindungsgemäßen Verbindungen können für die parenterale Verabreichung durch Bolusinjektion oder kontinuierliche Infusion formuliert werden. Formulierungen zur Injektion können in Dosiseinheitsform als Ampullen oder in Mehrfachdosenbehältern mit zugesetztem Konservierungsmittel vorliegen.

Die Arzneimittel können solche Formen, wie Suspensionen, Lösungen oder Emulsionen in öligen oder wäßrigen Trägern, einnehmen, und sie können Formulierungshilfsmittel, wie Suspendierungs-, Stabilisierungs- und/oder Dispergierungsmittel, enthalten.

Alternativ kann der Wirkstoff auch in Pulverform zur Rekonstitution mit einem geeigneten Träger, zum Beispiel sterilem, pyrogenfreiem Wasser, vor dem Gebrauch vorliegen.

Die erfindungsgemäßen Verbindungen können auch für rektale Zubereitungen, zum Beispiel Suppositorien oder Retentionseinläufe, formuliert werden, die zum Beispiel herkömmliche Suppositoriengrundlagen, wie Kakaobutter oder andere Glyceride, enthalten.

Zur topischen Anwendung können die erfindungsgemäßen Verbindungen als Salben, Cremes, Gels, Lotionen, Pulver oder Sprays in herkömmlicher Weise formuliert werden.

Für die orale Verabreichung ist eine geeignete Tagesdosis an erfindungsgemäßen Verbindungen 1 bis 4 Dosen bis insgesamt 5 mg bis 1 g/Tag, je nach Zustand des Patienten. Im Einzelfall kann es gegebenenfalls erforderlich sein, von den genannten Mengen abzuweichen, und zwar in Abhängigkeit vom individuellen Verhalten gegenüber dem Wirkstoff bzw. der Art seiner Formulierung und dem Zeitpunkt bzw. Intervall, zu welchem die Verabreichung erfolgt. So gibt es zum Beisp.Fälle,in denen mit weniger als der oben genannten Mindestmenge ausgekommen werden kann, während in anderen Fällen die genannte obere Grenze überschritten werden muß.

Die erfindungsgemäßen substituierten 6-Phenyl-dihydro-3(2H)-pyridazinone der allgemeinen Formel I zeigen bei guter oraler Verfügbarkeit ausgeprägte cardiovaskuläre, insbesondere cardiotone und antihypertensive Wirkungen und eignen sich daher zur Behandlung und Vorbeugung von Erkrankungen des Herzens und des Kreislaufs.
So zeigen sie eine ausgezeichnete inotropiesteigernde Wirksamkeit in einer Reihe von pharmakologischen Standardmodellen, z.B. in einem in vivo-Modell am narkotisierten Meerschweinchen nach i.d.-Applikation, und eine blutdrucksenkende Wirkung an der spontan hypertensiven Ratte. Weiterhin besitzen einige dieser Verbindungen eine ausgeprägte spasmolytische Wirkung, so daß sie als krampflösende Mittel einsetzbar sind. Dies ist insbesondere bei Verbindungen der Fall, bei denen $R_1$ für eine Cyano-Gruppe steht und die anderen Gruppen wie oben definiert sind.

**1.) Hämodynamische Charakterisierung der positiv inotropen Wirkung am narkotisierten Meerschweinchen (i.d.-Applikation)**

a) Methode

Die Tiere werden mit Urethan (1,5 g/kg) narkotisiert. Zur volumenkontrollierten Beatmung wird die Trachea kanüliert. Danach erfolgt die operative Freilegung beider Karotiden, über die rechte Karotis wird ein Tip-Katheter (3F) eingeführt, der unter fortlaufender Druckregistrierung dann über die Aorta ascendens in den linken Ventrikel vorgeführt wird. Die erfolgreiche Passage der Aortenklappen wird hierbei durch die typische linksventrikuläre Druckkurve erkannt. Über die linke Karotis wird zur Thermodilution ein Thermistorfühler (3F, F. Edwards) in den Aortenbogen vorgeschoben. Der Thermistorfühler hat gleichzeitig ein Lumen zur arteriellen Blutdruckregistrieung. Zur Applikation des Kälteinjektats (0,2 ml 0.9 % NaCl, 15 °C) wird durch die rechte Vena jugularis ein Katheter vor den rechten Vorhof plaziert. Die Registrierung des EKG's erfolgt in der 1. Ableitung. Das Duodenum wird mittels eines 1 cm langen Medianschnitts im oberen Abdominalbereich freigelegt; die Testsubstanzen werden über eine Nadel in das Duodenum injiziert. Alle Substanzen sind in Tylose (Injektionsvolumen 1 ml/kg) suspendiert, die Applikation erfolgt nach hämodynamischer Stabilisierung und unter ß-Blockade (Metoprolol 2 mg/kg i.m.). Alle Kreislaufparameter werden kontinuierlich auf einem Direktschreiber registriert. Die Kontraktilität (dp/dt) wird über die Volumendruckkurve berechnet und die Herzfrequenz aus dem EKG ermittelt.

b) Meßwerte

| Beispiel Nr. | Dosis mg/kg | Maximale prozentuale Veränderungen gegenüber den Ausgangswerten | | |
|---|---|---|---|---|
| | | Kontraktilität dp/dt | Blutdruck systolisch | Herzfrequenz |
| 2 | 0,2 | + 70 % | - 20 % | + 13 % |
| 3 | 3,1 | + 40 % | - 20 % | + 10 % |
| 6 | 12,5 | + 67 % | - 21 % | + 7 % |
| 12 | 10 | + 70 % | - 25 % | + 25 % |
| Vergleich 1* | 50 | + 22 % | - 44 % | + 22 % |
| Vergleich 2** | 50 | + 58 % | - 40 % | + 48 % |

* Vergleich 1: 6-(4-Morpholino-3-nitro-phenyl)-4,5-dihydro-5-methyl-3(2H)-pyridazinon

** Vergleich 2: 6-[4-(4-Ethoxycarbonyl-piperazin-1-yl)-phenyl]-4,5-dihydro-3(2H)-pyridazinon (DE-OS 22 07 517)

2.) Antihypertensive Wirkung an der spontan hypertensiven Ratte

Es wurde die Literaturmethode nach I.B. Armah, Arzneimittelforsch. 27, 1882-1884 (1977) und M. Gerold u. H. Tschirky, Arzneimittelforsch. 18, 1285 (1968) angewendet. Die Substanzen werden oral verabreicht und die Blutdrucksenkung drei Stunden nach Substanzapplikation bestimmt.

| Bsp. Nr. | Dosis mg/kg | Blutdrucksenkung nach 3 Stunden |
|---|---|---|
| 2 | 5 | 82 % |
| Vergleich 1 | 5 | 17 % |
| Vergleich 2 | 5 | 29 % |

Beispiel 1

6-[3-Nitro-4-(1-piperazinyl)-phenyl]-4,5-dihydro-5-methyl-3(2H)-pyridazinon

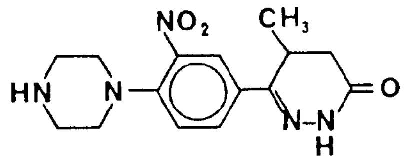

21,4 g (80 mmol) 6-(4-Chlor-3-nitro-phenyl)-4,5-dihydro-5-methyl-3(2H)-pyridazinon werden mit 41,35 g (480 mmol) Piperazin in 120 ml Dioxan 5 h unter Rückfluß gekocht.
Nach dem Abkühlen gießt man auf 400 ml Wasser, rührt 1 h bis zur Vervollständigung der Kristallisation, saugt ab und wäscht mit Wasser nach.
Durch Umkristallisieren aus Methoxyethanol/Diethylether erhält man 19,4 g (76 % d. Th.) eines orangeroten Pulvers vom Schmelzpunkt 224-225°C.
$C_{15}H_{19}N_5O_3$ (317,35)
Rf = 0,40 (Dichlormethan/Methanol/Triethylamin 90/7/3)

Beispiel 2

6-[4-(4-Ethoxycarbonyl-piperazin-1-yl)-3-nitro-phenyl]-4,5-dihydro-5-methyl-3(2H)-pyridazinon

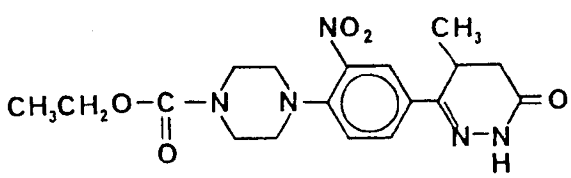

Zu einer Lösung von 15,0 g (56 mmol) 6-(4-Chlor-3-nitro-phenyl)-4,5-dihydro-5-methyl-3(2H)-pyridazinon in 50 ml Dimethylformamid werden 24,5 ml (168 mmol) 1-Ethoxycarbonyl-piperazin gegeben und die Lösung 3 Stunden bei 100°C Innentemperatur gerührt. Das erkaltete Reaktionsgemisch wird unter starkem Rühren auf 300 ml Eiswasser gegossen. Der ausgefallene Feststoff wird abgesaugt, mit etwas Ethanol und Diethylether gewaschen und aus 500 ml Ethanol umkristallisiert. Man erhält 16,7 g (76 %) orangegelbe Kristalle vom Schmp. 140-142°C.
$C_{18}H_{23}N_5O_5$ (389,41)
Rf = 0,50 (Dichlormethan/Methanol 95/5)

Beispiel 3

6-[4-(4-Ethoxycarbonyl-piperazin-1-yl)-3-nitro-phenyl]-4,5-dihydro-3(2H)-pyridazinon

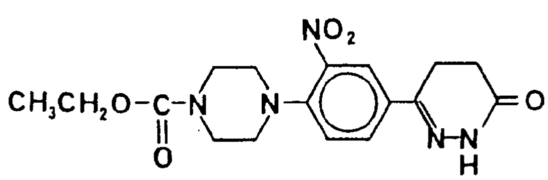

Analog zu Beispiel 2 werden aus 7,61 g (30 mmol) 6-(4-Chlor-3-nitro-phenyl)-4,5-dihydro-3(2H)-pyridazinon und 14,25 g (90 mmol) 1-Ethoxycarbonyl-piperazin 10,14 g (90%) orangegelbe Kristalle vom Schmp. 203-204°C erhalten.
$C_{17}H_{21}N_5O_5$ (375,39)

Rf = 0,18 (Dichlormethan/Methanol 97/3)

Beispiel 4

6-[4-(4-Benzyl-piperazin-1-yl)-3-nitro-phenyl]-4,5-dihydro-5-methyl-3(2H)-pyridazinon

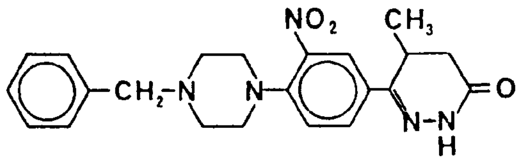

16,1 g (60 mmol) 6-(4-Chlor-3-nitro-phenyl)-4,5-dihydro-5-methyl-3(2H)-pyridazinon, 15,9 g (90 mmol) 1-Benzylpiperazin und 7,3 ml (90 mmol) Pyridin werden in 60 ml Dimethylformamid bei 100°C gerührt. Nach 7 Stunden werden weitere 5,8 g (33 mmol) 1-Benzyl-piperazin zugesetzt und das Reaktionsgemisch 12 Stunden bei 100°C weitergerührt. Das erkaltete Reaktionsgemisch wird auf 300 ml Eiswasser gegossen und der ausgefallene Feststoff abgesaugt. Die wässrige Phase wird zweimal mit 200 ml Dichlormethan extrahiert; die vereinigten organischen Phasen werden mit Wasser und gesättigter Natriumchlorid-Lösung gewaschen, getrocknet, filtriert und i. Vak. eingedampft. Der dabei erhaltene feste Rückstand wird zusammen mit dem anfangs erkalteten Feststoff aus 500 ml Ethanol umkristallisiert. Man erhält 23,4 g (95 %) orangerote Kristalle vom Schmp. 220-221°C.
$C_{22}H_{25}N_5O_3$ (407,48)
Rf = 0,50 (Dichlormethan/Methanol 95/5)

Beispiel 5

6-[3-Nitro-4-[4-(2-pyrimidyl)-piperazin-1-yl]phenyl]-4,5-dihydro-5-methyl-3(2H)-pyridazinon

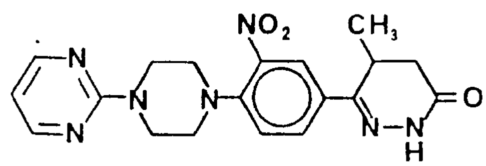

3,0 g (11,2 mmol) 6-(4-Chlor-3-nitro-phenyl)-4,5-dihydro-5-methyl-3(2H)-pyridazinon, 3,7 g (22,4 mmol) 1-(2-Pyrimidyl)-piperazin und 0,9 g (11,2 mmol) Pyridin werden in 50 ml Dioxan unter Rückfluß gekocht. Nach 6 Stunden werden nochmals 1,9 g (11,6 mmol) 1-(2-Pyrimidyl)-piperazin zugesetzt und das Reaktionsgemisch weitere 4 Stunden gekocht. Das abgekühlte Reaktionsgemisch wird auf 300 ml Eiswasser gegossen, der ausgefallene Feststoff abgesaugt, mit etwas Diethylether nachgewaschen und aus 40 ml 2-Methoxyethanol umkristallisiert. Man erhält 3,1 g (70 %) orangerote Kristalle vom Schmp. 232-233°C.
$C_{19}H_{21}N_7O_3$ (395,42)
Rf = 0,60 (Dichlormethan/Methanol 95/5)

Beispiel 6

6-[4-(4-Acetyl-piperazin-1-yl)-3-nitro-phenyl]-5-methyl-4,5-dihydro-3(2H)-pyridazinon

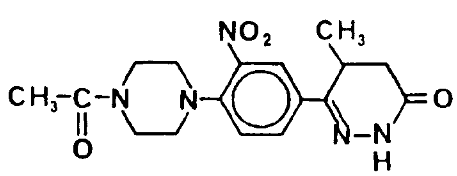

a) 1,27 g (4 mmol) 6-[3-Nitro-4-(1-piperazinyl)-phenyl]-4,5-dihydro-5-methyl-3(2H)-pyridazinon in 30 ml Essigsäure werden bei RT mit 0,57 ml (6 mmol) Essigsäureanhydrid versetzt.
Man erhitzt 10 min auf 60°C, läßt abkühlen, gießt auf Eiswasser und extrahiert mit Chloroform. Die Chloroform-Phase wird mit wäßriger $NaHCO_3$-Lösung gewaschen und im Vakuum eingeengt. Der feste Rückstand wird aus 20 ml Methoxyethanol umkristallisiert. Man erhält 0,8 g (56 % d. Th.) der Titelverbindung als orangefarbenes Pulver vom Schmelzpunkt 240-241°C.
$C_{17}H_{21}N_5O_4$ (359,31)
Rf = 0,55 (Dichlormethan/Methanol 95/5)
b) 2,68 g (10 mmol) 6-(4-Chlor-3-nitro-phenyl)-4,5-dihydro-5-methyl-3(2H)-pyridazinon und 3,84 g (30 mmol) 1-Acetylpiperazin werden in 20 ml Dimethylformamid 4 Stunden bei 100°C Innentemperatur gerührt. Das abgekühlte Reaktionsgemisch wird auf 200 ml Eiswasser gegossen, der entstandene Feststoff abgesaugt und aus Methoxyethanol umkristallisiert. Man erhält 2,30 g (64 %) orangefarbene Kristalle vom Schmp. 240-241°C, identisch mit dem unter a) beschriebenen Produkt.

Beispiel 7

6-[4-(4-Formyl-piperazin-1-yl)-3-nitro-phenyl]-4,5-dihydro-5-methyl-3(2H)-pyridazinon

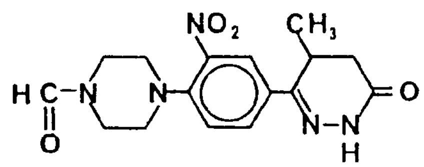

Analog zu Beispiel 6 b) erhält man aus 2,68 g (10 mmol) 6-(4-Chlor-3-nitro-phenyl)-4,5-dihydro-5-methyl-3(2H)-pyridazinon und 3,42 g (30 mmol) 1-Formylpiperazin 2,35 g (68 %) orangerote Kristalle vom Schmp. 216,5°C (aus Chloroform/Methanol 1:3)
$C_{16}H_{19}N_5O_4$ (345.36)

| | | | |
|---|---|---|---|
| Ber.: | C 55,65 | H 5,55 | N 20,28 |
| Gef.: | C 55,70 | H 5,56 | N 20,25 |

Rf = 0,62 (Dichlormethan/Methanol/konz. Ammoniak 90/7/3)

Beispiel 8

6-[4-[4-(4-Methoxybenzoyl)-piperazin-1-yl]-3-nitro-phenyl]-5-methyl-4,5-dihydro-3(2H)-pyridazinon

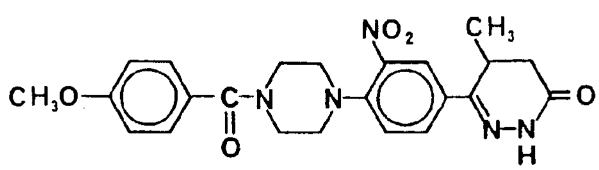

Eine Aufschlämmung von 1.0 g (3,15 mmol) 6-[3-Nitro-4-(1-piperazinyl)-phenyl]-5-methyl-4,5-dihydro-3(2H-pyridazinon und 1,14 ml (8,2 mmol) Triethylamin in 40 ml 1,2-Dichlorethan wird mit 0,70 g (4,1 mmol) 4-Methoxybenzoylchlorid versetzt und 0,5 h unter Rückfluß gekocht. Man läßt auf RT abkühlen, versetzt mit 50 ml Wasser und trennt die organische Phase ab.
Nach dem Einengen der organischen Phase wird der Rückstand an Kieselgel chromatographiert (Laufmittel Dichlormethan/Methanol 97/3). Man erhält 0,69 g (49 % d. Th.) der Titelverbindung als orangegelbe Kristalle vom Schmelzpunkt 200-201°C.
$C_{23}H_{25}N_5O_5$ (451,48)
Rf = 0,44 (Dichlormethan/Methanol 95/5)

Beispiel 9

6-[4-(4-Cyano-piperazin-1-yl)-3-nitro-phenyl]-5-methyl-4,5-dihydro-3(2H)-pyridazinon

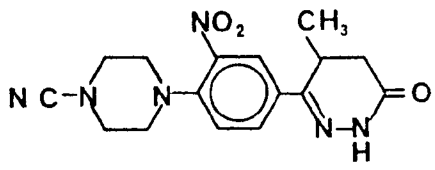

1,58 g (5 mmol) 6-[3-Nitro-4-(1-piperazinyl)-phenyl]-5-methyl-4,5-dihydro-3(2H)-pyridazinon werden in 60 ml Chloroform aufgeschlämmt und bei 10°C tropfenweise mit einer Lösung von 0,64 g (6 mmol) Bromcyan in 10 ml Chloroform versetzt.
Nach 0,5 h versetzt man das Reaktionsgemisch nacheinander mit 40 ml Wasser und einer Lösung von 2 g Kaliumcarbonat in 5 ml Wasser. Es wird noch 1 h gerührt, währenddessen die Titelverbindung auskristallisiert.

Man saugt vom Feststoff ab, wäscht mit Wasser und Aceton und kristallisiert aus Aceton/1,2-Dichlorethan um.

Man erhält 0,96 g (56 % d.Th.) orangerote Kristalle vom Schmelzpunkt 247-248°C.
$C_{16}H_{18}N_6O_3$ (342,36)
Rf = 0,45 (Dichlormethan/Methanol 97/3)

Beispiel 10

6-[4-(2-tert.-Butoxycarbonylamino)ethylamino-3-nitro-phenyl]-4,5-dihydro-5-methyl-3(2H)-pyridazinon

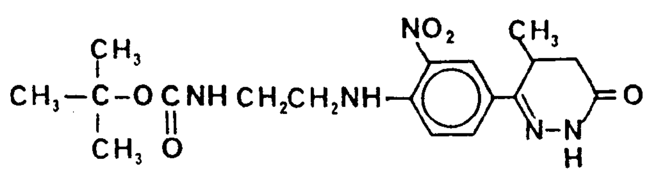

2,68 g (10 mmol) 6-(4-Chlor-3-nitro-phenyl)-4,5-dihydro-5-methyl-3(2H)-pyridazinon und 4,80 g (30 mmol) N-tert.-Butoxycarbonylethylendiamin werden in 20 ml Dimethylformamid 3,5 Stunden bei 80°C gerührt. Die erkaltete Reaktionsmischung wird in 150 ml Wasser eingegossen und der ausfallende Feststoff abgesaugt. Nach Umkristallisieren aus Dichlormethan verbleiben 1,70 g (43 %) orangerote Kristalle vom Schmp. 225-226°C.
$C_{18}H_{25}N_5O_5$ (391,43)
Rf = 0,68 (Dichlormethan/Methanol 9/1)

Beispiel 11

6-[4-(3-tert.-Butoxycarbonylamino)propylamino-3-nitro-phenyl]-4,5-dihydro-5-methyl-3(2H)-pyridazinon

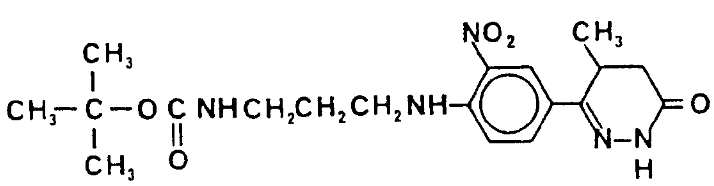

Analog zu Beispiel 10) erhält man aus 2,68 g (10 mmol) 6-(4-Chlor-3-nitro-phenyl)-4,5-dihydro-5-methyl-3(2H)-pyridazinon und 5,22 g (30 mmol) N-tert.-Butoxycarbonyl-1,3-propandiamin nach chromatographischer Reinigung an Kieselgel mit Dichlormethan/Methanol (90:10) als Laufmittel und Umkristallisation aus Dichlormethan 0,92 g (23 %) eines orangeroten Feststoffes vom Schmp. 172-173°C.
$C_{19}H_{27}N_5O_5$ (405,46)
Rf = 0,65 (Dichlormethan/Methanol 9/1)

Beispiel 12

6-[3-Amino-4-(4-ethoxycarbonyl-piperazin-1-yl)-phenyl]-4,5-dihydro-5-methyl-3(2H)-pyridazinon

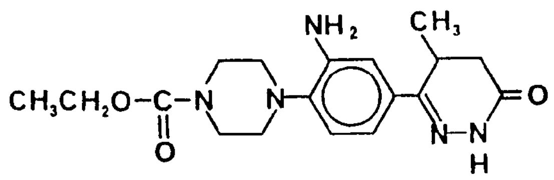

13,7 g (35 mmol) 6-[4-(Ethoxycarbonyl-piperazin-1-yl)-3-nitro-phenyl]-4,5-dihydro-5-methyl-3(2H)-pyridazinon in 500 ml Methanol werden in Gegenwart von 1,0 g Palladium auf Aktivkohle (10 % Pd) bei 55°C und einem Wasserstoffdruck von 6 bar eine Stunde hydriert. Nach Absaugen des Katalysators wird die Lösung i. Vak. weitgehend eingeengt und der Rückstand mit 50 ml eines Gemisches aus Diethylether/Ethylacetat (2:1) verrührt. Der ausgefallene Feststoff wird abgesaugt und aus Ethanol umkristallisiert. Man erhält 8,6 g (68 %) eines hellgelben Feststoffs vom Schmp. 193-195°C.
$C_{18}H_{25}N_5O_3$ (359,43)
Rf = 0,45 (Dichlormethan/Methanol 95/5)

Beispiel 13

6-[4-(2-Aminoethyl)amino-3-nitro-phenyl]-4,5-dihydro-5-methyl-3(2H)-pyridazinon

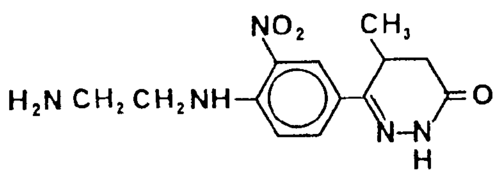

1,00 g (2,55 mmol) 6-[4-(2-tert.-Butoxycarbonylamino)ethylamino-3-nitro-phenyl]-4,5-dihydro-5-methyl-3( 2H)-pyridazinon in 10 ml Dichlormethan werden mit 1,0 ml (13,0 mmol) Trifluoressigsäure versetzt und die Lösung 2 Stunden unter Rückfluß gekocht. Nach Abkühlen auf Raumtemperatur wird das Reaktionsgemisch mit 10 ml Dichlormethan verdünnt und der ausgefallene Feststoff abfiltriert. Der Feststoff wird in 20 ml gesättigte Natriumhydrogencarbonat-Lösung eingetragen. Der sich dabei abscheidende Feststoff wird abfiltriert und aus Isopropanol umkristallisiert. Man erhält 0,44 g (59 %) eines orangeroten Feststoffs vom Schmp. 162-164°C.
$C_{13}H_{17}N_5O_3$ (291,31)
Rf = 0,65 (Ethylacetat/Ethanol/konz. Ammoniak 15/10/2)

Beispiel 14

6-[4-(3-Aminopropyl)amino-3-nitro-phenyl]-4,5-dihydro-5-methyl-3(2H)-pyridazinon

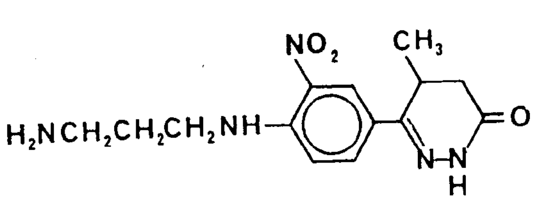

2,0 g (4,9 mmol) 6-[4-(3-tert.-Butoxycarbonylamino)propylamino-3-nitro-phenyl]-4,5-dihydro-5-methyl-3(2H)-pyridazinon und 2,0 ml (26,1 mmol) Trifluoressigsäure werden in 30 ml Chloroform 12 Stunden unter Rückfluß gekocht. Das nach Abdampfen des Lösungsmittels i.Vak. erhaltene Öl wird in 10 ml gesättigter Kaliumcarbonat-Lösung aufgenommen und die wässrige Phase dreimal mit jeweils 20 ml Isopropanol extrahiert. Die vereinigten organischen Phasen werden mit Natriumsulfat getrocknet, filtriert und i. Vak. eingedampft. Der erhaltene Rückstand mit an Kieselgel mit Dichlormethan/Methanol (9:1) als Laufmittel chromatographiert. Nach Eindampfen der Hauptfraktion i. Vak. und Umkristallisieren aus Isopropanol erhält man 1,0 g (66 %) eines orangeroten Feststoffs vom Schmp. 154-156°C.
$C_{14}H_{19}N_5O_3$ (305,33)
Rf = 0,46 (Ethylacetat/Ethanol/konz. Ammoniak 15/10/2)

Beispiel 15

6-[4-(4-Ethoxycarbonyl-piperidin-1-yl)-3-nitro-phenyl]-5-methyl-4,5-dihydro-3(2H)-pyridazinon

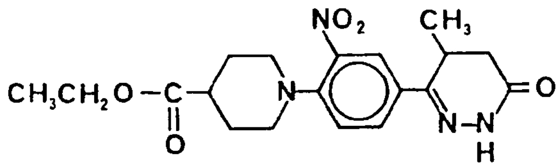

2,68 g (10 mmol) 6-(4-Chlor-3-nitro-phenyl)-5-methyl-4,5-dihydro-3(2H)-pyridazinon werden mit 3,14 g (20 mmol) Piperidin-4-carbonsäureethylester und 0,81 ml (10 mmol) Pyridin in 50 ml Dioxan 5 h unter Rückfluß erhitzt. Man gießt auf 200 ml Eiswasser, saugt vom Feststoff ab und wäscht den Filterkuchen mit Wasser nach. Nach Umkristallisation aus 100 ml Ethanol erhält man 2,8 g (72 % d. Th.) der Titelverbindung als orangefarbene Nädelchen vom Schmelzpunkt 159,5-160,5°C.
$C_{19}H_{24}N_4O_5$
Rf = 0,45 (Dichlormethan/Methanol 97/3)

Beispiel 16

6-[3-Amino-4-(2-tert.-butoxycarbonylamino)ethylamino-phenyl]-4,5-dihydro-5-methyl-3(2H)-pyridazinon

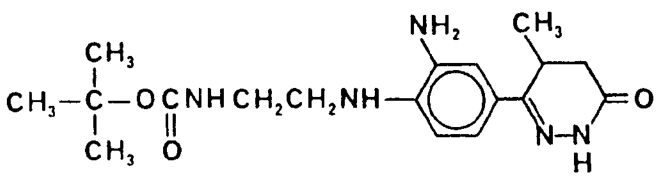

2,5 g (6,4 mmol) 6-[4-(2-tert.-Butoxycarbonylamino)ethylamino-3-nitro-phenyl]-4,5-dihydro-5-methyl-3(2H)-pyridazinon werden in Gegenwart von 0,5 g Palladium-Kohle (10 % Pd) bei Raumtemperatur und einem Wasserstoffdruck von 5 bar hydriert. Nach beendeter Wasserstoffaufnahme wird der Katalysator abgesaugt und das Filtrat i.Vak. eingedampft. Der Rückstand wird durch Chromatographie an Kieselgel mit Dichlormethan/Methanol (95:5) als Laufmittel gereinigt. Die Hauptfraktion ergibt nach Eindampfen i.Vak. 1,6 g (69 %) eines grünlichen, amorphen Feststoffs.
$C_{18}H_{27}N_5O_3$ (361,44)
Rf = 0,64 (Dichlormethan/Methanol 9 / 1)

Beispiel 17

1-[4-(5-Methyl-3-oxo-2,3,4,5-tetrahydro-6-pyridazinyl)-2-nitro-phenyl]-piperidin-4-carbonsäure

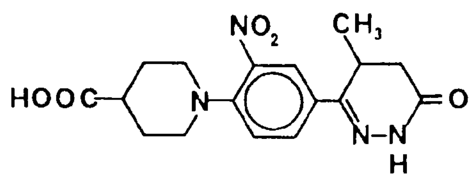

1,2g (3,1 mmol) 1-[2-Nitro-4-(5-methyl-3-oxo-2,3,4,5-tetrahydro-6-pyridazinyl)-phenyl]-piperidin-4-carbonsäureethylester werden in einer Lösung von 0,4 g (9,3 mmol) Natriumhydroxid in 40 ml Methoxyethanol unter Rückfluß erhitzt.
Nach 2,5 h gießt man auf 150 ml Eiswasser und wäscht die Wasserphase mit insgesamt 150 ml Dichlormethan. Nach dem Ansäuern der Wasserphase bis pH 4,5 kristallisiert die Titelverbindung aus.
Die Umkristallisation aus Methanol ergibt 0,6 g (54 % d. Th.) eines orangefarbenen Pulvers mit einem Schmelzpunkt von 213,6 - 214,5 °C.
$C_{17}H_{20}N_4O_5$ (360,37)
Rf = 0,23 (Chloroform/Ethanol/Essigsäure 96/2/2)

Beispiel 18

6-[3-Chlor-4-(4-ethoxycarbonylpiperazin-1-yl-phenyl]-4,5-dihydro-5-methyl-3(2H)-pyridazinon

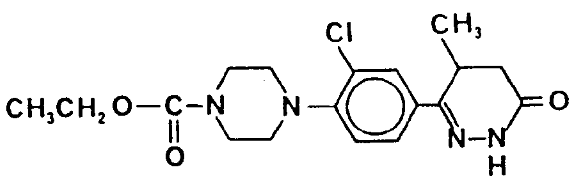

Zu einer Lösung von 3,0 g (8,3 mmol) 6-[3-Amino-4-(4-ethoxycarbonylpiperazin-1-yl)-phenyl]-4,5-dihydro-5-methyl-3(2H)-pyridazinon in 7 ml konz. Salzsäure und 3 ml Wasser wird bei -5 °C während 10 Minuten eine eiskalte Lösung von 0,9 g (8,5 mmol) Natriumnitrit in 5 ml Wasser getropft. Das erhaltene Reaktionsgemisch wird dann zu einer 0 °C kalten Lösung von 1,29 g (13 mmol) Kupfer-(I)-chlorid in 5 ml konz. Salzsäure gegeben. Nach beendeter Gasentwicklung wird die Lösung langsam auf 50 °C erwärmt, dann abgekühlt und mit Kaliumcarbonat auf pH 11 eingestellt. Die wässrige Phase wird dreimal mit 50 ml Dichlormethan extrahiert, die organischen Phasen werden getrocknet, filtriert und i. Vak. eingedampft. Der erhaltene Rückstand wird an Kieselgel mit Dichlormethan/Methanol (95:5) chromatographiert und ergibt nach Eindampfen der lipophilen Hauptfraktion i. Vak. 1,2 g (38 %) eines farblosen Feststoffs vom Schmp. 154 - 156 °C.
$C_{18}H_{23}ClN_4O_3$ (378,86)
Rf = 0,61 (Dichlormethan/Methanol 95/5)

Beispiel 19

6-[4-[3-(4-Imidazolyl)-propylamino]-3-nitro-phenyl]-5-methyl-4,5-dihydro-3(2H)-pyridazinon

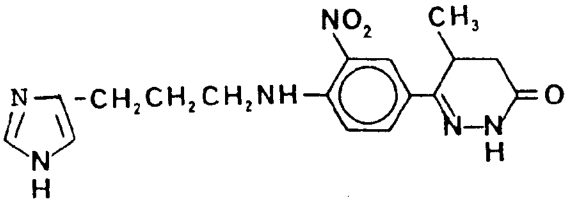

2,67 g (10 mmol) 6-(4-Chlor-3-nitro-phenyl)-5-methyl-4,5-dihydro-3(2H)-pyridazinon werden mit 2,5 g (20

mmol) 4-(3-Aminopropyl)-1H-imidazol in 50 ml Dioxan 5 h unter Rückfluß erhitzt.
Man gießt auf 300 ml Eiswasser, rührt noch 1 h nach und saugt vom ausgefallenen Feststoff ab.
Die Umkristallisation aus Aceton ergibt 2,05 g (58 % d. Th.) als organgefarbene Kristalle vom Schmelzpunkt 162,7 - 163,5 °C.
$C_{17}H_{20}N_6O_3$ (356,39)
Rf = 0,67 (Dichlormethan/Methanol/Ammoniak 85/13/2)

Beispiel 20

6-[3-Nitro-4-(1-piperazinyl)-phenyl]-4,5-dihydro-3(2H)-pyridazinon

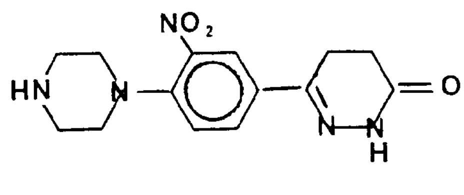

10,0 g (39,4 mmol) 6-(4-Chlor-3-nitro-phenyl)-4,5-dihydro-3(2H)-pyridazinon werden mit 10,2 g (118,2 mmol) Piperazin 5 h unter Rückfluß gekocht.
Nach Abkühlen auf 60°C gießt man auf Eiswasser, saugt vom Feststoff ab und kristallisiert aus Ethanol um.
Man erhält 8,8 g (74 % d.Th.) orangeroter Kristalle vom Schmp. 194,7-196,1°C.
$C_{14}H_{17}N_5O_3$ (303,32)
Rf = 0,31 (Dichlormethan/Methanol/Triethylamin 90/7/3)

Beispiel 21

6-[4-(2-Aminoethyl)amino-3-amino-phenyl]-4,5-dihydro-5-methyl-3(2H)-pyridazinon

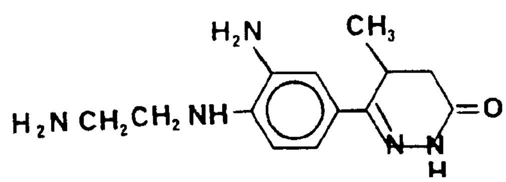

3,0 g (10,3 mmol) 6-[4-(2-Aminoethyl)amino-3-nitro-phenyl]-4,5-dihydro-5-methyl-3(2H)-pyridazinon in 200 ml Methanol werden in Gegenwart von 0,5 g Palladiumkohle (10 % Pd) bei Raumtemperatur und 2 bar Wasserstoffdruck bis zur beendeten Wasserstoffaufnahme hydriert. Nach Abfiltrieren des Katalysators wird die Lösung i. Vak. eingedampft. Der Rückstand wird an Kieselgel mit Methanol/konz. Ammoniak (95/5) als Laufmittel chromatographiert. Die Hauptfraktion wird i. Vak. eingedampft und der erhaltene Feststoff aus Ethanol umkristallisiert. Man erhält 1,37 g (51%) eines beigen Feststoffs vom Schmp. 176-177°C.
$C_{13}H_{19}N_5O$ (261,32)
Rf = 0,34 (Methanol/konz. Ammoniak 95/5)

Beispiel 22

6-[3-Amino-4-(4-ethoxycarbonyl-piperazin-1-yl)-phenyl]-4,5-dihydro-3(2H)-pyridazinon

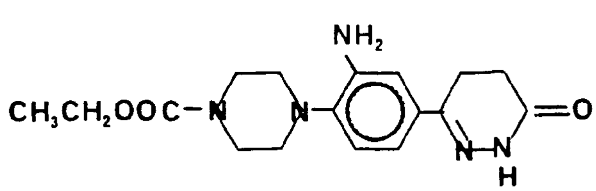

9,0 g (24 mmol) 6-[4-(4-Ethoxycarbonyl-piperazin-1-yl)-3-nitro-phenyl]-4,5-dihydro-3(2H)-pyridazinon werden in 150 ml Eisessig in Gegenwart von 1 g Pd/A-Kohle (10%) bei 6 bar und 35°C hydriert.
Nach beendeter $H_2$-Aufnahme filtriert man ab, engt im Vakuum ein und behandelt den Rückstand mit Ethylacetat und überschüssiger wässriger $NaHCO_3$-Lösung.
Man saugt vom Niederschlag ab, kristallisiert diesen aus Isopropanol um und erhält 4,35 g (52 % d. Th.) farbloser Kristalle vom Schmp. 201°C.
$C_{17}H_{23}N_5O_3$ (345,40)
Rf = 0,46 (Dichlormethan/Methanol 95/5)

Beispiel 23

6-[3-Amino-4-(1-piperazinyl)-phenyl]-5-methyl-4,5-dihydro-3(2H)-pyridazinon

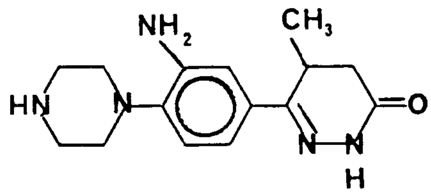

10,0 g (31,5 mmol) 5-Methyl-6-[3-nitro-4-(1-piperazinyl)-phenyl]-4,5-dihydro-3(2H)-pyridazinon werden analog Beispiel 22 hydriert. Die Kristallisation aus Isopropanol ergibt 3,6 g (40 % d.Th.) der Titelverbindung vom Schmelzpunkt 244,4-244,9°C.
$C_{15}H_{21}N_5O$ (287,37)
Rf = 0,24 (Dichlormethan/Methanol/Triethylamin 90/7/3)

Beispiel 24

6-[4-[4-(3-Aminopropyl)-piperazin-1-yl]-3-nitro-phenyl]-5-methyl-4,5-dihydro-3(2H)-pyridazinon

a) 5-Methyl-6-[3-nitro-4-[4-(3-phthalimidopropyl)-piperazin-1-yl]-phenyl]-4,5-dihydro-3(2H)-pyridazinon

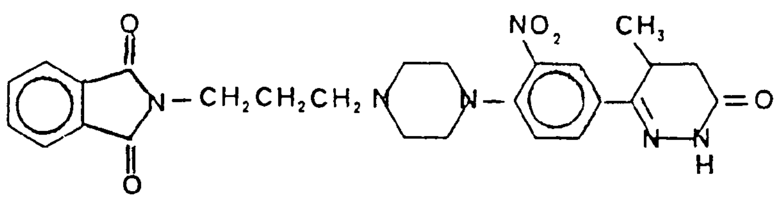

Ein Gemisch aus 3,16 g (10 mmol) 5-Methyl-6-[3-nitro-4-(1-piperazinyl)-phenyl]-4,5-dihydro-3(2H)-pyridazinon, 2,68 g (10 mmol) N-(3-Brompropyl)-phthalimid, 1,38 g (10 mmol) Kaliumcarbonat und 50 ml Dimethylformamid wird 24 h bei 100° C gerührt.
Man gießt auf 200 ml Eiswasser, rührt 1 h nach und saugt vom ausgefallenen Feststoff ab.
Die Umkristallisation aus 300 ml Ethanol ergibt 3,2 g (64 % d. Th.) eines orangeroten Pulvers vom Schmelzpunkt 179,4-179,9°C.
$C_{26}H_{28}N_6O_5$ (504,55)
Rf = 0,28 (Dichlormethan/Methanol 95/5)

b) 6-[4-[4-(3-Aminopropyl)-piperazin-1-yl]-3-nitro-phenyl]-5-methyl-4,5-dihydro-3(2H)-pyridazinon

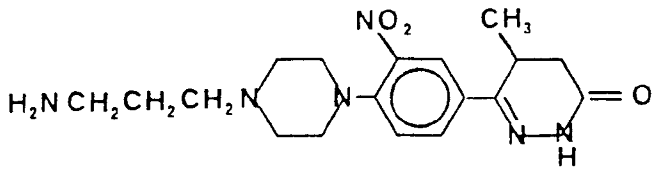

40,4 g (80 mmol) der vorstehenden Verbindung werden mit 30 ml Hydrazinhydrat (80 % in Wasser) in 700 ml Ethanol 5 h unter Rückfluß gekocht. Das Ethanol wird im Vakuum weitgehend abdestilliert, der Rückstand mit 200 ml $H_2O$ versetzt und mit verd. Salzsäure bis pH 2 angesäuert.

Man saugt vom ausgefallenen Feststoff ab, versetzt das Filtrat mit Natriumhydroxid bis pH 14 und rührt im Eisbad bis zur Vervollständigung der Kristallisation.

Das Kristallisat wird zweimal aus Isopropanol umkristallisiert. Man erhält 16,2 g (54 % d. Th.) eines orangefarbenen Pulvers vom Schmp. 110-110,5° C.

$C_{18}H_{26}N_6O_3$ (374,45)

Rf = 0,27 (Dichlormethan/Methanol/Ammoniak 85/13/2)

Beispiel 25

6-[4-(2-Acetamidoethyl)amino-3-nitro-phenyl]-4,5-dihydro-5-methyl-3(2H)-pyridazinon

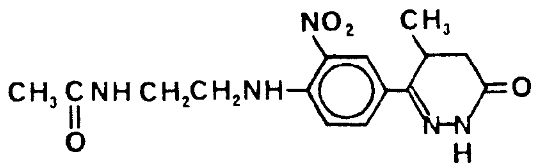

Eine Lösung von 1,00 g (3,43 mmol) 6-[4-(2-Aminoethyl)amino-3-nitro-phenyl]-4,5-dihydro-5-methyl-3(2H)-pyridazinon in 10 ml Eisessig wird mit 0,40 ml (4,23 mmol) Acetanhydrid versetzt und 20 Stunden bei Raumtemperatur gerührt. Der ausgefallene Feststoff wird abgesaugt und mit 5 ml Dichlormethan gewaschen. Die Mutterlauge wird i. Vak. eingeengt, der erhaltene Rückstand mit 5 ml Dichlormethan verrührt und filtriert. Die beiden Kristallfraktionen werden zusammen aus Ethanol (99,7 %) umkristallisiert. Man erhält 0,86 g (75 %) orangefarbene Kristalle vom Schmp. 222°C.

$C_{15}H_{19}N_5O_4$ (333,35)

Rf = 0,60 (Dichlormethan/Methanol 90/10)

Beispiel 26

6-[4-(4-Ethoxycarbonyl-piperazin-1-yl)-3-nitro-phenyl]-4,5-dihydro-5-hydroxymethyl-3(2H)-pyridazinon

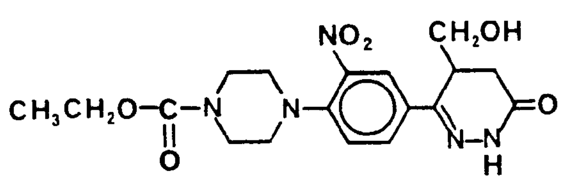

a) 4-[4-Chlor-3-nitro-benzoyl)-tetrahydrofuran-2-on

3,37 g (15 mmol) 4-[4-Chlorbenzoyl)-tetrahydrofuran-2-on werden bei -15°C portionsweise zu 30 ml Salpetersäure (100 %) gegeben. Die Temperatur soll -5°C nicht übersteigen. Nach beendeter Zugabe rührt man noch 30 min, gießt auf 300 ml Eiswasser, saugt vom ausgefallenen Feststoff ab und wäscht mit Wasser. Der Filterkuchen wird aus 100 ml Ethanol umkristallisiert. Man erhält 3,26 g (80 % d. Th.) nahezu farbloser

Kristalle vom Schmp. 118-120°C.
$C_{11}H_8ClNO_5$ (269,64)
Rf = 0,36 (Ethylacetat/Petrolether 60/40)

b) 6-(4-Chlor-3-nitro-phenyl)-4,5-dihydro-5-hydroxymethyl-3(2H)-pyridazinon

21,6 g (0,08 mol) des wie unter a) erhaltenen Tetrahydrofuranons werden mit 25 ml (0,4 mol) Hydrazinhydrat (80 % in Wasser) in 200 ml Eisessig 2 h bei 80°C gerührt.
Man läßt das Reaktionsgemisch abkühlen und rührt zur Vervollständigung der Kristallisation im Eisbad. Nach dem Absaugen erhält man 20,5 g (82 % d. Th.) eines schwach gelblichen Feststoffs vom Schmp. 236-238°C.
$C_{11}H_{10}ClN_3O_4$ (283,67)
Rf = 0,41 (Dichlormethan/Methanol 95/5)

c) 6-[4(4-Ethoxycarbonyl-piperazin-1-yl)-3-nitro-phenyl]-4,5-dihydro-5-hydroxymethyl-3(2H)-pyridazinon

3,12 g (10 mmol) des Pyridazinons aus Stufe b) werden analog Beispiel 1) mit 7,91 g (50 mmol) 1-Ethoxycarbonylpiperazin umgesetzt.
Nach Umkristallisation des Rohprodukts aus Ethanol erhält man 2,0 g (49 % d. Th.) eines orangefarbenen Pulvers vom Schmp. 163,5-164°C.
$C_{18}H_{23}N_5O_6$ (405,41)
Rf = 0,30 (Ethylacetat/Methanol 95/5)

Beispiel 27

6-[3-Nitro-4-(1-piperazinyl)-phenyl]-4,5-dihydro-5-hydroxymethyl-3(2H)-pyridazinon

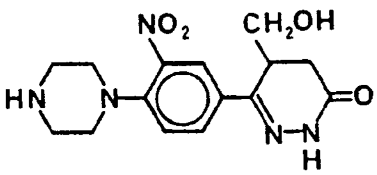

19,86 g (0,07 mol) 6-(4-Chlor-3-nitro-phenyl)-4,5-dihydro-5-hydroxymethyl-3(2H)-pyridazinon werden analog Beispiel 1) mit 18,1 g (0,21 mol) Piperazin umgesetzt.
Nach der Umkristallisation aus Ethanol erhält man 16,17 g (69 % d. Th.) eines orangeroten Pulvers vom Schmp. 182-184°C.
$C_{15}H_{19}N_5O_4$ (333,35)
Rf = 0,1 (Ethylacetat(Methanol/$NH_3$ 80/12/2)

Beispiel 28

6-[3-Cyano-4-(4-ethoxycarbonyl-piperazin-1-yl)phenyl]-4,5-dihydro-5-methyl-3(2H)-pyridazinon

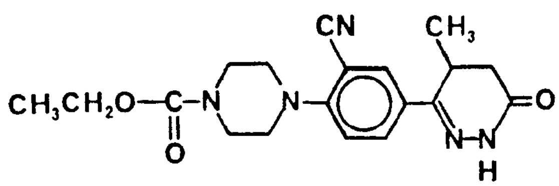

a) 6-(4-Chlor-3-cyano-phenyl)-4,5-dihydro-5-methyl-3(2H)-pyridazinon

Eine Suspension von 5,0 g (21 mmol) 6-(3-Amino-4-chlor-phenyl)-4,5-dihydro-5-methyl-3(2H)-pyridazinon in 160 ml Wasser und 16 ml konz. Salzsäure wird mit einer Lösung von 1,5 g (21 mmol) Natriumnitrit in 1o ml Wasser bei 0°C diazotiert. Anschließend wird die Diazoniumsalz-Lösung mit $K_2CO_3$ neutralisiert und bei 0°C portionsweise zu einer gut gerührten Lösung von 2,8 g (31,5 mmol) CuCN und 6,3 g (96 mmol) KCN in 100

ml Wasser gegeben. Man läßt auf RT erwärmen und saugt nach 12 h vom ausgefallenen Feststoff ab. Das Filtrat wird mit $CHCl_3/CH_3OH$ (80/20 vv) extrahiert, die organische Phase eingeengt und der Rückstand mit dem Filterkuchen vereinigt. Nach Umkristallisation aus Methanol erhält man 2,6 g (50 % d. Th.) farbloser Kristalle vom Schmp. 208-209,5°C.
$C_{12}H_{10}N_3ClO$ (247,68)
Rf = 0,62 (Dichlormethan/Methanol 95/5)

b) 6-[3-Cyano-4-(4-ethoxycarbonyl-piperazin-1-yl)phenyl]-4,5-dihydro-5-methyl-3(2H)-pyridazinon

1,5 g (6,1 mmol) des Chlorcyanophenylpyridazinons aus Stufe a) werden 5 h mit 5 ml 1-Ethoxycarbonyl-piperazin auf 170°C erhitzt. Nach dem Abkühlen gießt man auf Wasser, extrahiert mit Ethylacetat und engt die vereinigten org. Phasen im Vakuum ein.
Der Rückstand wird an Kieselgel (Laufmittel Dichlormethan/Methanol 96/4) chromatographiert. Die Hauptfraktionen werden vereinigt und der Rückstand aus wenig Ethanol (99,7 %) kristallisiert.
Man erhält 0,8 g (35 % d. Th.) farbloser Kristalle vom Schmp. 146,0-146,6°C.
$C_{19}H_{23}N_5O_3$ (369,43)
Rf = 0,50 (Dichormethan/Methanol 95/5)

Beispiel 29

6-[4-(4-Aminomethyl-piperidin-1-yl)-3-nitro-phenyl]-4,5-dihydro-5-methyl-3(2H)-pyridazinon

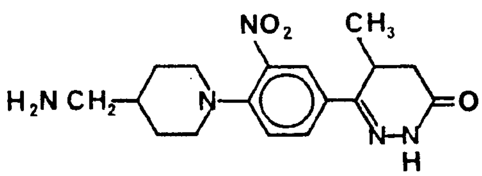

a) 6-[4-(4-Phthalimidomethyl-piperidin-1-yl)-3-nitro-phenyl]-4,5-dihydro-5-methyl-3(2H)-pyridazinon

2,68 g (10 mmol) 6-(4-Chlor-3-nitro-phenyl)-4,5-dihydro-5-methyl-3(2H)-pyridazinon und 2,81 g (10 mmol) 4-Phthalimidomethyl-piperidin-hydrochlorid werden in 30 ml Dioxan suspendiert. Nach Zugabe von 2,8 ml (20 mmol) Triethylamin wird das Gemisch 4 Stunden unter Rückfluß gekocht. Nach Abkühlen auf Raumtemperatur wird die Lösung vom Rückstand abfiltriert und i. Vak. eingedampft. Nach Umkristallisation des verbleibenden Feststoffs aus Acetonitril erhält man 3,76 g (79 %) eines orangegelben Feststoffs vom Schmp. 256-258°C.
$C_{25}H_{25}N_5O_5$ (475,51)
Rf = 0,45 (Dichlormethan/Methanol 95/5)

b) 6-[4-(4-Aminomethyl-piperidin-1-yl)-3-nitrophenyl]-4,5-dihydro-5-methyl-3(2H)-pyridazinon

3,0 g (6,3 mmol) der Phthalimidoverbindung aus Stufe a) und 1,5 ml (33,3 mmol) Hydrazinhydrat (99 %) werden in 50 ml Ethanol 2 Stunden unter Rückfluß gekocht. Nach Abkühlen auf Raumtemperatur wird filtriert und das Filtrat i. Vak. eingedampft. Der erhaltene Rückstand wird im 20 ml Wasser aufgenommen, die Lösung mit 2N Salzsäure auf pH 1 gestellt und abermals filtriert. Das erhaltene Filtrat wird mit konz. Ammoniak auf pH 10 gestellt, wobei ein orangegelber Feststoff ausfällt, der abgesaugt und aus Acetonitril/Ethanol (7:1) umkristallisiert wird. Man erhält 1,16 g (53 %) eines orangegelben Feststoffs vom Schmp. 131-132°C.
$C_{17}H_{23}N_5O_3$ (345,40)
Rf = 0,57 (Ethylacetat/Ethanol/konz. Ammoniak 15/10/2)

Beispiel 30

6-[4-[4-(4-Aminobutyl)-piperazin-1-yl]-3-nitro-phenyl]-5-methyl-4,5-dihydro-3(2H)-pyridazinon

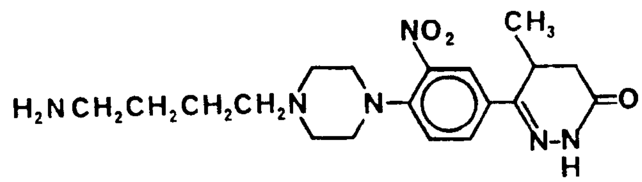

a) 5-Methyl-6-[3-nitro-4-[4-[4-(1-phthalimido)butyl]piperazin-1-yl]phenyl]-4,5-dihydro-3(2H)-pyridazinon

5,6 g (17,7 mmol) 5-Methyl-6-[3-nitro-4-(1-piperazinyl)phenyl]-4,5-dihydro-3(2H)-pyridazinon werden mit 5,0 g N-(4-Brombutyl) phthalimid analog Beispiel 24 a) umgesetzt.
Man erhält 6,94 g (76 % d. Th.) eines orangefarbenen Pulvers vom Schmp. 179 - 180°C.
$C_{27}H_{30}N_6O_5$ (518,58)
Rf = 0,62 (Ethylacetat/Methanol/Ammoniak 80/18/2)

b) 6-[4-[4-(4-Aminobutyl)-piperazin-1-yl]-3-nitro-phenyl]-5-methyl-4,5-dihydro-3(2H)-pyridazinon

6,64 g (12,8 mmol) der wie unter a) erhaltenen Verbindung werden mit 1,92 g (38,6 mmol) Hydrazinhydrat (99 %) analog Beispiel 24 b) umgesetzt.
Die Ausbeute an der Titelverbindung in Form orangener Kristalle vom Schmp. 145 - 146°C beträgt 4,2 g (85 % d. Th.).
$C_{19}H_{28}N_6O_3$ (388,47)
Rf = 0,28 (Dichlormethan/Methanol/Ammoniak 85/13/2)

Beispiel 31

6-[3-Cyano-4-(1-piperazinyl)phenyl]-4,5-dihydro-5-methyl-3(2H)-pyridazinon

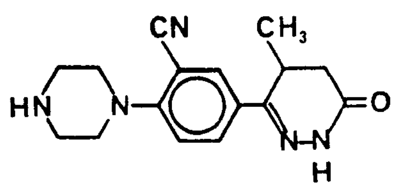

10,0 g (40,4 mmol) 6-(4-Chlor-3-cyano-phenyl)-4,5-dihydro-5-methyl-3(2H)-pyridazinon werden analog Beispiel 1) mit 10 g Piperazin umgesetzt.
Die Umkristallisation aus Ethanol ergibt 9,8 g (82 % d. Th.) eines farblosen Pulvers vom Schmp. 188 - 189°C.
$C_{16}H_{19}N_5O$ (297,36)
Rf = 0,37 (Dichlormethan/Methanol/Ammoniak 85/13/2)

Beispiel 32

6-[4-(4-Aminopiperidin-1-yl)-3-nitro-phenyl]-5-methyl-4,5-dihydro-3(2H)-pyridazinon

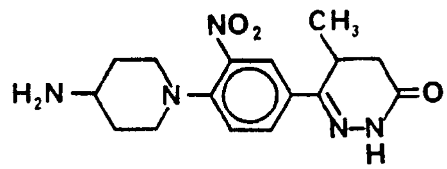

3,5 g (13 mmol) 4-(4-Chlor-3-nitro-phenyl)-5-methyl-4,5-dihydro-3(2H)-pyridazinon werden mit 3,0 g (13 mmol) 4-(1-Phthalimido)-piperidin und 4 ml Pyridin in 100 ml Dioxan 8 h unter Rückfluß gekocht.
Das Dioxan wird im Vakuum abdestilliert, der Rückstand mit 100 ml Ethanol und 3,2 ml Hydrazinhydrat versetzt, und das Gemisch weitere 3 h unter Rückfluß erhitzt.
Anschließend versetzt man mit Wasser und stellt mit NaOH-Lösung stark alkalisch (pH 12).
Nach Extraktion mit Chloroform und Verdampfen des Lösungsmittels i. V. verbleibt ein dunkelbraunes Öl, das an Kieselgel chromatographiert wird (Laufmittel Ethylacetat/Methanol/$NH_3$ 80/18/2).
Die Hauptfraktionen werden vereinigt, eingedampft, und der Rückstand aus Methanol umkristallisiert.
Man erhält 560 mg (13 % d. Th.) der Titelverbindung als orange Kristalle vom Schmp. 151 - 153°C.
$C_{16}H_{21}N_5O_3$ (331,38)
Rf = 0,12 (Ethylacetat/Methanol/Ammoniak 80/18/2)

Beispiel 33

6-[3-Nitro-4-[4-[4-(1-phthalimido)butyryl]piperazin-1-yl] phenyl]-5-methyl-4,5-dihydro-3(2H)-pyridazinon

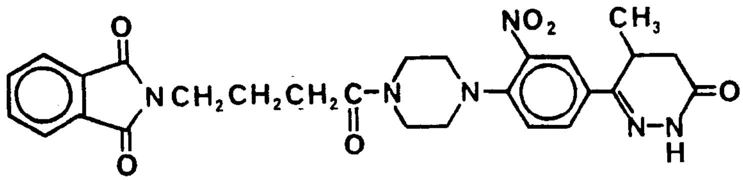

Zu einer Aufschlämmung von 10,9 g (31,5 mmol) 5-Methyl-6-[3-nitro-4-(1-piperazinyl)phenyl]-4,5-dihydro-3(2H)-pyridazinon in 80 ml Pyridin tropft man rasch 8,0 g (31,8 mmol) 4-(1-Phthalimido)-buttersäurechlorid.
Nach 8 h bei RT gießt man das Reaktionsgemisch auf 700 ml Wasser, rührt weitere 2 h bei RT, saugt vom Feststoff ab und wäscht den Filterkuchen mit Wasser.
Die Titelverbindung wird nach der Vakuumtrocknung als gelbes Pulver vom Schmp. 125 - 126°C mit einer Ausbeute von 14,4 g (86 % d. Th.) erhalten.
$C_{27}H_{28}N_7O_6$ (532,56)
Rf = 0.63 (Ethylacetat/Methanol/Ammoniak 80/18/2)

Beispiel 34

6-[3-Amino-4-[4-(2-pyrimidyl)-piperazin-1-yl]phenyl] -4,5-dihydro-5-methyl-3(2H)-pyridazinon

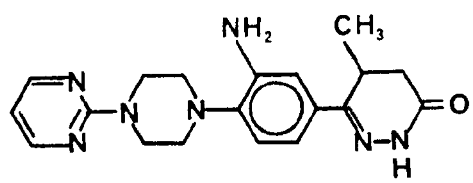

2,5 g (6,3 mmol) 6-[3-Nitro-4-[4-(2-pyrimidyl)-piperazin-1-yl]-phenyl]-4,5-dihydro-5-methyl-3(2H)-pyridazinon in 130 ml Methanol werden in Gegenwart von 0,3 g Palladium auf Aktivkohle (10 % Pd) bei 50 °C und einem Wasserstoffdruck von 5 bar drei Stunden hydriert. Nach Absaugen des Katalysators wird das Filtrat i. Vak. weitgehend eingeengt und der erhaltene Rückstand an Kieselgel mit Dichlormethan/Methanol (95:5) als Laufmittel chromatographiert. Nach Eindampfen der Produktfraktionen wird der erhaltene Rückstand mit Aceton kristallisiert. Man erhält 0,70 g (30 %) hellbeige Kristalle vom Schmp. 220 - 221 °C.
$C_{19}H_{23}N_7O$ (365,44)
Rf = 0,15 (Dichlormethan/Methanol 95/5)

Beispiel 35

6-[3-Fluor-4-(piperazin-1-yl)-phenyl]-4,5-dihydro-5-methyl-3(2H)-pyridazinon

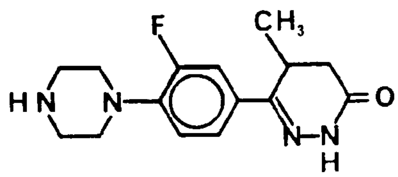

5,0 g (22,3 mmol) 6-(3,4-Difluorphenyl)-4,5-dihydro-5-methyl-3(2H)-pyridazinon und 19,2 g (222 mmol) Piperazin werden 15 Stunden bei 160 °C gerührt. Das abgekühlte Reaktionsgemisch wird in 200 ml Dichlormethan aufgenommen und mit 3 x 30 ml Wasser gewaschen. Nach dem Trocknen wird die organ. Phase eingedampft und der Rückstand mit Methanol kristallisiert. Der erhaltene Feststoff wird abgesaugt und aus Ethylacetat umkristallisiert. Man erhält 3,5 g (54 %) farblose Kristalle vom Schmp. 172 - 174 °C.
$C_{15}H_{19}FN_4O$ (290,34)
Rf = 0,53 (Methanol/konz. Ammoniak 95/5)

Beispiel 36

6-[4-(4-Ethoxycarbonyl-piperazin-1-yl]-3-fluor-phenyl]-4,5-dihydro-5-methyl-3(2H)-pyridazinon

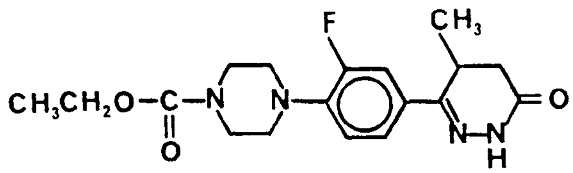

Zu einer Lösung von 1,0 g (3,4 mmol) 6-[3-Fluor-4-(piperazin-1-yl)-phenyl]-4,5-dihydro-5-methyl-3(2H)-pyridazinon in 10 ml Aceton und 10 ml Wasser werden 1,2 g (8,6 mmol)Kaliumcarbonat gegeben. Die auf 0°C abgekühlte Suspension wird dann mit 0,42 ml (4,4 mmol)Chlorameisensäureethylester versetzt und 30 Minuten weitergerührt. Der ausgefallene Feststoff wird abgesaugt, mit 10 ml Aceton-Wasser-Gemisch gewaschen und aus 40 ml Acetonitril umkristallisiert. Es werden 0,73 g (60 %) eines farblosen Feststoffs vom Schmp. 193 - 194 °C erhalten.
$C_{18}H_{23}FN_4O_3$ (362,41)
Rf = 0,40 (Dichlormethan/Methanol 95/5)

Beispiel 37

6-[4-[(3-Aminopropyl)amino]-3-nitro-phenyl]-4,5-dihydro-5-hydroxymethyl-3(2H)-pyridazinon

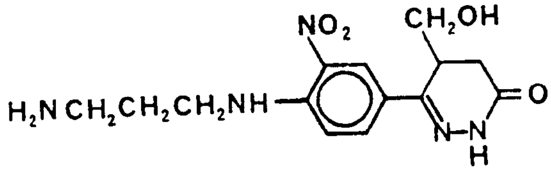

14,0 g (50 mmol) 6-(4-Chlor-3-nitro-phenyl)-4,5-dihydro-5-hydroxymethyl-3(2H)-pyridazinon werden mit 50 ml 1,3-Diaminopropan in 40 ml Dioxan 8 h auf 60 °C erhitzt.
Anschließend gießt man das Reaktionsgemisch auf 200 ml Eiswasser und läßt unter Kühlung kristallisieren. Nach dem Absaugen und Waschen des Filterkuchens mit Wasser und Ethanol erhält man einen orangefarbenen Feststoff vom Schmp. 180-181 °C. Die Ausbeute beträgt 14,05 g (87 % d. Th.).
$C_{14}H_{19}N_5O_4$ (321,34)
Rf = 0,17 (Dichlormethan/Methanol/$NH_3$ 85/13/2)

Beispiel 38

6-[3-Cyano-4-(4-ethoxycarbonyl-piperazin-1-yl)-phenyl]-4,5-dihydro-3(2H)-pyridazinon

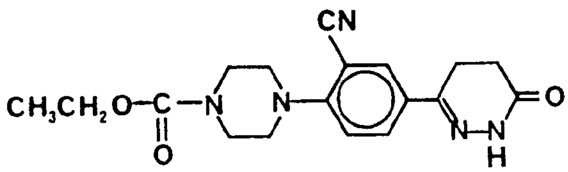

Erhalten analog Beispiel 36) aus 1,0 g (3,5 mmol) 6-[3-Cyano-4-(piperazin-1-yl)-phenyl]-4,5-dihydro-3(2H)-pyridazinon und 0,43 ml (4,5 mmol) Chlorameisensäureethylester.
Nach Umkristallisieren des Rohprodukts aus Ethylacetat erhält man 0,66 g (53 %) gelbliche Kristalle vom Schmp. 187,3 - 188,9 °C.
$C_{18} H_{21} N_5 O_3$ (355,40)
Rf = 0,40 (Dichlormethan/Methanol 95/5)

Beispiel 39

6-[4-(3-Aminopropyl)amino-3-cyano-phenyl]-4,5-dihydro-5-methyl-3(2H)-pyridazinon

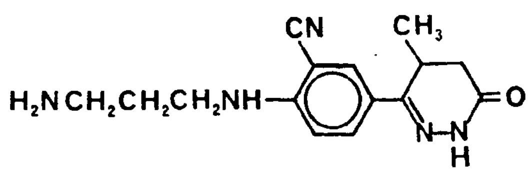

8,7 g(35 mmol) 6-(4-Chlor-3-cyano-phenyl)-4,5-dihydro-5-methyl-3(2H)-pyridazinon und 30 ml 1,3-Diaminopropan werden 6 Stunden unter Rückfluß gekocht.
Das erkaltete Reaktionsgemisch wird in 200 ml Eiswasser gegossen und die erhaltene Lösung mit 3 x 100 ml Dichlormethan extrahiert. Das aus der organischen Phase nach Trocknen und Eindampfen erhaltene Rohprodukt wird an 200 g Kieselgel mit Dichlormethan/Methanol/konz. Ammoniak 85/13/2 chromatographiert.
Aus der Hauptfraktion werden nach Eindampfen 2,2 g (22 %) eines blaßgelben Feststoffes vom Schmp. 174,5 - 175,5 °C erhalten.
$C_{15} H_{19} N_5 O$ (285,35)

Rf = 0,33 (Dichlormethan/Methanol/konz. Ammoniak 85/13/2)

Beispiel 40

6-[4-[4-[4-(Aminomethyl)-thiazol-2-yl]-piperazin-1-yl]-3-nitro-phenyl]-4,5-dihydro-5-methyl-3(2H)-pyridazinon

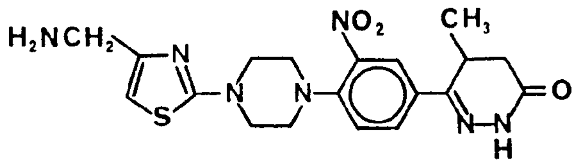

8,1 g (14,4 mmol) 6-[4-[4-[4-(Phtalimidomethyl)-thiazol-2-yl]-piperazin-1-yl]-3-nitro-phenyl]-4,5-dihydro-5-methyl-3(2H)-pyridazinon werden in 150 ml Ethanol mit 2 ml (40 mmol) Hydrazinhydrat 3 Stunden unter Rückfluß gekocht.
Der nach Eindampfen des Reaktionsgemisches im Vakuum erhaltene Rückstand wird in 50 ml Wasser aufgenommen und mit konz. Salzsäure auf pH1 gestellt.
Nach Absaugen des Niederschlages wird das Filtrat mit Natronlauge auf pH12 gestellt und 15 min gerührt. Man saugt den ausgefallenen Feststoff ab und kristallisiert aus Ethanol um.
Es werden 5,56 g (90 %) eines orangefarbenen Pulvers vom Schmp. 187,8 - 188,0 °C erhalten.
$C_{19}H_{23}N_7O_3S$ (429,50)
Rf = 0,62 (Dichlormethan/Methanol/konz. Ammoniak 85:13:2)

## Patentansprüche

### Patentansprüche für folgende Vertragsstaaten : AT, BE, CH, DE, FR, GB, IT, LI, LU, NL, SE

1. 6-Phenyldihydro-3(2H)-pyridazinone der allgemeinen Formel I

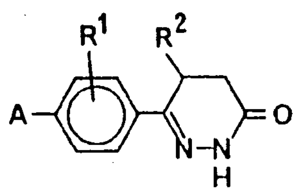

(I)

in der $R^1$ eine Nitrogruppe, eine Aminogruppe, eine Hydroxygruppe, eine Cyanogruppe oder ein Halogenatom bedeutet,
$R^2$ für ein Wasserstoffatom, eine Methylgruppe oder eine Hydroxymethylgruppe steht und
A für die Gruppe

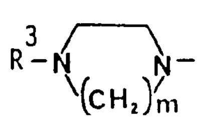 , $R^3-NH-(CH_2)_n-NH-$, 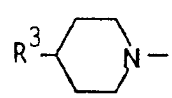

oder 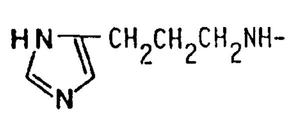

steht, worin $R^3$ ein Wasserstoffatom, eine gegebenenfalls mit einer Amino- oder Hydroxygruppe substituierte $C_1$-$C_6$-Alkylgruppe, eine gegebenenfalls substituierte Aryl- oder Heteroarylgruppe, eine Cyanogruppe, eine Acylgruppe, eine gegebenenfalls substituierte Aroylgruppe, eine Carboxylgruppe oder eine $C_1$-$C_6$-Alkoxycar-

bonylgruppe bedeutet, m eine ganze Zahl von 1 bis 3 und n eine ganze Zahl von 2 bis 4 ist, sowie die physiologisch annehmbaren Salze davon.

2. 6-Phenyldihydro-3(2H)-pyridazinone nach Anspruch 1, dadurch **gekennzeichnet,** daß $R^1$ für eine Nitrogruppe, eine Aminogruppe, eine Hydroxygruppe, eine Cyanogruppe oder ein Halogenatom steht, $R^2$ für ein Wasserstoffatom, eine Methylgruppe oder ein Hydroxymethylgruppe steht und
A die Gruppe

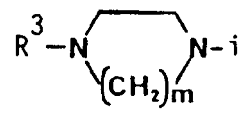

ist, worin $R^3$ ein Wasserstoffatom, eine gegebenenfalls mit einer Amino- oder Hydroxygruppe substituierte $C_1$-$C_6$-Alkylgruppe, eine gegebenenfalls substituierte Aryl- oder Heteroarylgruppe, eine Cyanogruppe, eine Acylgruppe, eine gegebenenfalls substituierte Aroylgruppe oder eine $C_1$-$C_6$-Alkoxycarbonylgruppe ist und m eine ganze Zahl von 1 bis 3 ist.

3. 6-Phenyldihydro-3(2H)-pyridazinone nach Anspruch 1, dadurch **gekennzeichnet,** daß $R^1$ für eine Nitrogruppe, eine Aminogruppe, eine Hydroxygruppe, eine Cyanogruppe oder ein Halogenatom steht, $R^2$ ein Wasserstoffatom oder eine Methylgruppe bedeutet und A für die Gruppe $R^3$-NH-$(CH_2)_n$-NH- steht, wobei $R^3$ ein Wasserstoffatom, eine gegebenenfalls substituierte Aryl- oder Heteroarylgruppe, eine Acylgruppe, eine gegebenenfalls substituierte Aroylgruppe oder eine $C_1$-$C_6$-Alkoxycarbonylgruppe bedeutet und n eine ganze Zahl von 2 bis 4 ist.

4. 6-Phenyldihydro-3(2H)-pyridazinone nach Anspruch 1, dadurch **gekennzeichnet,** daß $R^1$ für eine Nitrogruppe, eine Aminogruppe, eine Hydroxygruppe, eine Cyanogruppe oder ein Halogenatom steht, $R^2$ ein Wasserstoffatom oder eine Methylgruppe ist und
A die Gruppe

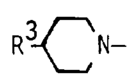

bedeutet, worin $R^3$ für eine gegebenenfalls mit einer Amino- oder Hydroxygruppe substituierte $C_1$-$C_6$-Alkylgruppe, eine gegebenenfalls substituierte Aryl- oder Heteroarylgruppe, eine Acylgruppe, eine gegebenenfalls substituierte Aroylgruppe, eine Carboxylgruppe oder eine $C_1$-$C_6$-Alkoxycarbonylgruppe steht.

5. 6-Phenyldihydro-3(2H)-pyridazinone nach Anspruch 1, dadurch **gekennzeichnet,** daß $R^1$ für eine Nitrogruppe, eine Aminogruppe, eine Hydroxygruppe, eine Cyanogruppe oder ein Halogenatom steht, $R^2$ ein Wasserstoffatom, eine Methylgruppe oder eine Hydroxymethylgruppe bedeutet und A für die Gruppe

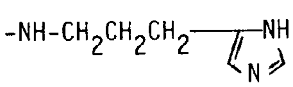

steht.

6. 6-[3-Nitro-4-(1-piperazinyl)-phenyl]-4,5-dihydro-5-methyl-3(2H)-pyridazinon und die physiologisch annehmbaren Salze.

7. 6-[4-(4-Ethoxycarbonylpiperazin-1-yl)-3-nitrophenyl]-4,5-dihydro-5-methyl-3(2H)-pyridazinon und die physiologisch annehmbaren Salze.

8. 6-[3-Amino-4-(4-ethoxycarbonylpiperazin-1-yl)-phenyl]-4,5-dihydro-5-methyl-3(2H)-pyridazinon und die physiologisch annehmbaren Salze.

9. 6-[4-(4-Acetylpiperazin-1-yl)-3-nitrophenyl]-4,5-dihydro-5-methyl-3(2H)-pyridazinon und die physiologisch annehmbaren Salze.

10. 6-[4-(4-Ethoxycarbonylpiperazin-1-yl)-3-nitrophenyl]-4,5-dihydro-3(2H)-pyridazinon und die physiologisch annehmbaren Salze.

11. Verfahren zur Herstellung von 6-Phenyldihydro-3(2H)-pyridazinonen nach den Ansprüchen 1 bis 10, dadurch **gekennzeichnet,** daß man eine Verbindung der allgemeinen Formel II

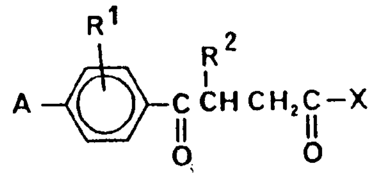

(II)

in der A, $R^1$ und $R^2$ wie in Anspruch 1 definiert sind und X für die Gruppe -OH oder die Gruppe $-OR^4$ steht, in der $R^4$ eine gegebenenfalls substituierte $C_1$-$C_6$-Alkylgruppe oder eine gegebenenfalls substituierte Arylgruppe ist, mit Hydrazin oder einem chemischen Äquivalent davon zu einer Verbindung der allgemeinen Formel I umsetzt und die erhaltene Verbindung gegebenenfalls in an sich bekannter Weise in ihr physiologisch annehmbares Salz umwandelt.

12. Verfahren zur Herstellung von 6-Phenyldihydro-3(2H)-pyridazinonen nach den Ansprüchen 1 bis 10,dadurch **gekennzeichnet,** daß man

a) zur Herstellung von Verbindungen der allgemeinen Formel I, in der A und $R^2$ wie in Anspruch 1 definiert sind und $R^1$ für eine Nitrogruppe steht, eine Verbindung der allgemeinen Formel III

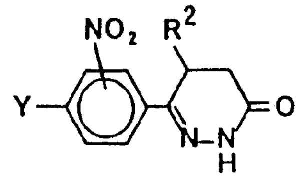

(III)

in der $R^2$ wie in Anspruch 1 definiert ist und Y für ein Halogenatom steht, mit einer Verbindung der allgemeinen Formel IV

H-A (IV)

in der A wie in Anspruch 1 definiert ist, umsetzt oder

b) zur Herstellung von Verbindungen der allgemeinen Formel I, in der A und $R^2$ wie in Anspruch 1 definiert sind und $R^1$ für eine Aminogruppe steht, eine Verbindung der allgemeinen Formel Ia

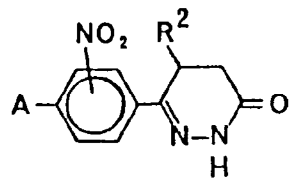

(Ia)

in der $R^2$ und A wie in Anspruch 1 definiert sind und $R^1$ eine Nitrogruppe ist, mit einem Reduktionsmittel reduziert oder

c) zur Herstellung von Verbindungen der allgemeinen Formel I, in der A und $R^2$ die in Anspruch 1 angegebenen Bedeutungen haben und $R^1$ für eine Hydroxygruppe, eine Cyanogruppe oder ein Halogenatom steht, eine Verbindung der allgemeinen Formel Ib

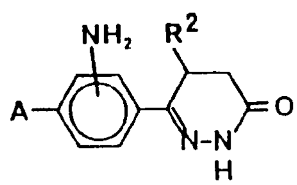

(Ib)

in der A und $R^2$ wie in Anspruch 1 definiert sind, zu einer Verbindung der allgemeinen Formel V

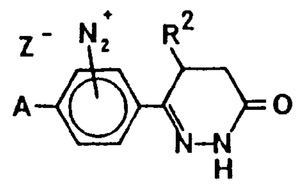

(V)

in der A und $R^2$ wie in Anspruch 1 definiert sind und Z für ein Halogenatom, eine Hydroxygruppe, eine Cyanogruppe oder die Tetrafluoroboratgruppe steht, diazotiert und das Diazoniumsalz der allgemeinen Formel V, gegebenenfalls in Gegenwart von Kupferpulver oder eines Kupfer(I)halogenids oder von Kupfer(I)cyanid, zu einer Verbindung der allgemeinen Formel I, in der $R^2$ und A wie in Anspruch 1 definiert sind und $R^1$ eine Hydroxygruppe, eine Cyanogruppe oder ein Halogenatom ist, umsetzt oder

d) zur Herstellung von Verbindungen der allgemeinen Formel I, in der $R^1$ und $R^2$ wie in Anspruch 1 definiert sind und A für die Gruppe

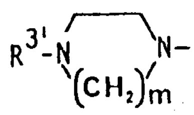

oder die Gruppe $R^{3'}$-NH-$(CH_2)_n$NH- steht, wobei m und n wie in Anspruch 1 definiert sind und $R^{3'}$ für eine Cyanogruppe, eine Acylgruppe, eine gegebenenfalls substituierte Aroylgruppe oder eine $C_1$-$C_6$-Alkoxycarbonylgruppe steht, eine Verbindung der allgemeinen Formeln Ic oder Id

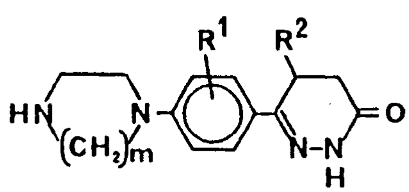

(Ic)

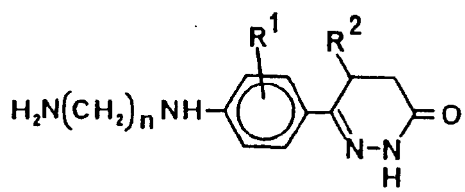

(Id)

in denen $R^1$, $R^2$, m und n die in Anspruch 1 angegebenen Bedeutungen besitzen, mit einem Acylierungsmittel der allgemeinen Formel VI

$R^{3'}$-L (VI)

in der $R^{3'}$ die oben angegebene Bedeutung hat und L für ein Halogenatom, die Gruppe -$OR^{3'}$, die Hydroxygruppe oder den über ein Stickstoffatom gebundenen Rest eines Azols oder Benzazols mit mindestens zwei Stickstoffatomen im Fünfring steht, zur Reaktion bringt oder

e) zur Herstellung von Verbindungen der allgemeinen Formel I, in der $R^1$ und $R^2$ wie in Anspruch 1 definiert sind und A für die Gruppe

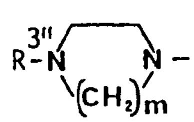

oder die Gruppe $R^{3''}$-NH-$(CH_2)_n$-NH- steht, worin m und n wie in Anspruch 1 definiert sind und $R^{3''}$ für ein Wasserstoffatom steht, eine Verbindung der allgemeinen Formel I, in der $R^1$ und $R^2$ wie in Anspruch 1 definiert sind und A für die Gruppen

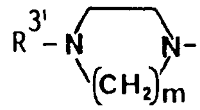

oder $R^{3'}$-NH-$(CH_2)_n$-NH- steht, worin $R^{3'}$ eine Cyanogruppe, eine Acylgruppe, eine gegebenenfalls substituierte Aroylgruppe oder eine $C_1$-$C_6$-Alkoxycarbonylgruppe ist, sauer oder basisch hydrolysiert und gegebenenfalls decarboxyliert

und daß man die nach den Verfahrensvarianten a) bis e) erhaltene Verbindung der allgemeinen Formel I gegebenenfalls in an sich bekannter Weise in ein physiologisch annehmbares Salz umwandelt.

13. Arzneimittel, dadurch **gekennzeichnet,** daß es eine Verbindung nach einem der Ansprüche 1 bis 10 zusammen mit mindestens einem inerten, pharmazeutisch annehmbaren Träger oder Verdünnungsmittel enthält.

**Patentansprüche für folgenden Vertragsstaat: ES**

1. Verfahren zur Herstellung von 6-Phenyldihydro-3(2H)-pyridazinonen der allgemeinen Formel I

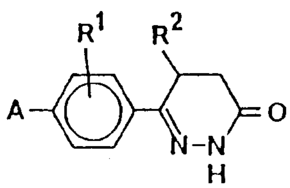 (I)

in der $R^1$ eine Nitrogruppe, eine Aminogruppe, eine Hydroxygruppe, eine Cyanogruppe oder ein Halogenatom bedeutet,
$R^2$ für ein Wasserstoffatom, eine Methylgruppe oder eine Hydroxymethylgruppe steht und
A für die Gruppe

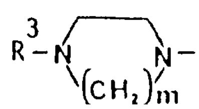 , $R^3$-NH-$(CH_2)_n$-NH-, 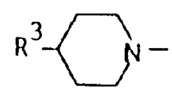

oder 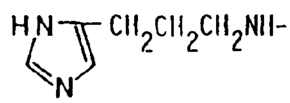

steht, worin $R^3$ ein Wasserstoffatom, eine gegebenenfalls mit einer Amino- oder Hydroxygruppe substituierte $C_1$-$C_6$-Alkylgruppe, eine gegebenenfalls substituierte Aryl- oder Heteroarylgruppe, eine Cyanogruppe, eine Acylgruppe, eine gegebenenfalls substituierte Aroylgruppe, eine Carboxylgruppe oder eine $C_1$-$C_6$-Alkoxycarbonylgruppe bedeutet, m eine ganze Zahl von 1 bis 3 und n eine ganze Zahl von 2 bis 4 ist, sowie die physiologisch annehmbaren Salze davon, dadurch **gekennzeichnet,** daß man eine Verbindung der allgemeinen Formel II

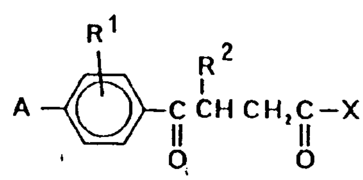 (II)

in der A, $R^1$ und $R^2$ wie oben definiert sind und X für die Gruppe -OH oder die Gruppe -$OR^4$ steht, in der $R^4$ eine gegebenenfalls substituierte $C_1$-$C_6$-Alkylgruppe oder eine gegebenenfalls substituierte Arylgruppe ist, mit

Hydrazin oder einem chemischen Äquivalent davon zu einer Verbindung der allgemeinen Formel I umsetzt und die erhaltene Verbindung gegebenenfalls in an sich bekannter Weise in ihr physiologisch annehmbares Salz umwandelt.

2. Verfahren zur Herstellung von 6-Phenyldihydro-3(2H)-pyridazinonen der allgemeinen Formel I

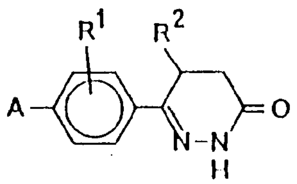

(I)

in der $R^1$ eine Nitrogruppe, eine Aminogruppe, eine Hydroxygruppe, eine Cyanogruppe oder ein Halogenatom bedeutet,
$R^2$ für ein Wasserstoffatom, eine Methylgruppe oder eine Hydroxymethylgruppe steht und
A für die Gruppe

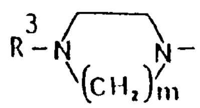 , $R^3-NH-(CH_2)_n-NH-$, 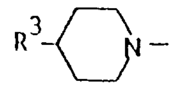

oder 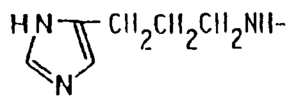

steht, worin $R^3$ ein Wasserstoffatom, eine gegebenenfalls mit einer Amino- oder Hydroxygruppe substituierte $C_1$-$C_6$-Alkylgruppe, eine gegebenenfalls substituierte Aryl- oder Heteroarylgruppe, eine Cyanogruppe, eine Acylgruppe, eine gegebenenfalls substituierte Aroylgruppe, eine Carboxylgruppe oder eine $C_1$-$C_6$-Alkoxycarbonylgruppe bedeutet, m eine ganze Zahl von 1 bis 3 und n eine ganze Zahl von 2 bis 4 ist, sowie der physiologisch annehmbaren Salze davon, dadurch **gekennzeichnet**, daß man

a) zur Herstellung von Verbindungen der allgemeinen Formel I, in der A und $R^2$ wie oben definiert sind und $R^1$ für eine Nitrogruppe steht, eine Verbindung der allgemeinen Formel III

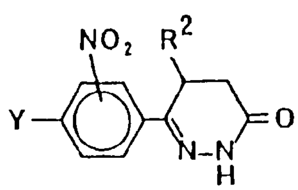

(III)

in der $R^2$ wie oben definiert ist und Y für ein Halogenatom steht, mit einer Verbindung der allgemeinen Formel IV

H-A (IV)

in der A wie oben definiert ist, umsetzt oder

b) zur Herstellung von Verbindungen der allgemeinen Formel I, in der A und $R^2$ wie oben definiert sind und $R^1$ für eine Aminogruppe steht, eine Verbindung der allgemeinen Formel Ia

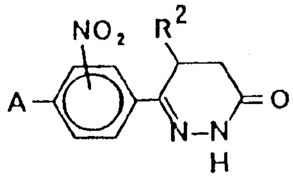

(Ia)

in der $R^2$ und A wie oben definiert sind und $R^1$ eine Nitrogruppe ist, mit einem Reduktionsmittel reduziert oder

c) zur Herstellung von Verbindungen der allgemeinen Formel I, in der A und $R^2$ die oben angegebenen Bedeutungen haben und $R^1$ für eine Hydroxygruppe, eine Cyanogruppe oder ein Halogenatom steht, eine Verbindung der allgemeinen Formel Ib

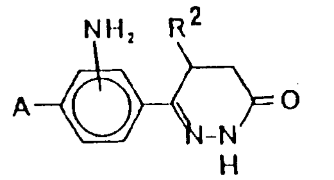

(Ib)

in der A und $R^2$ wie in Anspruch 1 definiert sind, zu einer Verbindung der allgemeinen Formel V

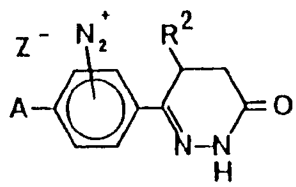

(V)

in der A und $R^2$ wie oben definiert sind und Z für ein Halogenatom, eine Hydroxygruppe, eine Cyanogruppe oder die Tetrafluoroboratgruppe steht, diazotiert und das Diazoniumsalz der allgemeinen Formel V, gegebenenfalls in Gegenwart von Kupferpulver oder eines Kupfer(I)halogenids oder von Kupfer(I)cyanid, zu einer Verbindung der allgemeinen Formel I, in der $R^2$ und A wie oben definiert sind und $R^1$ eine Hydroxygruppe, eine Cyanogruppe oder ein Halogenatom ist, umsetzt oder

d) zur Herstellung von Verbindungen der allgemeinen Formel I, in der $R^1$ und $R^2$ wie oben definiert sind und A für die Gruppe

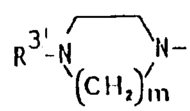

oder die Gruppe $R^{3'}$-NH-$(CH_2)_n$NH- steht, wobei m und n wie oben definiert sind und $R^{3'}$ für eine Cyanogruppe, eine Acylgruppe, eine gegebenenfalls substituierte Aroylgruppe oder eine $C_1$-$C_6$-Alkoxycarbonylgruppe steht, eine Verbindung der allgemeinen Formeln Ic oder Id

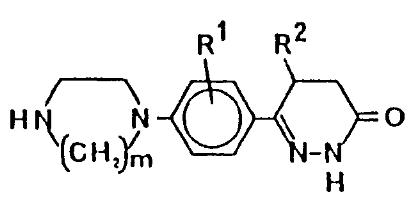

(Ic)

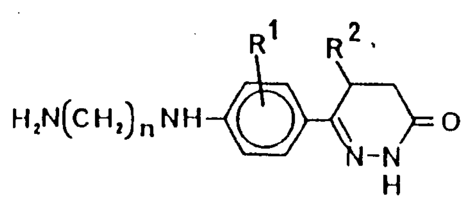

(Id)

in denen $R^1$, $R^2$, m und n die oben angegebenen Bedeutungen besitzen, mit einem Acylierungsmittel der allgemeinen Formel VI

$$R^{3'}\text{-}L \quad (VI)$$

in der $R^{3'}$ die oben angegebene Bedeutung hat und L für ein Halogenatom, die Gruppe -$OR^{3'}$ die Hydroxygruppe oder den über ein Stickstoffatom gebundenen Rest eines Azols oder Benzazols mit mindestens zwei Stickstoffatomen im Fünfring steht, zur Reaktion bringt oder

e) zur Herstellung von Verbindungen der allgemeinen Formel I, in der $R^1$ und $R^2$ wie oben definiert sind und A für die Gruppe

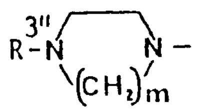

oder die Gruppe $R^{3''}$-NH-$(CH_2)_n$-NH- steht, worin m und n wie oben definiert sind und $R^{3''}$ für ein Wasserstoffatom steht, eine Verbindung der allgemeinen Formel I, in der $R^1$ und $R^2$ wie oben definiert sind und A für die Gruppen

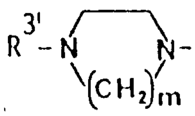

oder $R^{3'}$-NH-$(CH_2)_n$-NH- steht, worin $R^{3'}$ eine Cyanogruppe, eine Acylgruppe, eine gegebenenfalls substituierte Aroylgruppe oder eine $C_1$-$C_6$-Alkoxycarbonylgruppe ist, sauer oder basisch hydrolysiert und gegebenenfalls decarboxyliert

und daß man die nach den Verfahrensvarianten a) bis e) erhaltene Verbindung der allgemeinen Formel I gegebenenfalls in an sich bekannter Weise in ein physiologisch annehmbares Salz umwandelt.

3. Verfahren nach Anspruch 1 oder 2 zur Herstellung von Verbindungen, bei denen $R^1$ für eine Nitrogruppe eine Aminogruppe, eine Hydroxygruppe, eine Cyanogruppe oder ein Halogenatom steht, $R^2$ für ein Wasserstoffatom, eine Methylgruppe oder eine Hydroxymethylgruppe steht und
A die Gruppe

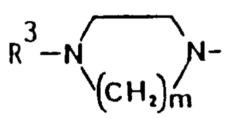

ist, worin $R^3$ ein Wasserstoffatom, eine gegebenenfalls mit einer Amino- oder Hydroxygruppe substituierte $C_1$-$C_6$-Alkylgruppe, eine gegebenenfalls substituierte Aryl- oder Heteroarylgruppe, eine Cyanogruppe, eine Acylgruppe, eine gegebenenfalls substituierte Aroylgruppe oder eine $C_1$-$C_6$-Alkoxycarbonylgruppe ist und m eine ganze Zahl von 1 bis 3 ist.

4. Verfahren nach Anspruch 1 oder 2 zur Herstellung von Verbindungen, bei denen $R^1$ für eine Nitrogruppe eine Aminogruppe, eine Hydroxygruppe, eine Cyanogruppe oder ein Halogenatom steht, $R^2$ ein Wasserstoffatom oder eine Methylgruppe bedeutet und A für die Gruppe $R^3$-NH-$(CH_2)_n$-NH- steht, wobei $R^3$ ein Wasserstoffatom, eine gegebenenfalls substituierte Aryl- oder Heteroarylgruppe, eine Acylgruppe, eine gegebenenfalls substituierte Aroylgruppe oder eine $C_1$-$C_6$-Alkoxycarbonylgruppe bedeutet und n eine ganze Zahl von 2 bis 4 ist.

5. Verfahren nach Anspruch 1 oder 2 zur Herstellung von Verbindungen, bei denen $R^1$ für eine Nitrogruppe eine Aminogruppe, eine Hydroxygruppe, eine Cyanogruppe oder ein Halogenatom steht, $R^2$ ein Wasserstoffatom oder eine Methylgruppe ist und
A die Gruppe

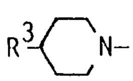

bedeutet, worin $R^3$ für eine gegebenenfalls mit einer Amino- oder Hydroxygruppe substituierte $C_1$-$C_6$-Alkylgruppe, eine gegebenenfalls substituierte Aryl- oder Heteroarylgruppe, eine Acylgruppe, eine gegebenenfalls substituierte Aroylgruppe, eine Carboxylgruppe oder eine $C_1$-$C_6$-Alkoxycarbonylgruppe steht.

6. Verfahren nach Anspruch 1 oder 2 zur Herstellung von Verbindungen, bei denen $R^1$ für eine Nitrogruppe eine Aminogruppe, eine Hydroxygruppe, eine Cyanogruppe oder eine Halogenatom steht, $R^2$ ein Wasserstoffatom, eine Methylgruppe oder eine Hydroxymethylgruppe bedeutet und A für die Gruppe

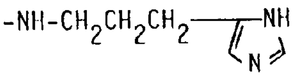

steht.

7. Verfahren nach Anspruch 1 oder 2 zur Herstellung von 6-[3-Nitro-4-(1-piperazinyl)-phenyl]-4,5-dihydro-5-methyl-3(2H)-pyridazinon und der physiologisch annehmbaren Salze.

8. Verfahren nach Anspruch 1 oder 2 zur Herstellung von 6-[4-(4-Ethoxycarbonylpiperazin-1-yl)-3-nitrophenyl]-4,5-dihydro-5-methyl-3(2H)-pyridazinon und der physiologisch annehmbaren Salze.

9. Verfahren nach Anspruch 1 oder 2 zur Herstellung von 6-[3-Amino-4-(4-ethoxycarbonylpiperazin-1-yl)-phenyl]-4,5-dihydro-5-methyl-3(2H)-pyridazinon und der physiologisch annehmbaren Salze.

10. Verfahren nach Anspruch 1 oder 2 zur Herstellung von 6-[4-(4-Acetylpiperazin-1-yl)-3-nitrophenyl]-4,5-dihydro-5-methyl-3(2H)-pyridazinon und der physiologisch annehmbaren Salze.

11. Verfahren nach Anspruch 1 oder 2 zur Herstellung von 6-[4-(4-Ethoxycarbonylpiperazin-1-yl)-3-nitrophenyl]-4,5-dihydro-3(2H)-pyridazinon und der physiologisch annehmbaren Salze.

**Patentansprüche für folgenden Vertragsstaat: GR**

1. 6-Phenyldihydro-3(2H)-pyridazinone der allgemeinen Formel I

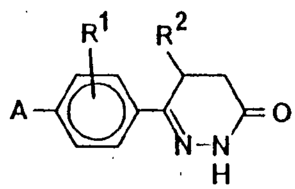

(I)

in der $R^1$ eine Nitrogruppe, eine Aminogruppe, eine Hydroxygruppe, eine Cyanogruppe oder ein Halogenatom bedeutet,
$R^2$ für ein Wasserstoffatom, eine Methylgruppe oder eine Hydroxymethylgruppe steht und
A für die Gruppe

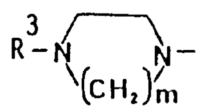 , $R^3-NH-(CH_2)_n-NH-$, 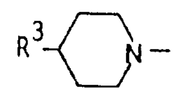

oder 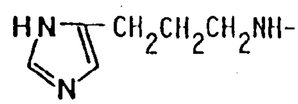

steht, worin $R^3$ ein Wasserstoffatom, eine gegebenenfalls mit einer Amino- oder Hydroxygruppe substituierte $C_1$-$C_6$-Alkylgruppe, eine gegebenenfalls substituierte Aryl- oder Heteroarylgruppe, eine Cyanogruppe, eine Acylgruppe, eine gegebenenfalls substituierte Aroylgruppe, eine Carboxylgruppe oder eine $C_1$-$C_6$-Alkoxycarbonylgruppe bedeutet, m eine ganze Zahl von 1 bis 3 und n eine ganze Zahl von 2 bis 4 ist, sowie die Salze davon.

2. 6-Phenyldihydro-3(2H)-pyridazinone nach Anspruch 1, dadurch **gekennzeichnet,** daß $R^1$ für eine Nitrogruppe, eine Aminogruppe, eine Hydroxygruppe, eine Cyanogruppe oder ein Halogenatom steht, $R^2$ für ein Wasserstoffatom, eine Methylgruppe, oder eine Hydroxymethylgruppe steht und
A die Gruppe

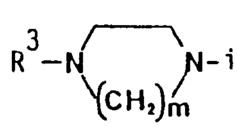

ist, worin $R^3$ ein Wasserstoffatom, eine gegebenenfalls mit einer Amino- oder Hydroxygruppe substituierte $C_1$-$C_6$-Alkylgruppe, eine gegebenenfalls substituierte Aryl- oder Heteroarylgruppe, eine Cyanogruppe, eine Acylgruppe, eine gegebenenfalls substituierte Aroylgruppe oder eine $C_1$-$C_6$-Alkoxycarbonylgruppe ist und m eine ganze Zahl von 1 bis 3 ist.

3. 6-Phenyldihydro-3(2H)-pyridazinone nach Anspruch 1, dadurch **gekennzeichnet,** daß $R^1$ für eine Nitrogruppe, eine Aminogruppe, eine Hydroxygruppe, eine Cyanogruppe oder ein Halogenatom steht, $R^2$ ein Wasserstoffatom oder eine Methylgruppe bedeutet und A für die Gruppe $R^3$-NH-$(CH_2)_n$-NH- steht, wobei $R^3$ ein Wasserstoffatom, eine gegebenenfalls substituierte Aryl- oder Heteroarylgruppe, eine Acylgruppe, eine gegebenenfalls substituierte Aroylgruppe oder eine $C_1$-$C_6$-Alkoxycarbonylgruppe bedeutet und n eine ganze Zahl von 2 bis 4 ist.

4. 6-Phenyldihydro-3(2H)-pyridazinone nach Anspruch 1, dadurch **gekennzeichnet,** daß $R^1$ für eine Nitrogruppe, eine Aminogruppe, eine Hydroxygruppe, eine Cyanogruppe oder ein Halogenatom steht, $R^2$ ein Wasserstoffatom oder eine Methylgruppe ist und
A die Gruppe

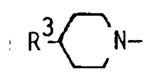

bedeutet, worin $R^3$ für eine gegebenenfalls mit einer Amino- oder Hydroxygruppe substituierte $C_1$-$C_6$-Alkylgruppe, eine gegebenenfalls substituierte Aryl- oder Heteroarylgruppe, eine Acylgruppe, eine gegebenenfalls substituierte Aroylgruppe, eine Carboxylgruppe oder eine $C_1$-$C_6$-Alkoxycarbonylgruppe steht.

5. 6-Phenyldihydro-3(2H)-pyridazinone nach Anspruch 1, dadurch **gekennzeichnet,** daß $R^1$ für eine Nitrogruppe, eine Aminogruppe, eine Hydroxygruppe, eine Cyanogruppe oder ein Halogenatom steht, $R^2$ ein Wasserstoffatom, eine Methylgruppe oder eine Hydroxymethylgruppe bedeutet und A für die Gruppe

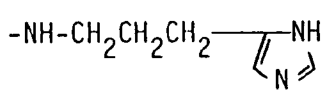

steht.

6. 6-[3-Nitro-4-(1-piperazinyl)-phenyl]-4,5-dihydro-5-methyl-3(2H)-pyridazinon und die Salze.

7. 6-[4-(4-Ethoxycarbonylpiperazin-1-yl)-3-nitrophenyl]-4,5-dihydro-5-methyl-3(2H)-pyridazinon und die Salze.

8. 6-[3-Amino-4-(4-ethoxycarbonylpiperazin-1-yl)-phenyl]-4,5-dihydro-5-methyl-3(2H)-pyridazinon und die Salze.

9. 6-[4-(4-Acetylpiperazin-1-yl)-3-nitrophenyl]-4,5-dihydro-5-methyl-3(2H)-pyridazinon und die Salze.

10. 6-[4-(4-Ethoxycarbonylpiperazin-1-yl)-3-nitrophenyl]-4,5-dihydro-3(2H)-pyridazinon und die Salze.

11. Verfahren zur Herstellung von 6-Phenyldihydro-3(2H)-pyridazinonen nach den Ansprüchen 1 bis 10, dadurch **gekennzeichnet,** daß man eine Verbindung der allgemeinen Formel II

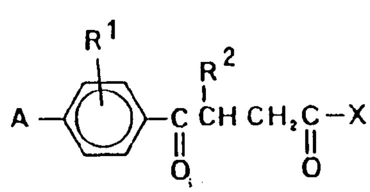

(II)

in der A, $R^1$ und $R^2$ wie in Anspruch 1 definiert sind und X für die Gruppe -OH oder die Gruppe -$OR^4$ steht, in der $R^4$ eine gegebenenfalls substituierte $C_1$-$C_6$-Alkylgruppe oder eine gegebenenfalls substituierte Arylgruppe ist, mit Hydrazin oder einem chemischen Äquivalent davon zu einer Verbindung der allgemeinen Formel I umsetzt und die erhaltene Verbindung gegebenenfalls in an sich bekannter Weise in ihr physiologisch annehmbares Salz umwandelt.

12. Verfahren zur Herstellung von 6-Phenyldihydro-3(2H)-pyridazinonen nach den Ansprüchen 1 bis 10, dadurch **gekennzeichnet,** daß man

a) zur Herstellung von Verbindungen der allgemeinen Formel I, in der A und $R^2$ wie in Anspruch 1 definiert sind und $R^1$ für eine Nitrogruppe steht, eine Verbindung der allgemeinen Formel III

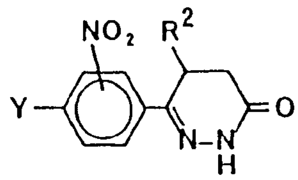

(III)

in der $R^2$ wie in Anspruch 1 definiert ist und Y für ein Halogenatom steht, mit einer Verbindung der allgemeinen Formel IV

H-A (IV)

in der A wie in Anspruch 1 definiert ist, umsetzt oder

b) zur Herstellung von Verbindungen der allgemeinen Formel I, in der A und $R^2$ wie in Anspruch 1 definiert sind und $R^1$ für eine Aminogruppe steht, eine Verbindung der allgemeinen Formel Ia

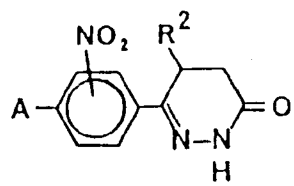

(Ia)

in der $R^2$ und A wie in Anspruch 1 definiert sind und $R^1$ eine Nitrogruppe ist, mit einem Reduktionsmittel reduziert oder

c) zur Herstellung von Verbindungen der allgemeinen Formel I, in der A und $R^2$ die in Anspruch 1 angegebenen Bedeutungen haben und $R^1$ für eine Hydroxygruppe, eine Cyanogruppe oder ein Halogenatom steht, eine Verbindung der allgemeinen Formel Ib

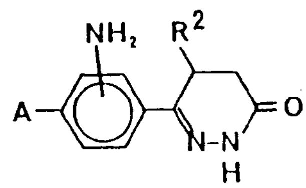

(Ib)

in der A und $R^2$ wie in Anspruch 1 definiert sind, zu einer Verbindung der allgemeinen Formel V

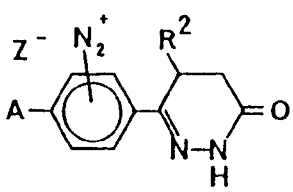

(V)

in der A und $R^2$ wie in Anspruch 1 definiert sind und Z für ein Halogenatom, eine Hydroxygruppe, eine Cyanogruppe oder die Tetrafluoroboratgruppe steht, diazotiert und das Diazoniumsalz der allgemeinen Formel V, gegebenenfalls in Gegenwart von Kupferpulver oder eines Kupfer(I)halogenids oder von Kupfer(I)cyanid, zu einer Verbindung der allgemeinen Formel I, in der $R^2$ und A wie in Anspruch 1 definiert sind und $R^1$ eine Hydroxygruppe, eine Cyanogruppe oder ein Halogenatom ist, umsetzt oder

d) zur Herstellung von Verbindungen der allgemeinen Formel I, in der $R^1$ und $R^2$ wie in Anspruch 1 definiert sind und A für die Gruppe

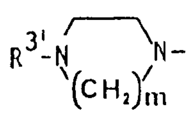

oder die Gruppe $R^{3'}$-NH-$(CH_2)_n$NH- steht, wobei m und n wie in Anspruch 1 definiert sind und $R^{3'}$ für eine Cyanogruppe, eine Acylgruppe, eine gegebenenfalls substituierte Aroylgruppe oder eine $C_1$-$C_6$-Alkoxycarbonylgruppe steht, eine Verbindung der allgemeinen Formeln Ic oder Id

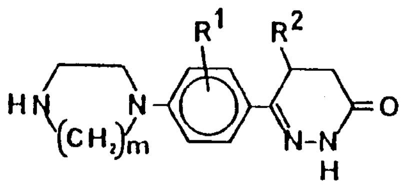

(Ic)

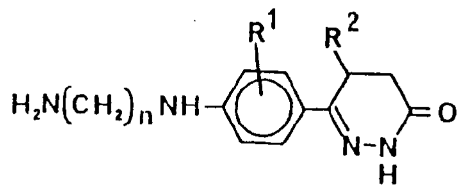

(Id)

in denen $R^1$, $R^2$, m und n die in Anspruch 1 angegebenen Bedeutungen besitzen, mit einem Acylierungsmittel der allgemeinen Formel VI

$R^{3'}$-L (VI)

in der $R^{3'}$ die oben angegebene Bedeutung hat und L für ein Halogenatom, die Gruppe -$OR^{3'}$, die Hydroxygruppe oder den über ein Stickstoffatom gebundenen Rest eines Azols oder Benzazols mit mindestens zwei Stickstoffatomen im Fünfring steht, zur Reaktion bringt oder

e) zur Herstellung von Verbindungen der allgemeinen Formel I, in der $R^1$ und $R^2$ wie in Anspruch 1 definiert sind und A für die Gruppe

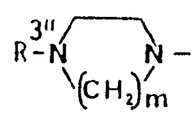

oder die Gruppe $R^{3''}$-NH-$(CH_2)_n$-NH- steht, worin m und n wie in Anspurch 1 definiert sind und $R^{3''}$ für ein Wasserstoffatom steht, eine Verbindung der allgemeinen Formel I, in der $R^1$ und $R^2$ wie in Anspruch 1 definiert sind und A für die Gruppen

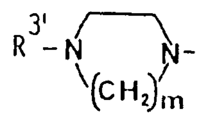

oder $R^{3'}$-NH-$(CH_2)_n$-NH- steht, worin $R^{3'}$ eine Cyanogruppe, eine Acylgruppe, eine gegebenenfalls substituierte Aroylgruppe oder eine $C_1$-$C_6$-Alkoxycarbonylgruppe ist, sauer oder basisch hydrolysiert und gegebenenfalls decarboxyliert

und daß man die nach den Verfahrensvarianten a) bis e) erhaltene Verbindung der allgemeinen Formel I gegebenenfalls in an sich bekannter Weise in ein physiologisch annehmbares Salz umwandelt.

## Claims

### Claims for the following Contracting States : AT, BE, CH, DE, FR, GB, IT, LI, LU, NL, SE

1. 6-Phenyldihydro-3(2H)-pyridazinones corresponding to the general formula I

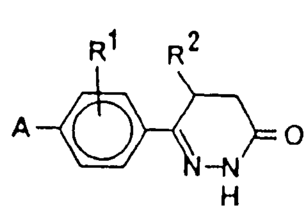

(I)

in which $R^1$ denotes a nitro group, an amino group, a hydroxyl group, a cyano group or a halogen atom, $R^2$ stands for a hydrogen atom, a methyl group or a hydroxymethyl group and
A stands for the group

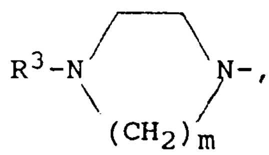

$R^3-NH-(CH_2)_n-NH-$,

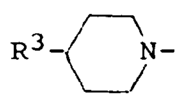

or

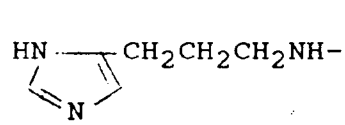

wherein $R^3$ denotes a hydrogen atom, a $C_1$-$C_6$-alkyl group optionally substituted with an amino or hydroxyl group, an optionally substituted aryl or heteroaryl group, a cyano group, an acyl group, an optionally substituted aroyl group, a carboxyl group or a $C_1$-$C_6$-alkoxycarbonyl group, m stands for an integer from 1 to 3 and n stands for an integer from 2 to 4, and the physiologically acceptable salts thereof.

2. 6-Phenyldihydro-3(2H)-pyridazinones according to Claim 1, characterised in that $R^1$ stands for a nitro group, an amino group, a hydroxyl group, a cyano group or a halogen atom, $R^2$ stands for a hydrogen atom, a methyl group or a hydroxymethyl group and
A stands for the group

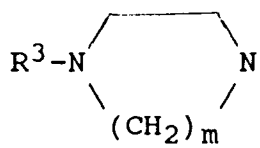

wherein $R^3$ denotes a hydrogen atom, a $C_1$-$C_6$-alkyl group optionally substituted with an amino or hydroxyl group, an optionally substituted aryl or heteroaryl group, a cyano group, an acyl group, an optionally substituted aroyl group or a $C_1$-$C_6$-alkoxycarbonyl group and m stands for an integer from 1 to 3.

3. 6-Phenyldihydro-3(2H)-pyridazinone according to Claim 1, characterised in that $R^1$ stands for a nitro group, an amino group, a hydroxyl group, a cyano group or a halogen atom, $R^2$ stands for a hydrogen atom or a methyl group and A stands for the group $R^3$-H-$(CH_2)_n$-NH wherein $R^3$ denotes a hydrogen atom, an optionally substituted aryl or heteroaryl group, an acyl group, an optionally substituted aroyl group or a $C_1$-$C_6$-alkoxycarbonyl group and n stands for an integer from 2 to 4.

4. 6-Phenyldihydro-3(2H)-pyridazinones according to Claim 1, characterised in that $R^1$ stands for a nitro group, an amino group, a hydroxyl group, a cyano group or a halogen atom, $R^2$ stands for a hydrogen atom or a methyl group and
A denotes the group

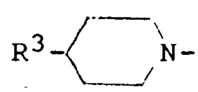

wherein $R^3$ denotes a $C_1$-$C_6$-alkyl group optionally substituted with an amino or hydroxyl group, an optionally substituted aryl or heteroaryl group, an acyl group, an optionally substituted aroyl group, a carboxyl group or a $C_1$-$C_6$-alkoxycarbonyl group.

5. 6-Phenyldihydro-3(2H)-pyridazinones according to Claim 1, characterised in that $R^1$ stands for a nitro group, an amino group, a hydroxyl group, a cyano group or a halogen atom, $R^2$ stands for a hydrogen atom, a methyl group or a hydroxymethyl group and A stands for the group

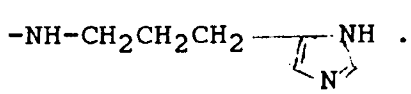

6. 6-[3-Nitro-4-(1-piperazinyl)-phenyl]-4,5-dihydro-5-methyl-3(2H)-pyridazinone and the physiologically acceptable salts.

7. 6-[4-(4-Ethoxycarbonylpiperazin-1-yl)-3-nitro-phenyl]-4,5-dihydro-5-methyl-3(2H)-pyridazinone and the physiologically acceptable salts.

8. 6-[3-Amino-4-(4-ethoxycarbonylpiperazin-1-yl)-phenyl]-4,5-dihydro-5-methyl-3(2H)-pyridazinone and the physiologically acceptable salts.

9. 6-[4-(4-Acetylpiperazin-1-yl)-3-nitrophenyl]-4,5-dihydro-5-methyl-3(2H)-pridazinone and the physiologically acceptable salts.

10. 6-[4-(4-Ethoxycarbonylpiperazin-1-yl)-3-nitro-phenyl]-4,5-dihydro-3(2H)-pyridazinone and the physiologically acceptable salts.

11. A process for the preparation of 6-phenyldihydro-3(2H)-pyridazinones according to Claims 1 to 10, characterised in that a compound corresponding to the general formula II

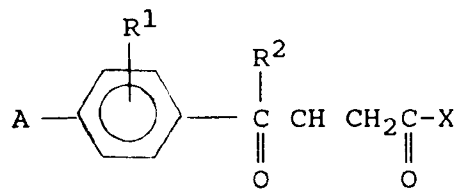

(II)

in which A, $R^1$ and $R^2$ are defined as in Claim 1 and X stands for the group OH or the group $OR^4$ wherein $R^4$ denotes an optionally substituted $C_1$-$C_6$-alkyl group or an optionally susbtituted aryl group is reacted with hydrazine or a chemical equivalent thereof to form a compound corresponding to the general formula I and the compound obtained is optionally converted into its physiologically acceptable salt in known manner.

12. A process for the preparation of 6-phenyldihydro-3(2H)-pyridazinones according to Claims 1 to 10, characterised in that

a) for the preparation of compounds corresponding to the general formula I in which A and $R^2$ have the meanings defined in Claim 1 and $R^1$ stands for a nitro group, a compound corresponding to the general formula III

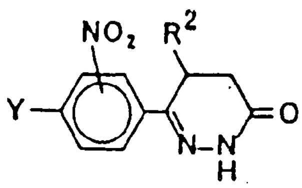

(III)

in which $R^2$ has the meaning defined in Claim 1 and Y stands for a halogen atom is reacted with a compound corresponding to the general formula IV

H-A (IV)

in which A has the meaning defined in Claim 1 or

b) for the preparation of compounds corresponding to the general formula I in which A and $R^2$ have the meanings defined in Claim 1 and $R^1$ stands for an amino group, a compound corresponding to the general formula Ia

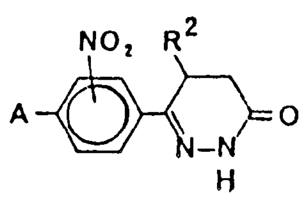

(Ia)

in which $R^2$ and A have the meanings defined in Claim 1 and $R^1$ stands for a nitro group is reduced with a reducing agent or

c) for the preparation of compounds corresponding to the general formula I in which A and $R^2$ have the meanings indicated in Claim 1 and $R^1$ stands for a hydroxyl group, a cyano group or a halogen atom, a

compound corresponding to the general formula Ib

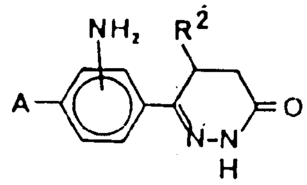

(Ib)

in which A and $R^2$ have the meanings defined in Claim 1 is diazotised to a compound corresponding to the general formula V

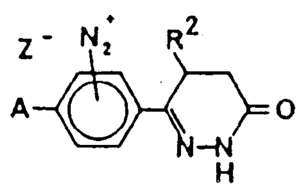

(V)

in which A and $R^2$ have the meanings defined in Claim 1 and Z stands for a halogen atom, a hydroxyl group, a cyano group or the tetrafluoroborate group, and the diazonium salt corresponding to the general formula V is converted into a compound corresponding to the general formula I, in which $R^2$ and A have the meanings defined in Claim 1 and $R^1$ stands for a hydroxyl group, a cyano group or a halogen atom, optionally in the presence of copper powder or a copper(I) halide or copper(I) cyanide, or

d) for the preparation of compounds corresponding to the general formula I in which $R^1$ and $R^2$ have the meanings defined in Claim 1 and A stands for the group

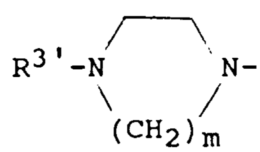

or the group $R^{3'}$-NH-$(CH_2)_n$NH- wherein m and n have the meanings defined in Claim 1 and $R^{3'}$ stands for a cyano group, an acyl group, an optionally substituted aroyl group or a $C_1$-$C_6$-alkoxycarbonyl group, a compound corresponding to the general formula Ic or Id

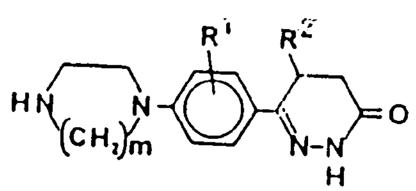

(Ic)

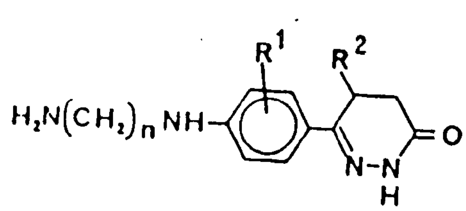

(Id)

in which $R^1$, $R^2$, m and n have the meanings indicated in Claim 1 is reacted with an acylating agent corresponding to the general formula VI

$R^{3'}$-L (VI)

in which $R_3'$ has the meaning indicated above and L stands for a halogen atom, the group $OR^{3'}$, the hydroxyl group or an azole or benzazole residue attached via a nitrogen atom and having at least two nitrogen atoms in the five-membered ring, or

e) for the preparation of compounds corresponding to the general formula I in which $R^1$ and $R^2$ are defined as in Claim 1 and A stands for the group

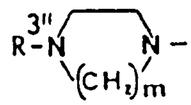

or the group $R^{3''}$-NH-$(CH_2)_n$-NH- wherein m and n have the meanings defined in Claim 1 and $R^{3''}$ stands for a hydrogen atom, a compound corresponding to the general formula I in which $R^1$ and $R^2$ have the meanings defined in Claim 1 and A stands for the group

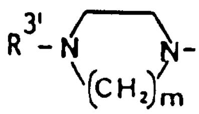

or $R^{3'}$-NH-$(CH_2)_n$-NH- wherein $R^{3'}$ denotes a cyano group, an acyl group, an optionally substituted aroyl group or a $C_1$-$C_6$-alkoxycarbonyl group is hydrolysed under acid or basic conditions and optionally decarboxylated

and in that the compound corresponding to the general formula I obtained by process variations a) to e) is optionally converted into a physiologically acceptable salt in known manner.

13. Pharmaceutical preparation, characterised in that it contains a compound according to one of the Claims 1 to 10 together with at least one inert, pharmaceutically acceptable carrier or diluent.

## Claims

### Claims for the following Contracting State : ES

1. A process for the preparation of 6-phenyldihydro-3(2H)-pyridazinones corresponding to the general formula I

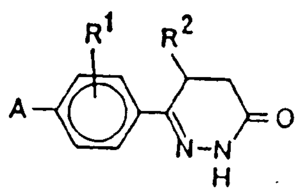

(I)

in which $R^1$ denotes a nitro group, an amino group, a hydroxyl group, a cyano group or a halogen atom,
$R^2$ stands for a hydrogen atom, a methyl group or a hydroxymethyl group and
A stands for the group

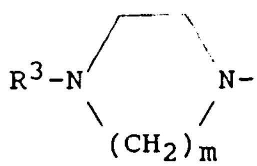

, $R^3$-NH-$(CH_2)_n$-NH-,

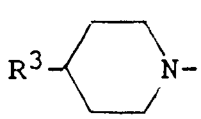

or

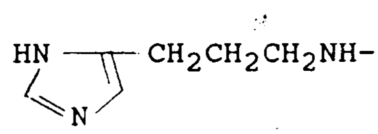

wherein $R^3$ denotes hydrogen atom, a $C_1$-$C_6$-alkyl group optionally substituted with an amino or hydroxyl group, an optionally substituted aryl or heteroaryl group, a cyano group, an acyl group, an optionally substituted aroyl group, a carboxyl group or a $C_1$-$C_6$-alkoxycarbonyl group, m stands for an integer from 1 to 3 and n stands for an integer from 2 to 4 and the physiologically acceptable salts thereof, characterised in that a compound corresponding to the general formula II

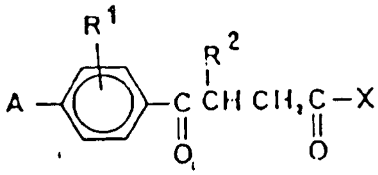

(II)

wherein A, $R^1$ and $R^2$ have the meanings defined above and X stands for the group OH or the group $OR^4$ in which $R^4$ denotes an optionally substituted $C_1$-$C_6$-alkyl group or an optionally substituted aryl group is converted by reaction with hydrazine or a chemical equivalent thereof into a compound corresponding to the general formula I and the compound obtained is optionally converted into its physiologically acceptable salt in known manner.

2. A process for the preparation of 6-phenyldihydro-3(2H)-pyridazinones corresponding to the general formula I

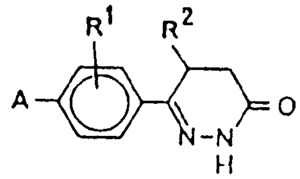

(I)

in which $R^1$ denotes a nitro group, an amino group, a hydroxyl group, a cyano group or a halogen atom, $R^2$ stands for a hydrogen atom, a methyl group or a hydroxymethyl group and
A stands for the group

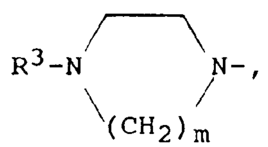, $R^3-NH-(CH_2)_n-NH-$,

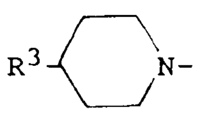 or 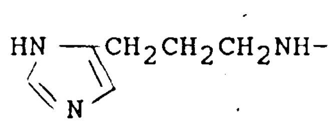

wherein $R^3$ denotes a hydrogen atom, a $C_1$-$C_6$-alkyl group optionally substituted with an amino or hydroxyl group, an optionally substituted aryl or heteroaryl group, a cyano group, an acyl group, an optionally substituted aroyl group, a carboxyl group or a $C_1$-$C_6$-alkoxycarbonyl group, m stands for an integer from 1 to 3 and n stands for an integer from 2 to 4, and the physiologically acceptable salts thereof, characterised in that

a) for the preparation of compounds corresponding to the general formula I in which A and $R^2$ have the meanings defined above and $R^1$ stands for a nitro group, a compound corresponding to the general formula III

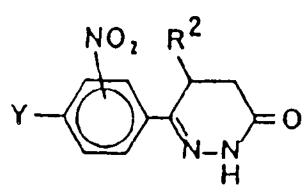

(III)

in which $R^2$ has the meaning defined above and Y stands for a halogen atom is reacted with a compound corresponding to the general formula IV

H-A (IV)

in which A has the meaning defined above or

b) for the preparation of compounds corresponding to the general formula I in which A and $R^2$ have the meanings defined above and $R^1$ stands for an amino group, a compound corresponding to the general formula Ia

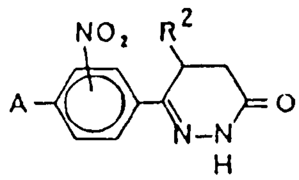

(Ia)

in which $R^2$ and A have the meanings defined above and $R^1$ stands for a nitro group, is reduced with a reducing agent or

c) for the preparation of compounds corresponding to the general formula I in which A and $R^2$ have the meanings indicated above and $R^1$ stands for a hydroxyl group, a cyano group or a halogen atom, a compound corresponding to the general formula Ib

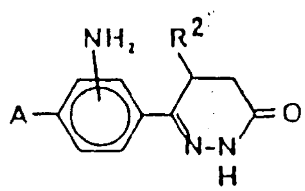

(Ib)

in which A and $R^2$ have the meanings defined in Claim 1 is diazotised to a compound corresponding to the general formula V

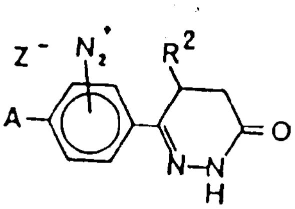

(V)

in which A and $R^2$ have the meanings defined above and Z stands for a halogen atom, a hydroxyl group, a cyano group or the tetrafluoroborate group, and the diazonium salt corresponding to the general formula V is converted into a compound corresponding to the general formula I in which $R^2$ and A have the meanings defined above and $R^1$ stands for a hydroxyl group, a cyano group or a halogen atom, optionally in the presence of copper powder or of a copper(I) halide or of copper(I) cyanide, or

d) for the preparation of compounds corresponding to the general formula I in which $R^1$ and $R^2$ have the meanings defined above and A stands for the group

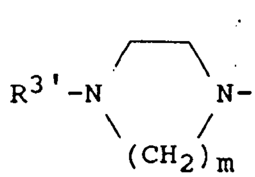

or the group $R^{3'}$-NH-$(CH_2)_n$NH- wherein m and n have the meanings defined above and $R^{3'}$ stands for a cyano group, an acyl group, an optionally substituted aroyl group or a $C_1$-$C_6$-alkoxycarbonyl group, a compound corresponding to the general formula Ic or Id

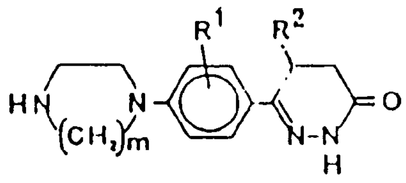

(Ic)

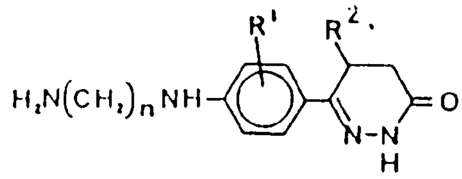

(Id)

in which $R^1$, $R^2$, m and n have the meanings indicated above, is reacted with an acylating agent corresponding to the general formula VI

$R^{3'}$-L (VI)

in which $R^{3'}$ has the meaning indicated above and L stands for a halogen atom, the group $OR^{3'}$, the hydroxyl group or the residue of an azole or benzazole having at least two nitrogen atoms in the five-membered ring and attached via a nitrogen atom, or

e) for the preparation of compounds corresponding to the general formula I in which $R^1$ and $R^2$ have the meanings defined above and A stands for the group

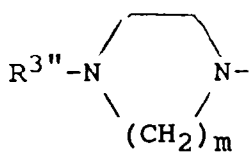

or the group $R^{3''}$-NH-$(CH_2)_n$-NH- wherein m and n have the meanings defined above and $R^{3''}$ stands for a hydrogen atom, a compound corresponding to the general formula I in which $R^1$ and $R^2$ have the meanings defined above and A stands for the group

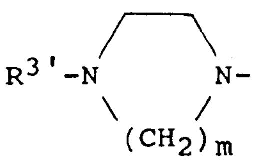

or $R^{3'}$-NH-$(CH_2)_n$-NH- wherein $R^{3'}$ denotes a cyano group, an acyl group, an optionally substituted aroyl group or a $C_1$-$C_6$-alkoxycarbonyl group is hydrolysed under acid or basic conditions and optionally decarboxylated

and in that the compound corresponding to the general formula I obtained by process variations a) to e) is optionally converted into a physiologically acceptable salt in known manner.

3. A process according to Claim 1 or Claim 2 for the preparation of compounds in which $R^1$ stands for a nitro group, an amino group, a hydroxyl group, a cyano group or a halogen atom, $R^2$ stands for a hydrogen atom, a methyl group or a hydroxymethyl group and

A stands for the group

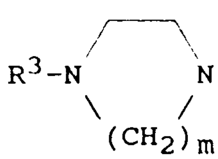

wherein $R^3$ denotes a hydrogen atom, a $C_1$-$C_6$-alkyl group optionally substituted with an amino or hydroxyl group, an optionally substituted aryl or heteroaryl group, a cyano group, an acyl group, an optionally substituted aroyl group or a $C_1$-$C_6$-alkoxycarbonyl group and m stands for an integer from 1 to 3.

4. A process according to Claim 1 or Claim 2 for the preparation of compounds in which $R^1$ stands for a nitro group, an amino group, a hydroxyl group, a cyano group or a halogen atom, $R^2$ stands for a hydrogen atom or a methyl group and A stands for the group $R^3$-NH-$(CH_2)_n$-NH wherein $R^3$ denotes a hydrogen atom, an optionally substituted aryl or heteroaryl group, an acyl group, an optionally substituted aroyl group or a $C_1$-$C_6$-alkoxycarbonyl group and n stands for an integer from 2 to 4.

5. A process according to Claim 1 or Claim 2 for the preparation of compounds in which $R^1$ stands for a nitro group, an amino group, a hydroxyl group, a cyano group or a halogen atom, $R^2$ denotes a hydrogen atom or a methyl group and A denotes the group

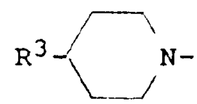

wherein $R^3$ stands for a $C_1$-$C_6$-alkyl group optionally substituted with an amino or hydroxyl group, an optionally substituted aryl or heteroaryl group, an acyl group, an optionally substituted aroyl group, a carboxyl group or a $C_1$-$C_6$-alkoxycarbonyl group.

6. A process according to Claim 1 or Claim 2 for the preparation of compounds in which $R^1$ stands for a nitro group, an amino group, a hydroxyl group, a cyano group or a halogen atom, $R^2$ denotes a hydrogen atom, a methyl group or a hydroxymethyl group and A stands for the group

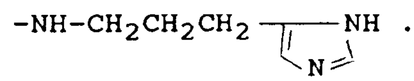

7. A process according to Claim 1 or Claim 2 for the preparation of 6-[3-nitro-4-(1-piperazinyl)-phenyl]-4,5-dihydro-5-methyl-3(2H)-pyridazinone and the physiologically acceptable salts.

8. A process according to Claim 1 or Claim 2 for the preparation of 6-[4-(4-ethoxycarbonylpiperazin-1-yl)-3-nitropheny1]-4,5-dihydro-5-methyl-3(2H)-pyridazinone and the physiologically acceptable salts.

9. A process according to Claim 1 or Claim 2 for the preparation of 6-[3-amino-4-(4-ethoxycarbonyl-piperazin-1-yl)-phenyl]-4,5-dihydro-5-methyl-3(2H)-pyridazinone and the physiologically acceptable salts.

10. A process according to Claim 1 or Claim 2 for the preparation of 6-[4-(4-acetylpiperazin-1-yl)-3-nitro-phenyl]-4,5-dihydro-5-methyl-3(2H)-pyridazinone and the physiologically acceptable salts.

11. A process according to Claim 1 or Claim 2 for the preparation of 6-[4-(4-ethoxycarbonylpiperazin-1-yl)-3-nitrophenyl]-4,5-dihydro-3(2H)-pyridazinone and the physiologically acceptable salts.

**Claims for the following Contracting State : GR**

1. 6-Phenyldihydro-3(2H)-pyridazinone corresponding to the general formula I

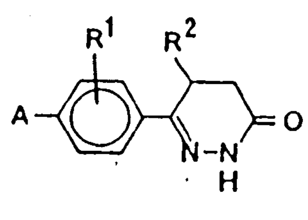

(I)

in which $R^1$ denotes a nitro group, an amino group, a hydroxyl group, a cyano group or a halogen atom, $R^2$ stands for a hydrogen atom, a methyl group or a hydroxymethyl group and
A stands for the group

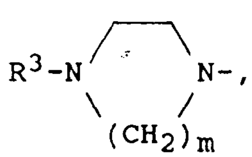 , $R^3-NH-(CH_2)_n-NH-$,

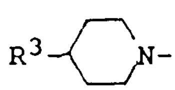 or 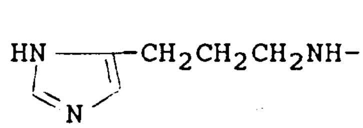

wherein $R^3$ denotes a hydrogen atom, a $C_1$-$C_6$-alkyl group optionally substituted with an amino or hydroxyl

group, an optionally substituted aryl or heteroaryl group, a cyano group, an acyl group, an optionally substituted aroyl group, a carboxyl group or a $C_1$-$C_6$-alkoxycarbonyl group, m stands for an integer from 1 to 3 and n stands for an integer from 2 to 4, and the salts thereof.

2. 6-Phenyldihydro-3(2H)-pyridazinones according to Claim 1, characterised in that $R^1$ stands for a nitro group, an amino group, a hydroxyl group, a cyano group or a halogen atom, $R^2$ stands for a hydrogen atom, a methyl group or a hydroxymethyl group and
A stands for the group

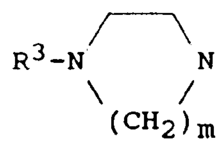

wherein $R^3$ denotes hydrogen atom, a $C_1$-$C_6$-alkyl group optionally substituted with an amino or hydroxyl group, an optionally substituted aryl or heteroaryl group, a cyano group, an acyl group, an optionally substituted aroyl group or a $C_1$-$C_6$-alkoxycarbonyl group and m stands for an integer from 1 to 3.

3. 6-Phenyldihydro-3(2H)-pyridazinones according to Claim 1, characterised in that $R^1$ stands for a nitro group, an amino group, a hydroxyl group, a cyano group or a halogen atom, $R^2$ stands for a hydrogen atom or a methyl group and A stands for the group $R^3$-NH-$(CH_2)_n$-NH wherein $R^3$ denotes a hydrogen atom, an optionally substituted aryl or heteroaryl group, an acyl group, an optionally substituted aroyl group or a $C_1$-$C_6$-alkoxycarbonyl group and n stands for an integer from 2 to 4.

4. 6-Phenyldihydro-3(2H)-pyridazinones according to Claim 1, characterised in that $R^1$ stands for a nitro group, an amino group, a hydroxyl group, a cyano group or a halogen atom, $R^2$ stands for a hydrogen atom or a methyl group and
A denotes the group

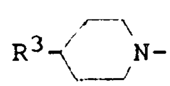

wherein $R^3$ stands for a $C_1$-$C_6$-alkyl group optionally substituted with an amino or hydroxyl group, an optionally substituted aryl or heteroaryl group, an acyl group, an optionally substituted aroyl group, a carboxyl group or a $C_1$-$C_6$-alkoxycarbonyl group.

5. 6-Phenyldihydro-3(2H)-pyridazinones according to Claim 1, characterised in that $R^1$ stands for a nitro group, an amino group, a hydroxyl group, a cyano group or a halogen atom, $R^2$ stands for a hydrogen atom, a methyl group or a hydroxymethyl group and A stands for the group

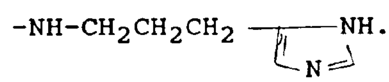

6. 6-[3-Nitro-4-(1-piperazinyl)-phenyl]-4,5-dihydro-5-methyl-3(2H)-pyridazinone and the salts.

7. 6-[4-(4-Ethoxycarbonylpiperazin-1-yl)-3-nitro-phenyl]-4,5-dihydro-5-methyl-3(2H)-pyridazinone and the salts.

8. 6-[3-Amino-4-(4-ethoxycarbonylpiperazin-1-yl)-phenyl]-4,5-dihydro-5-methyl-3(2H)-pyridazinone and the salts.

9. 6-[4-(4-Acetylpiperazin-1-yl)-3-nitrophenyl]-4,5-dihydro-5-methyl-3(2H)-pyridazinone and the salts.

10. 6-[4-(4-Ethoxycarbonylpiperazin-1-yl)-3-nitro-phenyl]-4,5-dihydro-3(2H)-pyridazinone and the salts.

11. A process for the preparation of 6-phenyldihydro-3(2H)-pyridazinones according to Claims 1 to 10, characterised in that a compound corresponding to the general formula II

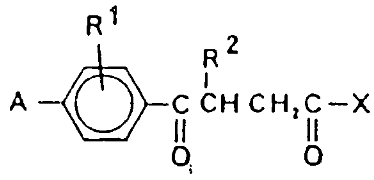 (II)

in which A, $R^1$ and $R^2$ have the meanings defined in Claim 1 and X stands for the group OH or the group $OR^4$ in which $R^4$ denotes an optionally substituted $C_1$-$C_6$-alkyl group or an optionally substituted aryl group is reacted with hydrazine or a chemical equivalent thereof to form a compound corresponding to the general formula I and the compound obtained is optionally converted into its physiologically acceptable salt in known manner.

12. A process for the preparation of 6-phenyldihydro-3(2H)-pyridazinones according to Claims 1 to 10, characterised in that

a) for the preparation of compounds corresponding to the general formula I in which A and $R^2$ have the meanings defined in Claim 1 and $R^1$ stands for a nitro group, a compound corresponding to the general formula III

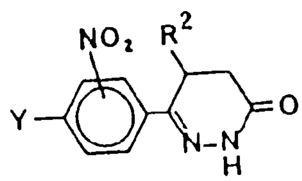 (III)

in which $R^2$ has the meaning defined in Claim 1 and Y stands for a halogen atom is reacted with a compound corresponding to the general formula IV

H-A (IV)

in which A has the meaning defined in Claim 1 or

b) for the preparation of compounds corresponding to the general formula I in which A and $R^2$ have the meanings defined in Claim 1 and $R^1$ stands for an amino group, a compound corresponding to the general formula Ia

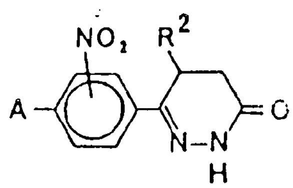 (Ia)

in which $R^2$ and A have the meanings defined in Claim 1 and $R^1$ stands for a nitro group is reduced with a reducing agent or

c) for the preparation of compounds corresponding to the general formula I in which A and $R^2$ have the meanings indicated in Claim 1 and $R^1$ stands for a hydroxyl group, a cyano group a halogen atom, a compound corresponding to the general formula Ib

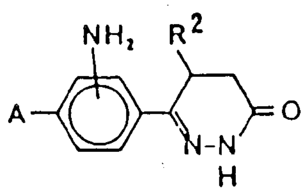 (Ib)

in which A and $R^2$ have the meanings defined in Claim 1 is diazotised to a compound corresponding to the general formula V

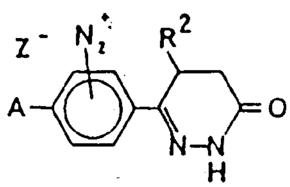

(V)

in which A and $R^2$ have the meanings defined in Claim 1 and Z stands for a halogen atom, a hydroxyl group, a cyano group or the tetrafluoroborate group, and the diazonium salt corresponding to the general formula V is converted into a compound corresponding to the general formula I in which $R^2$ and A have the meanings defined in Claim 1 and $R^1$ stands for a hydroxyl group, a cyano group or a halogen atom, optionally in the presence of copper powder or a copper(I) halide or copper(I) cyanide, or

d) for the preparation of compounds corresponding to the general formula I in which $R^1$ and $R^2$ have the meanings defined in Claim 1 and A stands for the group

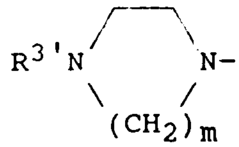

or the group $R^{3'}$-NH-$(CH_2)_n$NH- wherein m and n have the meanings defined in Claim 1 and $R^{3'}$ stands for a cyano group, an acyl group, an optionally substituted aroyl group or a $C_1$-$C_6$-alkoxycarbonyl group, a compound corresponding to the general formula Ic or Id

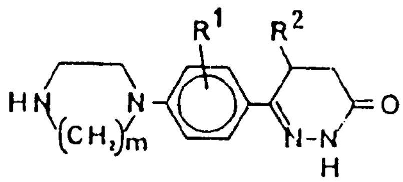

(Ic)

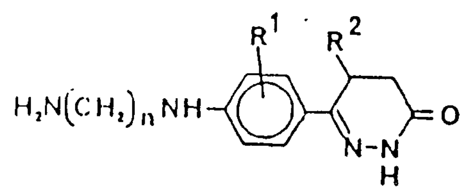

(Id)

in which $R^1$, $R^2$, m and n have the meanings indicated in Claim 1 is reacted with an acylating agent corresponding to the general formula VI

$R^{3'}$-L (VI)

in which $R^{3'}$ has the meaning indicated above and L stands for a halogen atom, the group $OR^{3'}$, the hydroxyl group or the residue of an azole or benzazole having at least two nitrogen atoms in the 5-membered ring and attached via a nitrogen atom, or

e) for the preparation of compounds corresponding to the general formula I in which $R^1$ and $R^2$ have the meanings defined in Claim 1 and A stands for the group

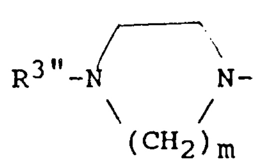

or the group $R^{3''}$-NH-$(CH_2)_n$-NH- wherein m and n have the meanings defined in Claim 1 and $R^{3''}$ stands for a hydrogen atom, a compound corresponding to the general formula I in which $R^1$ and $R^2$ have the meanings defined in Claim 1 and A stands for the group

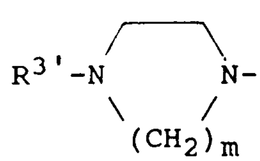

or $R^{3'}$-NH-$(CH_2)_n$-NH- wherein $R^{3'}$ denotes a cyano group, an acyl group, an optionally substituted aroyl group or a $C_1$-$C_6$-alkoxycarbonyl group is hydrolysed under acid or basic conditions and optionally decarboxylated

and in that the compounds corresponding to the general formula I obtained by process variations a) to e) are optionally converted into a physiologically acceptable salt in known manner.

## Revendications

### Revendications pour les Etats contractants suivants : AT, BE, CH, DE, FR, GB, IT, LI, LU, NL, SE

1. 6-phényldihydro-3(2H)-pyridazinones, caractérisées en ce qu'elles répondent à la formule générale I :

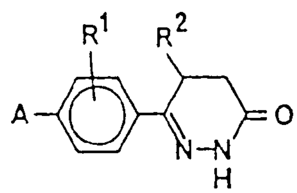

(I)

dans laquelle $R^1$ représente un groupe nitro, un groupe amino, un groupe hydroxy, un groupe cyano ou un atome d'halogène,
$R^2$ représente un atome d'hydrogène, un groupe méthyle ou un groupe hydroxyméthyle et
A représente le groupe

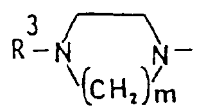 , $R^3$-NH-$(CH_2)_n$-NH-, 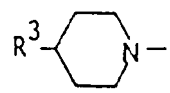

ou 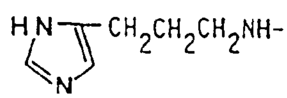 ,

dans lesquels $R^3$ représente un atome d'hydrogène, un groupe alkyle en $C_1$-$C_6$ éventuellement substitué par un groupe amino ou hydroxy, un groupe aryle ou hétéroaryle éventuellement substitué, un groupe cyano, un groupe acyle, un groupe aroyle éventuellement substitué, un groupe carboxyle ou un groupe (alcoxy en $C_1$-$C_6$)carbonyle, m est un entier de 1 à 3 et n est un entier de 2 à 4, ainsi que leurs sels physiologiquement acceptables.

2. 6-phényldihydro-3(2H)-pyridazinones selon la revendication 1, caractérisées en ce que $R^1$ représente un groupe nitro, un groupe amino, un groupe hydroxy, un groupe cyano ou un atome d'halogène, $R^2$ représente un atome d'hydrogène, un groupe méthyle ou un groupe hydroxyméthyle et A représente le groupe

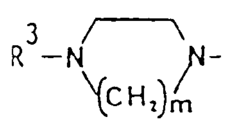

,dans lequel $R^3$ est un atome d'hydrogène, un groupe alkyle en $C_1$-$C_6$ éventuellement substitué par un groupe amino ou un groupe hydroxy, un groupe aryle ou hétéroaryle éventuellement substitué, un groupe cyano, un groupe acyle, un groupe aroyle éventuellement substitué ou un groupe (alcoxy en $C_1$-$C_6$)carbonyle et m est un entier de 1 à 3.

3. 6-phényldihydro-3(2H)-pyridazinones selon la revendication 1, caractérisées en ce que $R^1$ représente un groupe nitro, un groupe amino, un groupe hydroxy, un groupe cyano ou un atome d'halogène, $R^2$ représente un atome d'hydrogène ou un groupe méthyle et A représente le groupe $R^3$-NH$(CH_2)_n$-NH-, dans lequel $R^3$ représente un atome d'hydrogène, un groupe aryle ou hétéroaryle éventuellement substitué, un groupe acyle, un

groupe aroyle ou un groupe (alcoxy en $C_1$-$C_6$)carbonyle et n est un entier de 2 à 4.

4. 6-phényldihydro-3(2H)-pyridazinones selon la revendication 1, caractérisées en ce que $R^1$ représente un groupe nitro, un groupe amino, un groupe hydroxy, un groupe cyano ou un atome d'halogène, $R^2$ représente un atome d'hydrogène ou un groupe méthyle et A représente le groupe

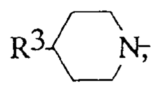

dans lequel $R^3$ représente un groupe alkyle en $C_1$-$C_6$ éventuellement substitué par un groupe amino ou un groupe hydroxy, un groupe aryle ou hétéroaryle éventuellement substitué, un groupe acyle, un groupe aroyle éventuellement substitué, un groupe carboxyle ou un groupe (alcoxy en $C_1$-$C_6$)carbonyle.

5. 6-phényldihydro-3(2H)pyridazinones selon la revendication 1, caractérisées en ce que $R^1$ représente un groupe nitro, un groupe amino, un groupe hydroxy, un groupe cyano ou un atome d'halogène, $R^2$ représente un atome d'hydrogène, un groupe méthyle ou un groupe hydroxyméthyle et A représente le groupe

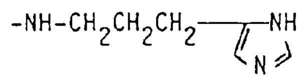

6. La 6-[3-nitro-4-(1-pipérazinyl)phényl]-4,5-dihydro-5-méthyl-3(2H)-pyridazinone et ses sels physiologiquement acceptables.

7. La 6-[4-(4-éthoxycarbonylpipérazine-1-yl)-3-nitrophényl]-4,5-dihydro-5-méthyl-3(2H)pyridazinone et ses sels physiologiquement acceptables.

8. La 6-[3-amino-4-(4-éthoxycarbonylpipérazine-1-yl)-phényl] -4,5-dihydro-5-méthyl-3(2H)-pyridazinone et ses sels physiologiquement acceptables.

9. La 6-[4-(4-acétylpipérazine-1-yl)-3-nitrophényl]-4,5-di-hydro-5-méthyl-3(2H)-pyridazinone et ses sels physiologiquement acceptables.

10. La 6-[4-(4-éthoxycarbonylpipérazine-1-yl)-3-nitro-phényl]-4,5-dihydro-3(2H)-pyridazinone et ses sels physiologiquement acceptables.

11. Procédé pour la fabrication de 6-phényldihydro-3(2H)-pyridazinones selon les revendications 1 à 10, caractérisé en ce que l'on fait réagir un composé de formule générale II :

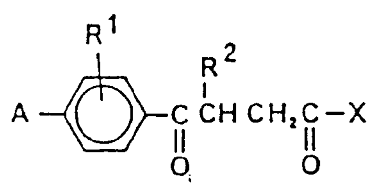

(II)

dans laquelle A, $R^1$ et $R^2$ sont définis comme à la revendication 1 et X représente le groupe -OH ou le groupe $-OR^4$ dans lequel $R^4$ est un groupe alkyle en $C_1$-$C_6$ éventuellement substitué ou un groupe aryle éventuellement substitué, avec l'hydrazine ou l'un des ses équivalents chimiques pour obtenir le composé de formule générale I et on transforme éventuellement de manière connue le composé obtenu en son sel physiologiquement acceptable.

12. Procédé pour la fabrication de 6-phényldihydro-3(2H)-pyridazinones selon les revendications 1 à 10, caractérisé en ce que

a) pour la fabrication de composés de formule générale I dans laquelle A et $R^2$ sont définis comme à la revendication 1 et $R^1$ représente un groupe nitro, on fait réagir un composé de formule générale III :

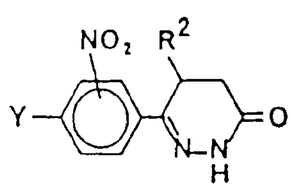

(III)

dans laquelle $R^2$ est défini comme à la revendication 1 et Y représente un atome d'halogène avec un composé de formule générale IV

H-A (IV)

dans laquelle A est défini comme à la revendication 1, ou bien

b) pour la fabrication de composés de formule générale I dans laquelle A et $R^2$ sont définis comme à la revendication I et $R^1$ représente un groupe amino, on réduit un composé de formule générale Ia

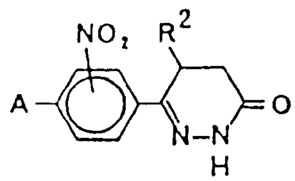

(Ia)

dans laquelle $R^2$ et A sont définis comme à la revendication 1 et $R^1$ représente un groupe nitro, par un agent réducteur, ou bien

c) pour la fabrication de composés de formule générale I dans laquelle $R^1$ et $R^2$ ont les significations indiquées dans la revendication 1 et $R^1$ représente un groupe hydroxy, un groupe cyano ou un atome d'halogène, on diazote un composé de formule générale Ib

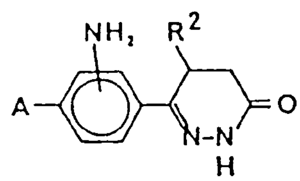

(Ib)

dans laquelle A et $R^2$ sont définis comme à la revendication 1, en un composé de formule générale V

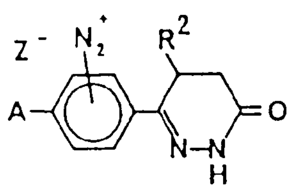

(V)

dans laquelle A et $R^2$ sont définis comme à la revendication 1 et Z représente un atome d'halogène, un groupe hydroxy, un groupe cyano ou un groupe tétrafluoroborate, et on fait réagir le sel de diazonium de formule générale V, éventuellement en présence de poudre de cuivre ou d'un halogénure de cuivre (I) ou de cyanure de cuivre (I), en un composé de formule générale I dans laquelle $R^2$ et A sont définis comme à la revendication 1 et $R^1$ représente un groupe hydroxy, un groupe cyano ou un atome d'halogène, ou bien

d) pour la fabrication de composés de formule générale I dans laquelle $R^1$ et $R^2$ sont définis comme à la revendication 1 et A représente le groupe

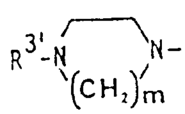

ou le groupe $R^{3'}$-NH-$(CH_2)_n$NH-, dans lesquels m et n sont définis comme à la revendication 1 et $R^{3'}$ représente un groupe cyano, un groupe acyle, un groupe aroyle éventuellement substitué ou un groupe (alcoxy en $C_1$-$C_6$)carbonyle, on fait réagir un composé de formule générale Ic ou Id

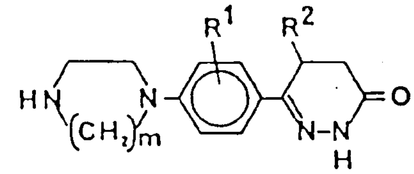 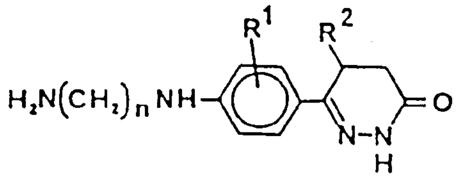

(Ic) (Id)

dans lesquelles $R^1$, $R^2$, m et n ont les significations indiquées dans la revendication 1, avec un agent acylant de formule générale VI

$R^{3'}$-L (VI)

dans laquelle $R^{3'}$ a la signification indiquée ci-dessus et L représente un atome d'halogène, le groupe -$OR^{3'}$, le groupe hydroxy ou le reste d'un azole ou benzazole à au moins 2 atomes d'azote dans le cycle à 5 chaînons, relié par un atome d'azote, ou bien

e) pour la fabrication de composés de formule générale I, dans laquelle $R^1$ et $R^2$ sont définis comme à la revendication 1 et A représente le groupe

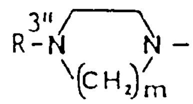

ou le groupe $R^{3''}$-NH-$(CH_2)_n$-NH-, dans lesquels m et n sont définis comme à la revendication 1 et $R^{3''}$ représente un atome d'hydrogène, on soumet à l'hydrolyse acide ou basique et éventuellement on décarboxyle un composé de formule générale I, dans laquelle $R^1$ et $R^2$ sont définis comme à la revendication 1 et A représente le groupe

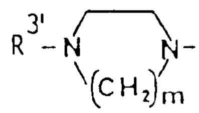

ou $R^{3'}$-NH-$(CH_2)_n$, dans lesquels $R^{3'}$ représente un groupe cyano, un groupe acyle, un groupe aroyle éventuellement substitué ou un groupe (alcoxy en $C_1$-$C_6$) carbonyle

et en ce que l'on transforme éventuellement de manière connue le composé de formule générale I obtenu selon les variantes du procédé a) à e) en un sel physiologiquement acceptable.

13. Médicament caractérisé en ce qu'il contient un composé selon l'une des revendications 1 à 10 en association avec au moins un support ou agent diluant inerte pharmaceutiquement acceptable.

**Revendications pour l'Etat contractant suivant : ES**

1. Procédé pour la fabrication de 6-phényldihydro-3(2H)-pyridazinones de formule générale I :

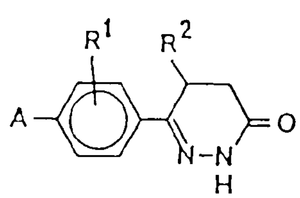

(I)

dans laquelle $R^1$ représente un groupe nitro, un groupe amino, un groupe hydroxy, un groupe cyano ou un atome d'halogène,

$R^2$ représente un atome d'hydrogène, un groupe méthyle ou un groupe hydroxyméthyle et

A représente le groupe

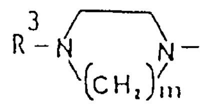 , $R^3$-NH-$(CH_2)_n$-NH-, 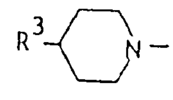

ou 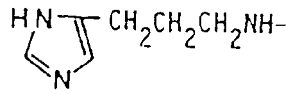 ,

dans lesquels $R^3$ représente un atome d'hydrogène, un groupe alkyle en $C_1$-$C_6$ éventuellement substitué par un groupe amino ou hydroxy, un groupe aryle ou hétéroaryle éventuellement substitué, un groupe cyano, un groupe acyle, un groupe aroyle éventuellement substitué, un groupe carboxyle ou un groupe (alcoxy en $C_1$-$C_6$)carbonyle, m est un entier de 1 à 3 et n est un entier de 2 à 4, et de leurs sels physiologiquement acceptables, caractérisé en ce que l'on fait réagir un composé de formule générale II :

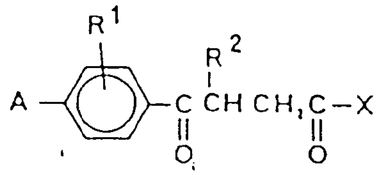 (II)

dans laquelle A, $R^1$ et $R^2$ sont définis comme à la revendication 1 et X représente le groupe -OH ou le groupe -$OR^4$ dans lequel $R^4$ est un groupe alkyle en $C_1$-$C_6$ éventuellement substitué ou un groupe aryle éventuellement substitué, avec l'hydrazine ou l'un des ses équivalents chimiques pour obtenir le composé de formule générale I et on transforme éventuellement de manière connue le composé obtenu en son sel physiologiquement acceptable.

2. Procédé pour la fabrication de 6-phényldihydro-3(2H)-pyridazinones de formule générale I

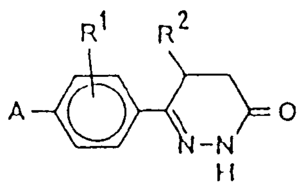 (I)

dans laquelle $R^1$ représente un groupe nitro, un groupe amino, un groupe hydroxy, un groupe cyano ou un atome d'halogène,
$R^2$ représente un atome d'hydrogène, un groupe méthyle ou un groupe hydroxyméthyle et
A représente le groupe

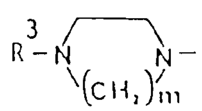 , $R^3$-NH-$(CH_2)_n$-NH-, 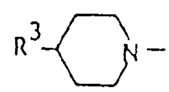

ou 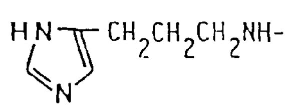 ,

dans lesquels $R^3$ représente un atome d'hydrogène, un groupe alkyle en $C_1$-$C_6$ éventuellement substitué par un groupe amino ou hydroxy, un groupe aryle ou hétéroaryle éventuellement substitué, un groupe cyano, un groupe acyle, un groupe aroyle éventuellement substitué, un groupe carboxyle ou un groupe (alcoxy en $C_1$-$C_6$)carbonyle, m est un entier de 1 à 3 et n est un entier de 2 à 4, et de leurs sels physiologiquement acceptables, caractérisé en ce que

a) pour la fabrication de composés de formule générale I dans laquelle A et $R^2$ sont définis comme ci-dessus et $R^1$ représente un groupe nitro, on fait réagir un composé de formule générale III :

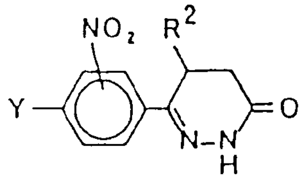

(III)

dans laquelle $R^2$ est défini comme à la revendication 1 et Y représente un atome d'halogène avec un composé de formule générale IV

H-A (IV)

dans laquelle A est défini comme ci-dessus 1, ou bien

b) pour la fabrication de composés de formule générale I dans laquelle A et $R^2$ sont définis comme ci-dessus et $R^1$ représente un groupe amino, on réduit un composé de formule générale Ia

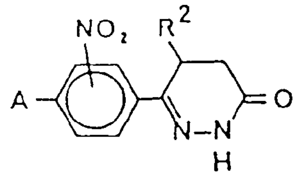

(Ia)

dans laquelle $R^2$ et A sont définis comme ci-dessus 1 et $R^1$ représente un groupe nitro, par un agent réducteur, ou bien

c) pour la fabrication de composés de formule générale I dans laquelle $R^1$ et $R^2$ ont les significations indiquées ci-dessus et $R^1$ représente un groupe hydroxy, un groupe cyano ou un atome d'halogène, on diazote un composé de formule générale Ib

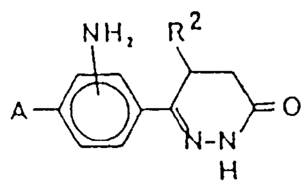

(Ib)

dans laquelle A et $R^2$ sont définis comme à la revendication 1, en un composé de formule générale V

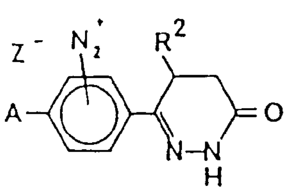

(V)

dans laquelle A et $R^2$ sont définis comme ci-dessus et Z représente un atome d'halogène, un groupe hydroxy, un groupe cyano ou un groupe tétrafluoroborate, et on fait réagir le sel de diazonium de formule générale V, éventuellement en présence de poudre de cuivre ou d'un halogénure de cuivre (I) ou de cyanure de cuivre (I), en un composé de formule générale I dans laquelle $R^2$ et A sont définis comme ci-dessus et $R^1$ représente un groupe hydroxy, un groupe cyano ou un atome d'halogène, ou bien

d) pour la fabrication de composés de formule générale I dans laquelle $R^1$ et $R^2$ sont définis comme ci-dessus et A représente le groupe

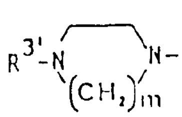

ou le groupe $R^{3'}$-NH-$(CH_2)_n$NH-, dans lesquels m et n sont définis comme ci-dessus et $R^{3'}$ représente un groupe cyano, un groupe acyle, un groupe aroyle éventuellement substitué ou un groupe (alcoxy en $C_1$-

$C_6$)carbonyle, on fait réagir un composé de formule générale Ic ou Id

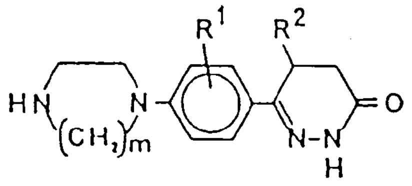

(Ic)

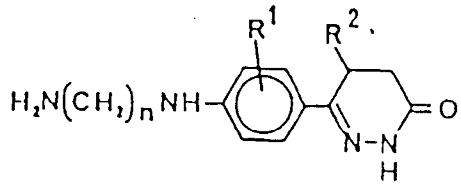

(Id)

dans lesquelles $R^1$, $R^2$, m et n ont les significations indiquées ci-dessus, avec un agent acylant de formule générale VI

$R^{3'}$-L (VI)

dans laquelle $R^{3'}$ a la signification indiquée ci-dessus et L représente un atome d'halogène, le groupe -$OR^{3'}$, le groupe hydroxy ou le reste d'un azole ou benzazole à au moins 2 atomes d'azote dans le cycle à 5 chaînons, relié par un atome d'azote, ou bien

e) pour la fabrication de composés de formule générale I, dans laquelle $R^1$ et $R^2$ sont définis comme ci-dessus et A représente le groupe

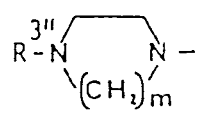

$R^2$ sont définis comme ci-dessus et A représente le groupe ou le groupe $R^{3''}$-NH-$(CH_2)_n$-NH-, dans lesquels m et n sont définis comme ci-dessus et $R^{3''}$ représente un atome d'hydrogène, on soumet à l'hydrolyse acide ou basique et éventuellement on décarboxyle un composé de formule générale I, dans laquelle $R^1$ et $R^2$ sont définis comme à la revendication 1 et A représente le groupe

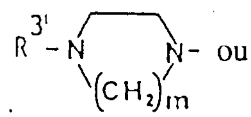 ou $R^{3'}$-NH-$(CH_2)_n$,

dans lesquels $R^{3'}$ représente un groupe cyano, un groupe acyle, un groupe aroyle éventuellement substitué ou un groupe (alcoxy en $C_1$-$C_6$) carbonyle et en ce que l'on transforme éventuellement de manière connue le composé de formule générale I obtenu selon les variantes du procédé a) à e) en un sel physiologiquement acceptable.

3. Procédé selon la revendication 1 ou 2 pour la fabrication de composés dans lesquels $R^1$ représente un groupe nitro, un groupe amino, un groupe hydroxy, un groupe cyano ou un atome d'halogène, $R^2$ représente un atome d'hydrogène, un groupe méthyle ou un groupe hydroxyméthyle et A représente le groupe

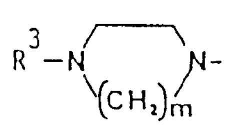

,dans lequel $R^3$ est un atome d'hydrogène, un groupe alkyle en $C_1$-$C_6$ éventuellement substitué par un groupe amino ou un groupe hydroxy, un groupe aryle ou hétéroaryle éventuellement substitué, un groupe cyano, un groupe acyle, un groupe aroyle éventuellement substitué ou un groupe (alcoxy en $C_1$-$C_6$)carbonyle et m est un entier de 1 à 3.

4. Procédé selon la revendication 1 ou 2 pour la fabrication de composés dans lesquels $R^1$ représente un groupe nitro, un groupe amino, un groupe hydroxy, un groupe cyano ou un atome d'halogène, $R^2$ représente un atome d'hydrogène ou un groupe méthyle et A représente le groupe $R^3$-NH$(CH_2)_n$-NH-, dans lequel $R^3$ représente un atome d'hydrogène, un groupe aryle ou hétéroaryle éventuellement substitué, un groupe acyle, un groupe aroyle ou un groupe (alcoxy en $C_1$-$C_6$)carbonyle et n est un entier de 2 à 4.

5. Procédé selon la revendication 1 ou 2 pour la fabrication de composés dans lesquels $R^1$ représente un

groupe nitro, un groupe amino, un groupe hydroxy, un groupe cyano ou un atome d'halogène, $R^2$ représente un atome d'hydrogène ou un groupe méthyle et A représente le groupe

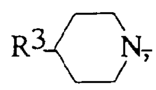

dans lequel $R^3$ représente un groupe alkyle en $C_1$-$C_6$ éventuellement substitué par un groupe amino ou un groupe hydroxy, un groupe aryle ou hétéroaryle éventuellement substitué, un groupe acyle, un groupe aroyle éventuellement substitué, un groupe carboxyle ou un groupe (alcoxy en $C_1$-$C_6$)carbonyle.

6. Procédé selon la revendication 1 ou 2 pour la fabrication de composés dans lesquels $R^1$ représente un groupe nitro, un groupe amino, un groupe hydroxy, un groupe cyano ou un atome d'halogène, $R^2$ représente un atome d'hydrogène, un groupe méthyle ou un groupe hydroxyméthyle et A représente le groupe

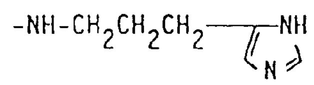

7. Procédé selon la revendication 1 ou 2 pour la fabrication de la 6-[3-nitro-4-(1-pipérazinyl)phényl]-4,5-dihydro-5-méthyl-3(2H)-pyridazinone et de ses sels physiologiquement acceptables.

8. Procédé selon la revendication 1 ou 2 pour la fabrication de la 6-[4-(4-éthoxycarbonylpipérazine-1-yl)-3-nitrophényl]-4,5-dihydro-5-méthyl-3(2H)pyridazinone et de ses sels physiologiquement acceptables.

9. Procédé selon la revendication 1 ou 2 pour la fabrication de la 6-[3-amino-4-(4-éthoxycarbonylpipérazine-1-yl)-phényl]-4,5-dihydro-5-méthyl-3(2H)-pyridazinone et de ses sels physiologiquement acceptables.

10. Procédé selon la revendication 1 ou 2 pour la fabrication de la 6-[4-(4-acétylpipérazine-1-yl)-3-nitrophényl]-4,5-dihydro-5-méthyl-3(2H)-pyridazinone et de ses sels physiologiquement acceptables.

11. Procédé selon la revendication 1 ou 2 pour la fabrication de la 6-[4-(4-éthoxycarbonylpipérazine- 1-yl)-3-nitrophényl]-4,5-dihydro-3(2H)-pyridazinone et de ses sels physiologiquement acceptables.

**Revendications pour l'Etat contractant suivant : GR**

1. 6-phényldihydro-3(2H)-pyridazinones, caractérisées en ce qu'elles répondent à la formule générale I :

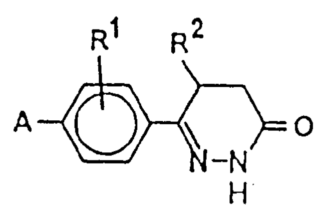

(I)

dans laquelle $R^1$ représente un groupe nitro, un groupe amino, un groupe hydroxy, un groupe cyano ou un atome d'halogène,

$R^2$ représente un atome d'hydrogène, un groupe méthyle ou un groupe hydroxyméthyle et

A représente le groupe

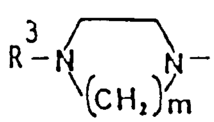, $R^3-NH-(CH_2)_n-NH-$, 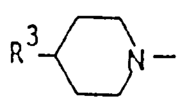

ou 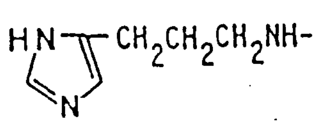 ,

dans lesquels $R^3$ représente un atome d'hygrogène, un groupe alkyle en $C_1$-$C_6$ éventuellement substitué par un groupe amino ou hydroxy, un groupe aryle ou hétéroaryle éventuellement substitué, un groupe cyano, un

groupe acyle, un groupe aroyle éventuellement substitué, un groupe carboxyle ou un groupe (alcoxy en $C_1$-$C_6$)carbonyle, m est un entier de 1 à 3 et n est un entier de 2 à 4, ainsi que leurs sels.

2. 6-phényldihydro-3(2H)-pyridazinones selon la revendication 1, caractérisées en ce que $R^1$ représente un groupe nitro, un groupe amino, un groupe hydroxy, un groupe cyano ou un atome d'halogène, $R^2$ représente un atome d'hydrogène, un groupe méthyle ou un groupe hydroxyméthyle et A représente le groupe

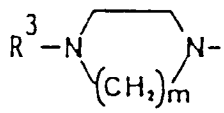

,dans lequel $R^3$ est un atome d'hydrogène, un groupe alkyle en $C_1$-$C_6$ éventuellement substitué par un groupe amino ou un groupe hydroxy, un groupe aryle ou hétéroaryle éventuellement substitué, un groupe cyano, un groupe acyle, un groupe aroyle éventuellement substitué ou un groupe (alcoxy en $C_1$-$C_6$)carbonyle et m est un entier de 1 à 3.

3. 6-phényldihydro-3(2H)-pyridazinones selon la revendication 1, caractérisées en ce que $R^1$ représente un groupe nitro, un groupe amino, un groupe hydroxy, un groupe cyano ou un atome d'halogène, $R^2$ représente un atome d'hydrogène ou un groupe méthyle et A représente le groupe $R^3$-$NH(CH_2)_n$-NH-, dans lequel $R^3$ représente un atome d'hydrogène, un groupe aryle ou hétéroaryle éventuellement substitué, un groupe acyle, un groupe aroyle ou un groupe (alcoxy en $C_1$-$C_6$)carbonyle et n est un entier de 2 à 4.

4. 6-phényldihydro-3(2H)-pyridazinones selon la revendication 1, caractérisées en ce que $R^1$ représente un groupe nitro, un groupe amino, un groupe hydroxy, un groupe cyano ou un atome d'halogène, $R^2$ représente un atome d'hydrogène ou un groupe méthyle et A représente le groupe

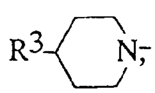

dans lequel $R^3$ représente un groupe alkyle en $C_1$-$C_6$ éventuellement substitué par un groupe amino ou un groupe hydroxy, un groupe aryle ou hétéroaryle éventuellement substitué, un groupe acyle, un groupe aroyle éventuellement substitué, un groupe carboxyle ou un groupe (alcoxy en $C_1$-$C_6$)carbonyle.

5. 6-phényldihydro-3(2H)pyridazinones selon la revendication 1, caractérisées en ce que $R^1$ représente un groupe nitro, un groupe amino, un groupe hydroxy, un groupe cyano ou un atome d'halogène, $R^2$ représente un atome d'hydrogène, un groupe méthyle ou un groupe hydroxyméthyle et A représente le groupe

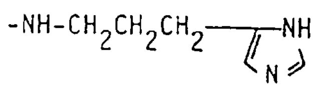

6. La 6-[3-nitro-4-(1-pipérazinyl)phényl]-4,5-dihydro-5-méthyl-3(2H)-pyridazinone et ses sels.

7. La 6-[4-(4-éthoxycarbonylpipérazine-1-yl)-3-nitrophényl]-4,5-dihydro-5-méthyl-3(2H)pyridazinone et ses sels.

8. La 6-[3-amino-4-(4-éthoxycarbonylpipérazine-1-yl)-phényl]-4,5-dihydro-5-méthyl-3(2H)-pyridazinone et ses sels.

9. La 6-[4-(4-acétylpipérazine-1-yl)-3-nitrophényl]-4,5-dihydro-5-méthyl-3(2H)-pyridazinone et ses sels.

10. La 6-[4-(4-éthoxycarbonylpipérazine-1-yl)-3-nitrophényl]-4,5-dihydro-3(2H)-pyridazinone et ses sels.

11. Procédé pour la fabrication de 6-phényldihydro-3(2H)-pyridazinones selon les revendications 1 à 10, caractérisé en ce que l'on fait réagir un composé de formule générale II :

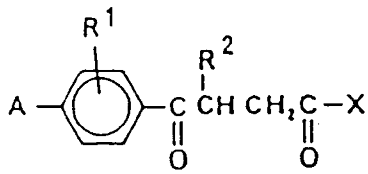

(II)

dans laquelle A, $R^1$ et $R^2$ sont définis comme à la revendication 1 et X représente le groupe -OH ou le groupe -$OR^4$ dans lequel $R^4$ est un groupe alkyle en $C_1$-$C_6$ éventuellement substitué ou un groupe aryle éventuellement

substitué, avec l'hydrazine ou l'un des ses équivalents chimiques pour obtenir le composé de formule générale I et on transforme éventuellement de manière connue le composé obtenu en son sel physiologiquement acceptable.

12. Procédé pour la fabrication de 6-phényldihydro-3(2H)-pyridazinones selon les revendications 1 à 10, caractérisé en ce que

a) pour la fabrication de composés de formule générale I dans laquelle A et $R^2$ sont définis comme à la revendication 1 et $R^1$ représente un groupe nitro, on fait réagir un composé de formule générale III :

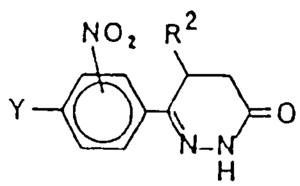

(III)

dans laquelle $R^2$ est défini comme à la revendication 1 et Y représente un atome d'halogène avec un composé de formule générale IV

H-A (IV)

dans laquelle A est défini comme à la revendication 1, ou bien

b) pour la fabrication de composés de formule générale I dans laquelle A et $R^2$ sont définis comme à la revendication 1 et $R^1$ représente un groupe amino, on réduit un composé de formule générale Ia

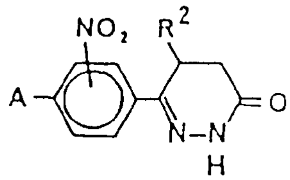

(Ia)

dans laquelle $R^2$ et A sont définis comme à la revendication 1 et $R^1$ représente un groupe nitro, par un agent réducteur, ou bien

c) pour la fabrication de composés de formule générale I dans laquelle $R^1$ et $R^2$ ont les significations indiquées dans la revendication 1 et $R^1$ représente un groupe hydroxy, un groupe cyano ou un atome d'halogène, on diazote un composé de formule générale Ib

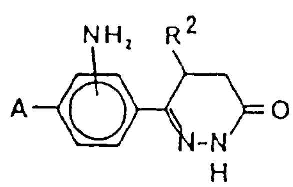

(Ib)

dans laquelle A et $R^2$ sont définis comme à la revendication 1, en un composé de formule générale V

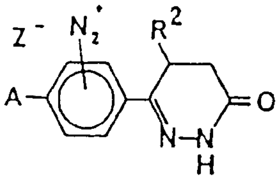

(V)

dans laquelle A et $R^2$ sont définis comme à la revendication 1 et Z représente un atome d'halogène, un groupe hydroxy, un groupe cyano ou un groupe tétrafluoroborate, et on fait réagir le sel de diazonium de formule générale V, éventuellement en présence de poudre de cuivre ou d'un halogénure de cuivre (I) ou de cyanure de cuivre (I), en un composé de formule générale I dans laquelle $R^2$ et A sont définis comme à la revendication 1 et $R^1$ représente un groupe hydroxy, un groupe cyano ou un atome d'halogène, ou bien

d) pour la fabrication de composés de formule générale I dans laquelle $R^1$ et $R^2$ sont définis comme à la

revendication 1 et A représente le groupe

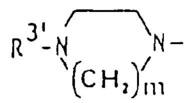

ou le groupe $R^{3'}$-NH-$(CH_2)_n$NH-, dans lesquels m et n sont définis comme à la revendication 1 et $R^{3'}$ représente un groupe cyano, un groupe acyle, un groupe aroyle éventuellement substitué ou un groupe (alcoxy en $C_1$-$C_6$)carbonyle, on fait réagir un composé de formule générale Ic ou Id

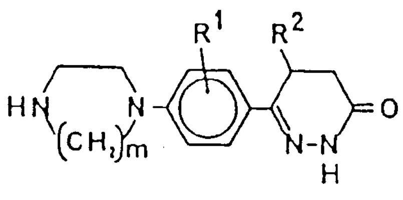

(Ic)

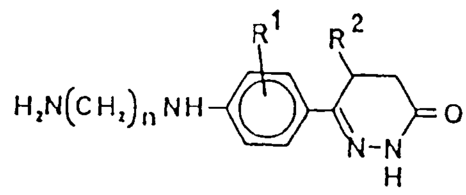

(Id)

dans lesquelles $R^1$, $R^2$, m et n ont les significations indiquées dans la revendication 1, avec un agent acylant de formule générale VI

$R^{3'}$-L (VI)

dans laquelle $R^{3'}$ a la signification indiquée ci-dessus et L représente un atome d'halogène, le groupe -$OR^{3'}$, le groupe hydroxy ou le reste d'un azole ou benzazole à au moins 2 atomes d'azote dans le cycle à 5 chaînons, relié par un atome d'azote, ou bien

e) pour la fabrication de composés de formule générale I, dans laquelle $R^1$ et $R^2$ sont définis comme à la revendication 1 et A représente le groupe

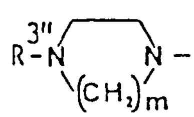

ou le groupe $R^{3''}$-NH-$(CH_2)_n$-NH-, dans lesquels m et n sont définis comme à la revendication 1 et $R^{3''}$ représente un atome d'hydrogène, on soumet à l'hydrolyse acide ou basique et éventuellement on décarboxyle un composé de formule générale I, dans laquelle $R^1$ et $R^2$ sont définis comme à la revendication 1 et A représente le groupe

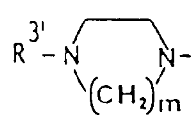

ou $R^{3'}$-NH-$(CH_2)_n$, dans lesquels $R^{3'}$ représente un groupe cyano, un groupe acyle, un groupe aroyle éventuellement substitué ou un groupe (alcoxy en $C_1$-$C_6$) carbonyle

et en ce que l'on transforme éventuellement de manière connue le composé de formule générale I obtenu selon les variantes du procédé a) à e) en un sel physiologiquement acceptable.